(19) Europäisches Patentamt
European Patent Office
Office européen des brevets

(11) EP 2 036 906 A1

(12) # EUROPÄISCHE PATENTANMELDUNG

(43) Veröffentlichungstag:
**18.03.2009 Patentblatt 2009/12**

(51) Int Cl.:
***C07D 471/04*** (2006.01) ***A61K 31/437*** (2006.01)
***A61P 15/16*** (2006.01)

(21) Anmeldenummer: **07075764.6**

(22) Anmeldetag: **05.09.2007**

(84) Benannte Vertragsstaaten:
**AT BE BG CH CY CZ DE DK EE ES FI FR GB GR HU IE IS IT LI LT LU LV MC MT NL PL PT RO SE SI SK TR**
Benannte Erstreckungsstaaten:
**AL BA HR MK RS**

(71) Anmelder:
- **Bayer Schering Pharma Aktiengesellschaft**
  **13353 Berlin (DE)**
- **Bayer HealthCare AG**
  **51368 Leverkusen (DE)**

(72) Erfinder:
- **Buchmann, Bernd**
  **16540 Hohen Neuendorf (DE)**
- **Härter, Michael**
  **51375 Leverkusen (DE)**
- **Cancho-Grande, Yolanda**
  **51373 Leverkusen (DE)**
- **Kosemund, Dirk**
  **07749 Jena (DE)**
- **Schirok, Hartmut**
  **42287 Wuppertal (DE)**
- **Nguyen, Duy**
  **10179 Berlin (DE)**
- **Fritsch, Martin**
  **10555 Berlin (DE)**

(54) **Azaindole als Inhibitoren der löslichen Adenylatzyklase**

(57) Die vorliegende Erfindung betrifft Azaindole der allgemeinen Formel I, Verfahren zu ihrer Herstellung sowie deren Verwendung zur Herstellung von pharmazeutischen Mitteln.

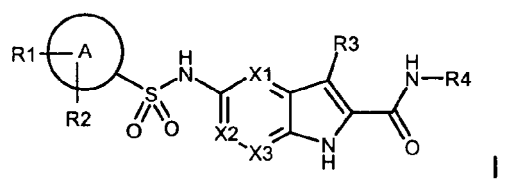

I

Printed by Jouve, 75001 PARIS (FR)

## Beschreibung

Die vorliegende Erfindung betrifft Inhibitoren der löslichen Adenylatzyklase, deren Herstellung sowie deren Verwendung zur Herstellung eines Arzneimittels zur Kontrazeption.

Derzeitig können Frauen von einer Vielzahl moderner Methoden zur Kontrazeption Gebrauch machen, für die männliche Fertilitätskontrolle hingegen stehen nur sehr wenige Methoden zur Verfügung (Kondom und Sterilisation). Die Entwicklung von neuen zuverlässigen Mitteln für die männliche Fertilitätskontrolle ist zwingend notwendig. Hierbei sollte die durch eine "männliche Pille" hervorgerufene Infertilität vollständig reversibel sein und genauso wirksam wie die existierenden Methoden, die der Frau zur Verfügung stehen. Die Unfruchtbarkeit sollte relativ schnell einsetzen und möglichst lang anhaltend sein. Eine derartige Verhütungsmethode sollte keine Nebenwirkungen haben, es kann sich hierbei neben hormonellen Ansätzen auch um nicht-hormonelle Ansätze handeln. Ein möglicher Ansatzpunkt ist die Regulation der Aktivität eines Enzyms, das eine wichtige Rolle bei der Befruchtung der Eizelle spielt, die lösliche Adenylatzyklase (sAC). Dieses Enzym wird hauptsächlich in den testikulären Stammzellen exprimiert und ist in reifem Sperma vorhanden.

1999 gelang es den Autoren Levin und Buck (Proc. Natl. Acad. Sci. USA 96 (1): 79-84), eine Isoform der sAC aus den Testes der Ratte zu reinigen und zu klonieren.

Das rekombinante Enzym der Ratte kann durch Bicarbonat stimuliert werden. Mit Hilfe von Antikörpern konnte nachgewiesen werden, dass die katalytische Domäne des Enzyms in Testes, Sperma, Nieren und dem Choroid Plexus lokalisiert ist. Diese Offenbarungen sind Gegenstand der Anmeldung WO01/85753, die in den US zur Erteilung gekommen ist (US6544768).

In der WO01/21829 (Conti et al.*)* werden isolierte Polynukleotidesequenzen, die für die humane Isoform der sAC kodieren, isolierte sAC Polypeptide und Testsysteme beansprucht, mit deren Hilfe Substanzen identifiziert werden können, die die Aktivität der sAC inhibieren. Die Möglichkeit, diese Substanzen zu benutzen, um die Anzahl der beweglichen Samenzellen reversibel zu reduzieren, sowie deren Verwendung als Mittel zur männlichen Fertilitätskontrolle, wird offenbart.

Die Gruppe von John Herr zeigte die Isolation und Charakterisierung der humanen Isoform der sAC aus Sperma. In der WO 02/20745 werden neben Nukleinsäuren, die für die sAC kodieren, auch Testsysteme beansprucht, mit deren Hilfe Substanzen identifiziert werden können, die die Expression oder die Aktivität der humanen sAC modulieren. Derartige Verbindungen könnten beispielsweise selektiv die Aktivität der sAC inhibieren, dies hätte zur Folge, dass die Samenzellen die Fähigkeit verlieren, eine Eizelle zu befruchten. Diese Inhibitoren der sAC könnten daher als Arzneimittel der nicht-hormonellen Kontrazeption dienen.

Die bereits bekannten Inhibitoren der sAC zeigen jedoch spezifische Probleme: Catecholöstrogene (T. Braun, Proc Soc Exp Biol Med 1990, 194(1): 58ff) und Gossypol (KL Olgiati, Arch Biochem Biophys 1984, 231(2): 411 ff) sind inhärent toxisch, während Adenosinanaloga nur mit sehr schwacher Wirkung inhibieren (MA Brown und ER Casillas, J Androl 1984, 5:361ff). Etwas potenter sind die Inhibitoren ($IC_{50} \leq 10\ \mu M$) der rekombinanten humanen sAC, die von Zippin *et al*. beschrieben werden (JH Zippin et al. J Cell Biol 2004, 164(4): 527ff).

In der Patentanmeldung WO 06/032541 (Bayer Schering Pharma) werden erstmalig Inhibitoren der sAC gezeigt, die in einem Bereich von 70nM - 10μM wirksam sind. Nach wie vor besteht jedoch Bedarf, noch wirksamere Verbindungen für die männliche Fertilitätskontrolle zur Verfügung stellen zu können.

Aufgabe der vorliegenden Erfindung ist es daher, stabile Inhibitoren der löslichen Adenylatzyklase zur Verfügung zu stellen.

Diese Aufgabe wird gelöst durch die Bereitstellung der Verbindungen der allgemeinen Formel I

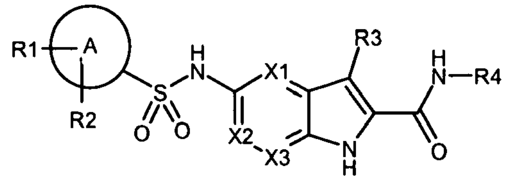

(I)

wobei

A ein $C_6$-$C_{12}$-Aryl- oder $C_5$-$C_{12}$-Heteroarylrest, der gegebenenfalls ein- oder mehrfach mit $R^1$ und/oder $R^2$ substituiert sein kann,

$R^1$ ein Wasserstoff, Halogen, Amino, eine $-S(O)_p-C_1-C_6$-Alkylgruppe, wobei p für 0 - 2 steht, ein $SO_2NH_2$, eine $-SO_2NH-C_1-C_6$-Alkylgruppe, eine $-NH-SO_2-C_1-C_6$-Alkylgruppe, ein $C_1-C_6$-Acyl-, $NH-CO-NH_2$, -O-CO-NH ($C_1-C_6$- Alkyl), -O-CO-N($C_1-C_6$-Alkyl)$_2$, NH-CO-$C_1-C_6$-Alkylrest, Hydroxy, Cyano, O-CO-($C_1-C_6$-Alkyl), $CO_2$-($C_1-C_6$-Alkyl), CO-NH-($C_1-C_6$- Alkyl), CO-N($C_1-C_6$-Alkyl)2, CO-NH($C_5-C_{12}$-Heteroaryl), NH-($C_1-C_6$- Alkyl), N-($C_1-C_6$-Alkyl)$_2$, oder
ein $C_1-C_6$-Alkyl,
welches gegebenenfalls ein- oder mehrfach, gleich oder verschieden substituiert sein kann mit COOH, CO-$NH_2$, CO- NH-($C_1-C_6$-Alkyl), CO-N($C_1-C_6$-Alkyl)$_2$, $C_1-C_6$-Acyl, $C_1-C_6$- Alkoxy, Hydroxy, Cyano, $CO_2$-($C_1-C_6$-Alkyl), N-($C_1-C_6$- Alkyl)$_2$, $C_5-C_{12}$-Heteroaryl,
welches gegebenenfalls mehrfach substituiert ist, $C_6-C_{12}$-Aryl,
welches gegebenenfalls mehrfach substituiert ist,
oder
ein $C_1-C_6$-Alkoxy,
welches gegebenenfalls ein- oder mehrfach, gleich oder verschieden substituiert sein kann mit Hydroxy, Cyano, $CO_2$-($C_1-C_6$-Alkyl), N-($C_1-C_6$-Alkyl)$_2$, NH-$C_3-C_6$-Cycloalkyl, COOH, CO-$NH_2$, CO-NH($C_1-C_6$-Alkyl) oder mit CO-N($C_1-C_6$- Alkyl)$_2$,
ein O-$C_6-C_{12}$-Aryl,
welches gegebenenfalls ein- oder mehrfach, gleich oder verschieden substituiert sein kann mit Halogen, Hydroxy, Cyano, COOH, CO-$NH_2$, $C_1-C_6$-Alkyl, $C_3-C_6$-Cycloalkyl, $C_1$- $C_6$-Acyl oder $C_1-C_6$-Alkoxy, oder
ein O-$C_5-C_{12}$-Heteroaryl,
welches gegebenenfalls ein- oder mehrfach, gleich oder verschieden mit Halogen, Hydroxy, Cyano, COOH, CO- $NH_2$, $C_1-C_6$-Alkyl, $C_3-C_6$-Cycloalkyl, $C_1-C_6$-Acyl oder $C_1-C_6$- Alkoxy substituiert sein kann, oder
ein $C_6-C_{12}$-Aryl,
welches gegebenenfalls ein- oder mehrfach, gleich oder verschieden substituiert sein kann mit Halogen, $C_1-C_6$-Alkyl, $C_3-C_6$-Cycloalkyl, $C_1-C_6$-Acyl, $C_1-C_6$-Alkoxy, $C_6-C_{12}$-Aryl, $C_5$- $C_{12}$-Heteroaryl, Hydroxy, $CH_2$-OH, Cyano, $CH_2$-CN, Amino, $CO_2$-($C_1-C_6$-Alkyl), N-($C_1-C_6$-Alkyl)$_2$, $NHSO_2CH_3$, $SO_2NH_2$, $SO_2NH$($C_1-C_6$-Alkyl), $SO_2N$($C_1-C_6$-Alkyl)$_2$, COOH, CO-$NH_2$, CO-NH-($C_1-C_6$-Alkyl), CO-N($C_1-C_6$-Alkyl)$_2$, CO-NH($C_5-C_{12}$- Heteroaryl), NH-CO($C_1-C_6$-Alkyl), $CH_2$-NH-CO($C_1-C_6$-Alkyl), NH-CO($C_5-C_{12}$-Heteroaryl) oder ein $-S(O)_r-CH_3$, wobei r für 0 - 2 steht, oder
zwei benachbarte Positionen durch $-O-CH_2-O-$ oder $-O-C(CH_3)_2-$ O- substituiert sein können, oder ein $C_5-C_{16}$-Heteroaryl,
welches gegebenenfalls ein- oder mehrfach, gleich oder verschieden substituiert sein kann mit Halogen, $C_1-C_6$-Alkyl, $C_3-C_6$-Cycloalkyl, $C_1-C_6$-Acyl, $C_1-C_6$-Alkoxy, $C_6-C_{12}$-Aryl, $C_5$- $C_{12}$-Heteroaryl, Hydroxy, $CH_2$-OH, Cyano, $CH_2$-CN, Amino, $CO_2$-($C_1-C_6$-Alkyl), N-($C_1-C_6$-Alkyl)$_2$, $NHSO_2CH_3$, $SO_2NH_2$, $SO_2NH$($C_1-C_6$-Alkyl), $SO_2N$($C_1-C_6$-Alkyl)$_2$, COOH, CO-$NH_2$, CO-NH-($C_1-C_6$-Alkyl), CO-N($C_1-C_6$-Alkyl)$_2$, CO-NH($C_5-C_{12}$- Heteroaryl), NH-CO($C_1-C_6$-Alkyl), $CH_2$-NH-CO($C_1-C_6$-Alkyl), NH-CO($C_5-C_{12}$-Heteroaryl) oder ein $-S(O)_r-CH_3$, wobei r für 0 - 2 steht, oder
ein $C_3-C_6$-Cycloalkyl,
welches gegebenenfalls ein- oder mehrfach, gleich oder verschieden mit Halogen, $C_1-C_6$-Alkyl, Hydroxy, Cyano, $CO_2$-($C_1-C_6$-Alkyl), $C_1-C_6$-Acyl, N-($C_1-C_6$-Alkyl)$_2$, COOH, CO- $NH_2$, CO-NH($C_1-C_6$-Alkyl), CO-N ($C_1-C_6$-Alkyl)$_2$ oder $C_1-C_6$- Alkoxy substituiert sein kann,

$R^2$ ein Wasserstoff, Halogen, Amino, eine $-S(O)_p-C_1-C_6$-Alkylgruppe, wobei p für 0 - 2 steht, ein $SO_2NH_2$, $-SO_2NH-C_1-C_6$-Alkylgruppe, $-NH-SO_2-C_1-C_6$- Alkylgruppe, $C_1-C_6$-Acyl-, $NH-CO-NH_2$, -O-CO-NH($C_1-C_6$-Alkyl), -O-CO-N($C_1-C_6$-Alkyl)$_2$ oder NH-CO-$C_1-C_6$-Alkylrest, Hydroxy, Cyano, O-CO-($C_1-C_6$-Alkyl), $CO_2$-($C_1-C_6$-Alkyl), CO-NH-($C_1-C_6$-Alkyl), CO- N($C_1-C_6$-Alkyl)$_2$, CO-NH($C_5-C_{12}$-Heteroaryl), NH-($C_1-C_6$-Alkyl), N- ($C_1-C_6$-Alkyl)$_2$, oder
ein $C_1-C_6$-Alkyl,
welches gegebenenfalls ein- oder mehrfach, gleich oder verschieden substituiert sein kann mit COOH, CO-$NH_2$, CO- NH($C_1-C_6$-Alkyl) oder mit CO-N($C_1-C_6$-Alkyl)$_2$, $C_1-C_6$-Acyl, $C_1-C_6$-Alkoxy, Hydroxy, Cyano, $CO_2$-($C_1-C_6$-Alkyl), N-($C_1-C_6$- Alkyl)$_2$, $C_5-C_{12}$-Heteroaryl,
welches gegebenenfalls mehrfach substituiert ist, $C_6-C_{12}$-Aryl,
welches gegebenenfalls mehrfach substituiert ist,
oder
ein $C_1-C_6$-Alkoxy,
welches gegebenenfalls ein- oder mehrfach, gleich oder verschieden substituiert sein kann mit Hydroxy, Cyano, $CO_2$-($C_1-C_6$-Alkyl), N-($C_1-C_6$-Alkyl)$_2$, NH-$C_3-C_6$-Cycloalkyl, COOH, CO-$NH_2$, CO-NH($C_1-C_6$-Alkyl) oder mit CO-N($C_1-C_6$- Alkyl)$_2$,

ein $O-C_6-C_{12}$-Aryl,
welches gegebenenfalls ein- oder mehrfach, gleich oder verschieden mit Halogen, Hydroxy, Cyano, COOH, CO- $NH_2$, $C_1-C_6$-Alkyl, $C_3-C_6$-Cycloalkyl, $C_1-C_6$-Acyl oder $C_1-C_6$- Alkoxy substituiert sein kann, oder
ein $O-C_5-C_{12}$-Heteroaryl,
welches gegebenenfalls ein- oder mehrfach, gleich oder verschieden mit Halogen, Hydroxy, Cyano, COOH, CO- $NH_2$, $C_1-C_6$-Alkyl, $C_3-C_6$-Cycloalkyl, $C_1-C_6$-Acyl oder $C_1-C_6$- Alkoxy substituiert sein kann, oder
ein $C_6-C_{12}$-Aryl,
welches gegebenenfalls ein- oder mehrfach, gleich oder verschieden substituiert sein kann mit Halogen, $C_1-C_6$-Alkyl, $C_3-C_6$-Cycloalkyl, $C_1-C_6$-Acyl, $C_1-C_6$-Alkoxy, $C_6-C_{12}$-Aryl, $C_5$- $C_{12}$-Heteroaryl, Hydroxy, $CH_2$-OH, Cyano, $CH_2$-CN, Amino, $CO_2$-($C_1-C_6$-Alkyl), N-($C_1-C_6$-Alkyl)$_2$, $NHSO_2CH_3$, $SO_2NH_2$, $SO_2NH$($C_1-C_6$-Alkyl), $SO_2N$($C_1-C_6$-Alkyl)$_2$, COOH, CO-$NH_2$, CO-NH($C_1-C_6$-Alkyl), CO-N($C_1-C_6$-Alkyl)$_2$, CO-NH($C_5-C_{12}$- Heteroaryl), NH-CO($C_1-C_6$-Alkyl), $CH_2$-NH-CO($C_1-C_6$-Alkyl), NH-CO($C_5-C_{12}$-Heteroaryl) oder ein -S(O)$_r$-$CH_3$, wobei r für 0 - 2 steht, oder
zwei benachbarte Positionen durch -O-$CH_2$-O- oder -O-C($CH_3$)$_2$- O- substituiert sein können, oder ein $C_5-C_{16}$-Heteroaryl,
welches gegebenenfalls ein- oder mehrfach, gleich oder verschieden substituiert sein kann mit Halogen, $C_1-C_6$-Alkyl, $C_3-C_6$-Cycloalkyl, $C_1-C_6$-Acyl, $C_1-C_6$-Alkoxy, $C_6-C_{12}$-Aryl, $C_5$- $C_{12}$-Heteroaryl, Hydroxy, $CH_2$-OH, Cyano, $CH_2$-CN, Amino, $CO_2$-($C_1-C_6$-Alkyl), N-($C_1-C_6$-Alkyl)$_2$, $NHSO_2CH_3$, $SO_2NH_2$, $SO_2NH$($C_1-C_6$-Alkyl), $SO_2N$($C_1-C_6$-Alkyl)$_2$, COOH, CO-$NH_2$, CO-NH-($C_1-C_6$-Alkyl), CO-N($C_1-C_6$-Alkyl)$_2$, CO-NH($C_5-C_{12}$- Heteroaryl), NH-CO($C_1-C_6$-Alkyl), $CH_2$-NH-CO($C_1-C_6$-Alkyl), NH-CO($C_5-C_{12}$-Heteroaryl) oder ein -S(O)$_r$$CH_3$, wobei r für 0 - 2 steht, oder
ein $C_3-C_6$-Cycloalkyl,
welches gegebenenfalls ein- oder mehrfach, gleich oder verschieden mit Halogen, $C_1-C_6$-Alkyl, Hydroxy, Cyano, $CO_2$- ($C_1-C_6$-Alkyl), $C_1-C_6$-Acyl, N-($C_1-C_6$-Alkyl)$_2$, COOH, CO-$NH_2$, CO-NH($C_1-C_6$-Alkyl), CO-N ($C_1-C_6$-Alkyl)$_2$ oder $C_1-C_6$-Alkoxy substituiert sein kann,

$R^3$ ein C6-C12-Aryl,
welches gegebenenfalls ein- oder mehrfach, gleich oder verschieden substituiert sein kann mit Halogen, $C_1-C_6$-Alkyl, $C_1-C_6$-Alkoxy, Hydroxy, $C_1-C_6$-Alkylen-OH, $C_1-C_6$-Alkylen- $NR^5R^6$, Cyano, $CO_2$-($C_1-C_6$-Alkyl), N-($C_1-C_6$-Alkyl)$_2$, CO-NH- ($C_1-C_6$-Alkyl), CO-NH-($C_1-C_6$-Alkylen-OH), CO-NH-($C_1-C_6$- Alkylen-$NR^5R^6$), CO-N($C_1-C_6$-Alkyl)$_2$ oder $C_1-C_6$-Acyl,
welches gegebenenfalls ein- oder mehrfach substituiert ist,
oder
mit zwei benachbarten Positionen durch -O-$CH_2$-O-, -O- $CH_2$-$CH_2$-O-, oder -O-C($CH_3$)$_2$-O- substituiert sein kann,
oder
ein $C_5-C_{16}$-Heteroaryl,
welches gegebenenfalls ein- oder mehrfach, gleich oder verschieden substituiert sein kann mit Halogen, $C_1-C_6$-Alkyl, $C_1-C_6$-Acyl, $C_1-C_6$-Alkoxy, Hydroxy, Cyano, $CO_2$-($C_1-C_6$- Alkyl), N-($C_1-C_6$-Alkyl)$_2$, CO-NH-($C_1-C_6$-Alkylen-OH), CO- NH-($C_1-C_6$-Alkylen-$NR^5R^6$), CO-NH($C_1-C_6$-Alkyl) oder CO- N($C_1-C_6$-Alkyl)$_2$
oder
ein $C_3-C_6$-Cycloalkyl, welches gegebenenfalls ein- oder mehrfach, gleich oder verschieden mit Halogen, $CF_3$, Hydroxy, Cyano, $CO_2$-($C_1-C_6$-Alkyl), $C_1-C_6$-Alkyl, $C_1-C_6$-Acyl, N-($C_1-C_6$-Alkyl)$_2$, CO-NH-($C_1-C_6$-Alkylen-OH), CO-NH-($C_1-C_6$-Alkylen-$NR^5R^6$), CO-NH($C_1-C_6$-Alkyl) oder CO-N($C_1-C_6$-Alkyl)$_2$ oder $C_1-C_6$- Alkoxy substituiert sein kann, ein $C_6-C_{12}$-Aryl,

$R^4$ ein Wasserstoff,
ein $C_1-C_6$-Alkyl,
welches gegebenenfalls ein- oder mehrfach, gleich oder verschieden substituiert sein kann mit einer -S (O)$_p$-$C_1-C_6$- Alkylgruppe oder -S(O)$_p$-$C_3-C_6$-Cycloalkylgruppe, wobei p für 0 - 2 steht, Hydroxy, Cyano, COOH, CO-$NH_2$, $SO_2NH_2$, $SO_2NH$-($C_1-C_6$-Alkyl), $C_3-C_6$-Cycloalkyl, $C_1-C_6$-Acyl, $C_1-C_6$- Alkoxy, $CO_2$-($C_1-C_6$-Alkyl), N-($C_1-C_6$-Alkyl)$_2$, NH-($C_1-C_6$- Alkyl), NH-($C_3-C_6$-Cycloalkyl), CO-NH($C_1-C_6$-Alkyl), CO- N($C_1-C_6$-Alkyl)$_2$ oder $C_6-C_{12}$-Aryl,
welches gegebenenfalls mehrfach substituiert ist,
oder
$C_5-C_{12}$-Heteroaryl,
welches gegebenenfalls mehrfach substituiert ist, oder
ein $C_3-C_6$-Cycloalkyl,

welches gegebenenfalls ein- oder mehrfach, gleich oder verschieden substituiert sein kann mit Hydroxy, $CH_2$-OH, Cyano, COOH, CO-$NH_2$, $SO_2NH_2$, $C_1$-$C_6$-Alkyl, $C_1$-$C_6$-Acyl oder $C_1$-$C_6$-Alkoxy, oder ein $C_6$-$C_{12}$-Aryl,
welches gegebenenfalls ein- oder mehrfach, gleich oder verschieden mit Halogen, Hydroxy, $C_1$-$C_6$-Alkyl, $C_1$-$C_6$-Acyl, $C_1$-$C_6$-Alkoxy, N-($C_1$-$C_6$-Alkyl)$_2$, NH-($C_1$-$C_6$-Alkyl) oder Cyano substituiert sein kann, oder ein $C_5$-$C_{12}$-Heteroaryl,
welches gegebenenfalls ein- oder mehrfach, gleich oder verschieden mit Halogen, Amino, Hydroxy, Cyano, COOH, $C_1$-$C_6$-Alkyl, $C_1$-$C_6$-Acyl, $C_1$-$C_6$-Alkoxy, N-($C_1$-$C_6$-Alkyl)$_2$ oder NH-($C_1$-$C_6$-Alkyl) substituiert sein kann,

$R^5$, $R^6$ unabhängig voneinander ein Wasserstoff oder ein $C_1$-$C_6$-Alkyl, und

$X^1$ ein Stickstoff, wenn $X^2$ und $X^3$ eine CH-Gruppe sind, oder

$X^2$ ein Stickstoff, wenn $X^1$ und $X^3$ eine CH-Gruppe sind, oder

$X^3$ ein Stickstoff, wenn $X^1$ und $X^2$ eine CH-Gruppe sind,

bedeuten,
sowie deren Stereoisomere, Diastereomere, Enantiomere und Salze, die die bekannten Nachteile überwinden und verbesserte Eigenschaften, d.h. gute Wirksamkeit, gute Löslichkeit und Stabilität, aufweisen.

Die erfindungsgemäßen Verbindungen inhibieren die lösliche Adenylatzyklase und verhindern so die Kapazitation des Spermiums und dienen somit der männlichen Fertilitätskontrolle.

Unter dem $C_1$-$C_6$- bzw. dem $C_1$-$C_4$-Alkylrest in $R^1$ - $R^6$ ist jeweils ein geradkettiger oder verzweigter Alkylrest, wie beispielsweise Methyl, Ethyl, Propyl, Isopropyl, Butyl, Isobutyl, sek. Butyl, tert. Butyl, Pentyl, Isopentyl und Hexyl, zu verstehen. Die Alkylreste können gegebenenfalls auch eine oder mehrere Doppelbindungen enthalten, beispielsweise seinen genannt Ethenyl, 1-Propenyl, 2-Propenyl.
Die Alkylreste können gegebenenfalls zusätzlich ein- oder mehrfach, gleich oder verschieden durch Halogen substituiert sein.

Unter dem $C_1$-$C_6$- bzw. dem $C_1$-$C_4$-Alkoxyrest in $R^1$ - $R^4$ ist jeweils ein geradkettiger oder verzweigter Alkoxyrest, wie beispielsweise Methoxy-, Ethoxy-, n-Propoxy-, iso-Propoxy-, n-Butoxy-, sec-Butoxy-, iso-Butoxy-, tert. Butyloxy-, Pentoxy-, iso-Pentoxy- und Hexoxy-, zu verstehen.
Die Alkoxyreste können gegebenenfalls zusätzlich ein- oder mehrfach, gleich oder verschieden durch Halogen substituiert sein.

Unter dem $C_1$-$C_6$- bzw. dem $C_1$-$C_4$-Acylrest in $R^1$ - $R^4$ ist jeweils ein geradkettiger oder verzweigter Rest, wie beispielsweise Formyl, Acetyl, Propionyl, Butyroyl, iso-Butyroyl, Valeroyl und Benzoyl zu verstehen.
Die Acylreste können gegebenenfalls zusätzlich ein- oder mehrfach, gleich oder verschieden durch Halogen substituiert sein.

Unter einem $C_1$-$C_6$- bzw. dem $C_1$-$C_4$-Alkylenrest in $R^3$ ist jeweils ein geradkettiger oder verzweigter Rest zu verstehen, wobei beispielsweise folgende Reste gemeint sind: Methylen, Ethylen, Propylen, Butylen, Pentylen, Hexylen.

Unter $C_3$-$C_6$-Cycloalkyl in $R^1$ - $R^4$ sind monocyclische Alkylringe wie Cyclopropyl, Cyclobutyl, Cyclopentyl und Cyclohexyl zu verstehen.

Die Cycloalklyreste können anstelle der Kohlenstoffatome auch eine $SO_2$-Gruppe oder eine oder zwei Ketogruppen oder ein oder mehrere Heteroatome, wie Sauerstoff, Schwefel und/oder Stickstoff enthalten. Bevorzugt sind solche Heterocycloalkyle mit 3 bis 6 Ringatomen, wie beispielsweise Aziridinyl, Oxetanyl, Tetrahydrofuranyl, Pyrrolidinyl, Morpholinyl, Thiomorpholinyl, Piperidinyl, Tetrahydropyranyl, Dioxanyl und Piperazinyl. Unter den Ringsystemen, bei denen gegebenenfalls ein- oder mehrere mögliche Doppelbindungen im Ring enthalten sein können, sind zum Beispiel Cycloalkenyle wie Cyclopropenyl, Cyclobutenyl, Cyclopentenyl, Cyclopentadienyl, Cyclohexenyl, Cycloheptenyl zu verstehen, wobei die Anknüpfung sowohl an der Doppelbindung wie auch an den Einfachbindungen erfolgen kann.

Unter Halogen ist jeweils Fluor, Chlor, Brom oder Jod zu verstehen.

Der Arylrest in $R^1$ - $R^4$ umfasst jeweils 6 - 12 Kohlenstoffatome und kann beispielsweise benzokondensiert sein. Beispielsweise genannt seien: Phenyl, Tropyl, Cyclooctadienyl, Indenyl, Naphthyl, Biphenyl, Florenyl, Anthracenyl etc. Der Heteroarylrest in $R^1$ - $R^4$ umfasst jeweils 5 bis maximal 16 Ringatome und kann anstelle des Kohlenstoffs ein- oder mehrere, gleiche oder verschiedene Heteroatome, wie Sauerstoff, Schwefel oder Stickstoff im Ring enthalten, und kann mono-, bi- oder tricyclisch sein und kann zusätzlich jeweils benzokondensiert sein.

Beispielsweise seien genannt:

Thienyl, Furanyl, Pyrrolyl, Oxazolyl, Imidazolyl, Pyrazolyl, Isoxazolyl, Isothiazolyl, Oxadiazolyl, Triazolyl, Thiadiazolyl, etc. und Benzoderivate davon, wie z.B. Benzofuranyl, Benzothienyl, Benzooxazolyl, Benzimdazolyl, Indazolyl,

Indolyl, Isoindolyl, etc; oder Pyridazinyl, Pyrimidinyl, Pyrazinyl, Triazinyl, etc. und Benzoderivate davon wie z.B. Chinolyl, Isochinolyl, etc; oder Azocinyl, Indolizinyl, Purinyl, etc. und Benzoderivate davon; oder Chinolinyl, Isochinolinyl, Cinnolinyl, Phthalazinyl, Chinazolinyl, Chinoxalinyl, Naphthyridinyl, Pteridinyl, Carbazolyl, Acridinyl, Phenazinyl, Phenothiazinyl, Phenoxazinyl, Xanthenyl, Oxepinyl, etc.

Der Heteroarylrest kann jeweils benzokondensiert sein. Beispielsweise seien als 5-Ringheteroaromaten genannt: Thiophen, Furan, Oxazol, Thiazol, Imidazol, Pyrazol und Benzoderivate davon und als 6-Ring-Heteroaromaten Pyridin, Pyrimidin, Triazin, Chinolin, Isochinolin und Benzoderivate.

Unter Heteroatomen sind Sauerstoff-, Stickstoff- oder Schwefel-Atome zu verstehen.

Ist eine saure Funktion enthalten, sind als Salze die physiologisch verträglichen Salze organischer und anorganischer Basen geeignet, wie beispielsweise die gut löslichen Alkali- und Erdalkalisalze sowie N-Methyl-glukamin, Dimethylglukamin, Ethyl-glukamin, Lysin, 1,6-Hexadiamin, Ethanolamin, Glukosamin, Sarkosin, Serinol, Tris-hydroxy-methyl-amino-methan, Aminopropandiol, Sovak-Base, 1-Amino-2,3,4-butantriol.

Ist eine basische Funktion enthalten, sind die physiologisch verträglichen Salze organischer und anorganischer Säuren geeignet wie Salzsäure, Schwefelsäure, Phosphorsäure, Zitronensäure, Weinsäure u.a.

Besonders bevorzugt sind solche Verbindungen der allgemeinen Formel (I), wobei

A ein Phenyl-, Naphthyl- oder $C_5$-$C_{12}$-Heteroarylrest, der gegebenenfalls ein- oder mehrfach mit $R^1$ und/oder $R^2$ substituiert sein kann,

$R^1$ ein Wasserstoff, Halogen, Amino,
eine -$S(O)_p$-$C_1$-$C_6$-Alkylgruppe, wobei p für 0 - 2 steht, ein $SO_2NH_2$, -$SO_2NH$-$C_1$-$C_6$-Alkylgruppe, -NH-$SO_2$-$C_1$-$C_6$- Alkylgruppe, $C_1$-$C_6$-Acyl-, NH-CO-$NH_2$, -O-CO-NH($C_1$-$C_6$-Alkyl), -O- CO-N($C_1$-$C_6$-Alkyl)$_2$, NH-CO-$C_1$-$C_6$-Alkylrest, Hydroxy, Cyano, O- CO-($C_1$-$C_6$-Alkyl), $CO_2$-($C_1$-$C_6$-Alkyl), CO-NH-($C_1$-$C_6$-Alkyl), CO- N ($C_1$-$C_6$-Alkyl)$_2$, CO-NH($C_5$-$C_{12}$-Heteroaryl), NH-($C_1$-$C_6$-Alkyl), N- ($C_1$-$C_6$-Alkyl)$_2$, oder
ein $C_1$-$C_6$-Alkyl,
welches gegebenenfalls ein- oder mehrfach, gleich oder verschieden substituiert sein kann mit COOH, CO-$NH_2$, CO- NH-($C_1$-$C_6$-Alkyl), CO-N($C_1$-$C_6$-Alkyl)$_2$, $C_1$-$C_6$-Acyl, $C_1$-$C_6$- Alkoxy, Hydroxy, Cyano, $CO_2$-($C_1$-$C_6$-Alkyl), N-($C_1$-$C_6$- Alkyl)$_2$, $C_5$-$C_{12}$-Heteroaryl,
welches gegebenenfalls mehrfach substituiert ist, $C_6$-$C_{12}$-Aryl,
welches gegebenenfalls mehrfach substituiert ist,
oder
ein $C_1$-$C_6$-Alkoxy,
welches gegebenenfalls ein- oder mehrfach, gleich oder verschieden substituiert sein kann mit Hydroxy, Cyano, $CO_2$-($C_1$-$C_6$-Alkyl), N-($C_1$-$C_6$-Alkyl)$_2$, NH-$C_3$-$C_6$-Cycloalkyl, COOH, CO-$NH_2$, CO-NH($C_1$-$C_6$-Alkyl) oder mit CO-N($C_1$-$C_6$- Alkyl)$_2$,
ein O-$C_6$-$C_{12}$-Aryl,
welches gegebenenfalls ein- oder mehrfach, gleich oder verschieden mit Halogen, Hydroxy, Cyano, COOH, CO- $NH_2$, $C_1$-$C_6$-Alkyl, $C_3$-$C_6$-Cycloalkyl, $C_1$-$C_6$-Acyl oder $C_1$-$C_6$- Alkoxy substituiert sein kann, oder
ein O-$C_5$-$C_{12}$-Heteroaryl,
welches gegebenenfalls ein- oder mehrfach, gleich oder verschieden mit Halogen, Hydroxy, Cyano, COOH, CO- $NH_2$, $C_1$-$C_6$-Alkyl, $C_3$-$C_6$-Cycloalkyl, $C_1$-$C_6$-Acyl oder $C_1$-$C_6$- Alkoxy substituiert sein kann, oder
ein $C_6$-$C_{12}$-Aryl,
welches gegebenenfalls ein- oder mehrfach, gleich oder verschieden substituiert sein kann mit Halogen, $C_1$-$C_6$-Alkyl, $C_3$-$C_6$-Cycloalkyl, $C_1$-$C_6$-Acyl, $C_1$-$C_6$-Alkoxy, $C_6$-$C_{12}$-Aryl, $C_5$- $C_{12}$-Heteroaryl, Hydroxy, $CH_2$-OH, Cyano, $CH_2$-CN, Amino, $CO_2$-($C_1$-$C_6$-Alkyl), N-($C_1$-$C_6$-Alkyl)$_2$, $NHSO_2CH_3$, $SO_2NH_2$, $SO_2NH$($C_1$-$C_6$-Alkyl), $SO_2N$($C_1$-$C_6$-Alkyl)$_2$, COOH, CO-$NH_2$, CO-NH-($C_1$-$C_6$-Alkyl), CO-N($C_1$-$C_6$-Alkyl)$_2$, CO-NH($C_5$-$C_{12}$- Heteroaryl), NH-CO($C_1$-$C_6$-Alkyl), $CH_2$-NH-CO($C_1$-$C_6$-Alkyl), NH-CO($C_5$-$C_{12}$-Heteroaryl) oder ein -$S(O)_r$-$CH_3$, wobei r für 0 - 2 steht, oder
zwei benachbarte Positionen durch -O-$CH_2$-O- oder -O-C($CH_3$)$_2$- O- substituiert sein können, oder
ein $C_5$-$C_{16}$-Heteroaryl,
welches gegebenenfalls ein- oder mehrfach, gleich oder verschieden substituiert sein kann mit Halogen, $C_1$-$C_6$-Alkyl, $C_3$-$C_6$-Cycloalkyl, $C_1$-$C_6$-Acyl, $C_1$-$C_6$-Alkoxy, $C_6$-$C_{12}$-Aryl, $C_5$- $C_{12}$-Heteroaryl, Hydroxy, $CH_2$-OH, Cyano, $CH_2$-CN, Amino, $CO_2$-($C_1$-$C_6$-Alkyl), N-($C_1$-$C_6$-Alkyl)$_2$, $NHSO_2CH_3$, $SO_2NH_2$, $SO_2NH$($C_1$-$C_6$-Alkyl), $SO_2N$($C_1$-$C_6$-Alkyl)$_2$, COOH, CO-$NH_2$, CO-NH-($C_1$-$C_6$-Alkyl), CO-N($C_1$-$C_6$-Alkyl)$_2$, CO-NH($C_5$-$C_{12}$- Heteroaryl), NH-CO($C_1$-$C_6$-Alkyl), $CH_2$-NH-CO($C_1$-$C_6$-Alkyl), NH-CO($C_5$-$C_{12}$-Heteroaryl) oder ein -$S(O)_r$-$CH_3$, wobei r für 0 - 2 steht, oder
ein $C_3$-$C_6$-Cycloalkyl,

welches gegebenenfalls ein- oder mehrfach, gleich oder verschieden mit Halogen, $C_1$-$C_6$-Alkyl, Hydroxy, Cyano, $CO_2$-($C_1$-$C_6$-Alkyl), $C_1$-$C_6$-Acyl, N-($C_1$-$C_6$-Alkyl)$_2$, COOH, CO- $NH_2$, CO-NH($C_1$-$C_6$-Alkyl), CO-N ($C_1$-$C_6$-Alkyl)$_2$ oder $C_1$-$C_6$- Alkoxy substituiert sein kann,

$R^2$ ein Wasserstoff, Halogen, Amino, eine -S(O)p-$C_1$-$C_6$-Alkylgruppe, wobei p für 0 - 2 steht, ein $SO_2NH_2$, eine -$SO_2$NH-$C_1$-$C_6$-Alkylgruppe, eine -NH-$SO_2$-$C_1$- $C_6$-Alkylgruppe, $C_1$-$C_6$-Acyl-, NH-CO-$NH_2$, -O-CO-NH($C_1$-$C_6$-Alkyl), -O-CO-N($C_1$-$C_6$-Alkyl)$_2$ oder ein NH-CO-$C_1$-$C_6$-Alkylrest, Hydroxy, Cyano, O-CO-($C_1$-$C_6$-Alkyl), $CO_2$-($C_1$-$C_6$-Alkyl), CO-NH-($C_1$-$C_6$- Alkyl), CO-N($C_1$-$C_6$-Alkyl)$_2$, CO-NH($C_5$-$C_{12}$-Heteroaryl), NH-($C_1$-$C_6$- Alkyl), N-($C_1$-$C_6$-Alkyl)$_2$, oder ein $C_1$-$C_6$-Alkyl,
welches gegebenenfalls ein- oder mehrfach, gleich oder verschieden substituiert sein kann mit COOH, CO-$NH_2$, CO- NH($C_1$-$C_6$-Alkyl) oder mit CO-N($C_1$-$C_6$-Alkyl)$_2$, $C_1$-$C_6$-Acyl, $C_1$-$C_6$-Alkoxy, Hydroxy, Cyano, $CO_2$-($C_1$-$C_6$-Alkyl), N-($C_1$-$C_6$- Alkyl)$_2$, $C_5$-$C_{12}$-Heteroaryl,
welches gegebenenfalls mehrfach substituiert ist, $C_6$-$C_{12}$-Aryl, welches gegebenenfalls mehrfach substituiert ist, oder
ein $C_1$-$C_6$-Alkoxy,
welches gegebenenfalls ein- oder mehrfach, gleich oder verschieden substituiert sein kann mit Hydroxy, Cyano, $CO_2$-($C_1$-$C_6$-Alkyl), N-($C_1$-$C_6$-Alkyl)$_2$, NH-$C_3$-$C_6$-Cycloalkyl, COOH, CO-$NH_2$, CO-NH($C_1$-$C_6$-Alkyl) oder mit CO-N($C_1$-$C_6$- Alkyl)$_2$,
ein O-$C_6$-$C_{12}$-Aryl,
welches gegebenenfalls ein- oder mehrfach, gleich oder verschieden mit Halogen, Hydroxy, Cyano, COOH, CO- $NH_2$, $C_1$-$C_6$-Alkyl, $C_3$-$C_6$-Cycloalkyl, $C_1$-$C_6$-Acyl oder $C_1$-$C_6$- Alkoxy substituiert sein kann, oder
ein O-$C_5$-$C_{12}$-Heteroaryl,
welches gegebenenfalls ein- oder mehrfach, gleich oder verschieden mit Halogen, Hydroxy, Cyano, COOH, CO- $NH_2$, $C_1$-$C_6$-Alkyl, $C_3$-$C_6$-Cycloalkyl, $C_1$-$C_6$-Acyl oder $C_1$-$C_6$- Alkoxy substituiert sein kann, oder
ein $C_6$-$C_{12}$-Aryl,
welches gegebenenfalls ein- oder mehrfach, gleich oder verschieden substituiert sein kann mit Halogen, $C_1$-$C_6$-Alkyl, $C_3$-$C_6$-Cycloalkyl, $C_1$-$C_6$-Acyl, $C_1$-$C_6$-Alkoxy, $C_6$-$C_{12}$-Aryl, $C_5$- $C_{12}$-Heteroaryl, Hydroxy, $CH_2$-OH, Cyano, $CH_2$-CN, Amino, $CO_2$-($C_1$-$C_6$-Alkyl), N-($C_1$-$C_6$-Alkyl)$_2$, $NHSO_2CH_3$, $SO_2NH_2$, $SO_2$NH($C_1$-$C_6$-Alkyl), $SO_2$N($C_1$-$C_6$-Alkyl)$_2$, COOH, CO-$NH_2$, CO-NH($C_1$-$C_6$-Alkyl), CO-N($C_1$-$C_6$-Alkyl)$_2$, CO-NH($C_5$-$C_{12}$- Heteroaryl), NH-CO($C_1$-$C_6$-Alkyl), $CH_2$-NH-CO($C_1$-$C_6$-Alkyl), NH-CO($C_5$-$C_{12}$-Heteroaryl) oder ein -S(O)$_r$-$CH_3$, wobei r für 0 - 2 steht, oder
zwei benachbarte Positionen durch -O-$CH_2$-O- oder -O-C($CH_3$)$_2$- O- substituiert sein können, oder
ein $C_5$-$C_{16}$-Heteroaryl,
welches gegebenenfalls ein- oder mehrfach, gleich oder verschieden substituiert sein kann mit Halogen, $C_1$-$C_6$-Alkyl, $C_3$-$C_6$-Cycloalkyl, $C_1$-$C_6$-Acyl, $C_1$-$C_6$-Alkoxy, $C_6$-$C_{12}$-Aryl, $C_5$- $C_{12}$-Heteroaryl, Hydroxy, $CH_2$-OH, Cyano, $CH_2$-CN, Amino, $CO_2$-($C_1$-$C_6$-Alkyl), N-($C_1$-$C_6$-Alkyl)$_2$, $NHSO_2CH_3$, $SO_2NH_2$, $SO_2$NH($C_1$-$C_6$-Alkyl), $SO_2$N($C_1$-$C_6$-Alkyl)$_2$, COOH, CO-$NH_2$, CO-NH-($C_1$-$C_6$-Alkyl), CO-N($C_1$-$C_6$-Alkyl)$_2$, CO-NH($C_5$-$C_{12}$- Heteroaryl), NH-CO($C_1$-$C_6$-Alkyl), $CH_2$-NH-CO($C_1$-$C_6$-Alkyl), NH-CO($C_5$-$C_{12}$-Heteroaryl) oder ein -S(O)$_r$-$CH_3$, wobei r für 0 - 2 steht, oder
ein $C_3$-$C_6$-Cycloalkyl,
welches gegebenenfalls ein- oder mehrfach, gleich oder verschieden mit Halogen, $C_1$-$C_6$-Alkyl, Hydroxy, Cyano, $CO_2$-($C_1$-$C_6$-Alkyl), $C_1$-$C_6$-Acyl, N-($C_1$-$C_6$-Alkyl)$_2$, COOH, CO- $NH_2$, CO-NH($C_1$-$C_6$-Alkyl), CO-N ($C_1$-$C_6$-Alkyl)$_2$ oder $C_1$-$C_6$- Alkoxy substituiert sein kann,

$R^3$ ein $C_6$-$C_{12}$-Aryl,
welches gegebenenfalls ein- oder mehrfach, gleich oder verschieden substituiert sein kann mit Halogen, $C_1$-$C_6$-Alkyl, $C_1$-$C_6$-Alkoxy, Hydroxy, $C_1$-$C_6$-Alkylen-OH, $C_1$-$C_6$-Alkylen- $NR^5R^6$, Cyano, $CO_2$-($C_1$-$C_6$-Alkyl), N-($C_1$-$C_6$-Alkyl)$_2$, CO-NH- ($C_1$-$C_6$-Alkyl), CO-NH-($C_1$-$C_6$-Alkylen-OH), CO-NH-($C_1$-$C_6$- Alkylen-$NR^5R^6$), CO-N($C_1$-$C_6$-Alkyl)$_2$ oder $C_1$-$C_6$-Acyl,
welches gegebenenfalls ein- oder mehrfach substituiert ist,
oder
mit zwei benachbarten Positionen durch -O-$CH_2$-O-, -O- $CH_2$-$CH_2$-O-, oder -O-C($CH_3$)$_2$-O- substituiert sein kann, oder
ein $C_5$-$C_{16}$-Heteroaryl,
welches gegebenenfalls ein- oder mehrfach, gleich oder verschieden substituiert sein kann mit Halogen, $C_1$-$C_6$-Alkyl, $C_1$-$C_6$-Acyl, $C_1$-$C_6$-Alkoxy, Hydroxy, Cyano, $CO_2$-($C_1$-$C_6$- Alkyl), N-($C_1$-$C_6$-Alkyl)$_2$, CO-NH-($C_1$-$C_6$-Alkylen-OH), CO-NH- ($C_1$-$C_6$-Alkylen-$NR^5R^6$), CO-NH($C_1$-$C_6$-Alkyl) oder CO-N($C_1$- $C_6$-Alkyl)$_2$ oder

ein $C_3$-$C_6$-Cycloalkyl, welches gegebenenfalls ein- oder mehrfach, gleich oder verschieden mit Halogen, $CF_3$, Hydroxy, Cyano, $CO_2$-($C_1$-$C_6$-Alkyl), $C_1$-$C_6$-Alkyl, $C_1$-$C_6$-Acyl, N-($C_1$-$C_6$-Alkyl)$_2$, CO-NH-($C_1$-$C_6$-Alkylen-OH), CO-NH-($C_1$-$C_6$-Alkylen-$NR^5R^6$), CO- NH($C_1$-$C_6$-Alkyl) oder CO-N($C_1$-$C_6$-Alkyl)$_2$ oder $C_1$-$C_6$-Alkoxy substituiert sein kann,

$R^4$ ein Wasserstoff, ein $C_1$-$C_6$-Alkyl, welches gegebenenfalls ein- oder mehrfach, gleich oder verschieden substituiert sein kann mit einer -S(O)$_p$-$C_1$-$C_6$- Alkylgruppe oder einer -S(O)$_p$-$C_3$-$C_6$-Cycloalkylgruppe, wobei p für 0 - 2 steht, Hydroxy, Cyano, COOH, CO-$NH_2$, $SO_2NH_2$, $SO_2NH$-($C_1$-$C_6$-Alkyl), $C_3$-$C_6$-Cycloalkyl, $C_1$-$C_6$-Acyl, $C_1$-$C_6$-Alkoxy, $CO_2$-($C_1$-$C_6$-Alkyl), N-($C_1$-$C_6$-Alkyl)$_2$, NH-($C_1$-$C_6$-Alkyl), NH-($C_3$-$C_6$-Cycloalkyl), CO-NH($C_1$-$C_6$-Alkyl) oder mit CO-N($C_1$-$C_6$-Alkyl)$_2$, $C_6$-$C_{12}$-Aryl, welches gegebenenfalls mehrfach substituiert ist, $C_5$-$C_{12}$-Heteroaryl, welches gegebenenfalls mehrfach substituiert ist, oder ein $C_3$-$C_6$-Cycloalkyl, welches gegebenenfalls ein- oder mehrfach, gleich oder verschieden mit Hydroxy, $CH_2$-OH, Cyano, COOH, CO-$NH_2$, $SO_2NH_2$, $C_1$-$C_6$-Alkyl, $C_1$-$C_6$-Acyl oder $C_1$-$C_6$-Alkoxy, oder ein $C_6$-$C_{12}$-Aryl, welches gegebenenfalls ein- oder mehrfach, gleich oder verschieden mit Halogen, Hydroxy, $C_1$-$C_6$-Alkyl, $C_1$-$C_6$-Acyl, $C_1$-$C_6$- Alkoxy, N-($C_1$-$C_6$-Alkyl)$_2$, NH-($C_1$-$C_6$-Alkyl) oder Cyano substituiert sein kann, oder ein $C_5$-$C_{12}$-Heteroaryl, welches gegebenenfalls ein- oder mehrfach, gleich oder verschieden mit Halogen, Amino, Hydroxy, Cyano, COOH, $C_1$-$C_6$-Alkyl, $C_1$-$C_6$-Acyl, $C_1$-$C_6$-Alkoxy, N-($C_1$-$C_6$-Alkyl)$_2$ oder NH-($C_1$-$C_6$-Alkyl) substituiert sein kann,

$R^5$, $R^6$ unabhängig voneinander Wasserstoff oder ein $C_1$-$C_6$-Alkyl, und

$X^1$ ein Stickstoff, wenn $X^2$ und $X^3$ eine CH-Gruppe sind, oder

$X^2$ ein Stickstoff, wenn $X^1$ und $X^3$ eine CH-Gruppe sind, oder

$X^3$ ein Stickstoff, wenn $X^1$ und $X^2$ eine CH-Gruppe sind,

bedeuten, sowie deren Stereoisomere, Diastereomere, Enantiomere und Salze.

Ebenfalls bevorzugt sind solche Verbindungen der allgemeinen Formel I, wobei

A ein Phenyl oder ein monocyclischer $C_5$-$C_7$-Heteroarylrest, der gegebenenfalls ein- oder mehrfach mit $R^1$ und/oder $R^2$ substituiert sein kann,

$R^1$ ein Wasserstoff, Halogen, Amino, eine -S(O)$_p$-$C_1$-$C_6$-Alkylgruppe, wobei p für 0 - 2 steht, ein $SO_2NH_2$, eine -$SO_2NH$-$C_1$-$C_6$-Alkylgruppe, eine -NH-$SO_2$-$C_1$- $C_6$-Alkylgruppe, $C_1$-$C_6$-Acyl-, NH-CO-$NH_2$, -O-CO-NH($C_1$-$C_6$-Alkyl), -O-CO-N($C_1$-$C_6$-Alkyl)$_2$, NH-CO-$C_1$-$C_6$-Alkylrest, Hydroxy, Cyano, O-CO-($C_1$-$C_6$-Alkyl), $CO_2$-($C_1$-$C_6$-Alkyl), CO-NH-($C_1$-$C_6$-Alkyl), CO-N($C_1$-$C_6$-Alkyl)$_2$, CO-NH($C_5$-$C_{12}$-Heteroaryl), NH-($C_1$-$C_6$-Alkyl), N- ($C_1$-$C_6$-Alkyl)$_2$, oder ein $C_1$-$C_6$-Alkyl, welches gegebenenfalls ein- oder mehrfach, gleich oder verschieden substituiert sein kann mit COOH, CO-$NH_2$, CO- NH-($C_1$-$C_6$-Alkyl), CO-N($C_1$-$C_6$-Alkyl)$_2$, $C_1$-$C_6$-Acyl, $C_1$-$C_6$- Alkoxy, Hydroxy, Cyano, $CO_2$-($C_1$-$C_6$-Alkyl), N-($C_1$-$C_6$- Alkyl)$_2$, $C_5$-$C_{12}$-Heteroaryl, welches gegebenenfalls mehrfach substituiert ist, $C_6$-$C_{12}$-Aryl, welches gegebenenfalls mehrfach substituiert ist, oder ein $C_1$-$C_6$-Alkoxy, welches gegebenenfalls ein- oder mehrfach, gleich oder verschieden substituiert sein kann mit Hydroxy, Cyano, $CO_2$-($C_1$-$C_6$-Alkyl), N-($C_1$-$C_6$-Alkyl)$_2$, NH-$C_3$-$C_6$-Cycloalkyl, COOH, CO-$NH_2$, CO-NH($C_1$-$C_6$-Alkyl) oder mit CO-N($C_1$-$C_6$- Alkyl)$_2$, ein O-$C_6$-$C_{12}$-Aryl, welches gegebenenfalls ein- oder mehrfach, gleich oder verschieden mit Halogen, Hydroxy, Cyano, COOH, CO- $NH_2$, $C_1$-$C_6$-Alkyl, $C_3$-$C_6$-Cycloalkyl, $C_1$-$C_6$-Acyl oder $C_1$-$C_6$- Alkoxy substituiert sein kann, oder

ein O-$C_5$-$C_{12}$-Heteroaryl,
welches gegebenenfalls ein- oder mehrfach, gleich oder verschieden mit Halogen, Hydroxy, Cyano, COOH, CO- $NH_2$, $C_1$-$C_6$-Alkyl, $C_3$-$C_6$-Cycloalkyl, $C_1$-$C_6$-Acyl oder $C_1$-$C_6$- Alkoxy substituiert sein kann, oder
ein $C_6$-$C_{12}$-Aryl,
welches gegebenenfalls ein- oder mehrfach, gleich oder verschieden substituiert sein kann mit Halogen, $C_1$-$C_6$-Alkyl, $C_3$-$C_6$-Cycloalkyl, $C_1$-$C_6$-Acyl, $C_1$-$C_6$-Alkoxy, $C_6$-$C_{12}$-Aryl, $C_5$- $C_{12}$-Heteroaryl, Hydroxy, $CH_2$-OH, Cyano, $CH_2$-CN, Amino, $CO_2$-($C_1$-$C_6$-Alkyl), N-($C_1$-$C_6$-Alkyl)$_2$, $NHSO_2CH_3$, $SO_2NH_2$, $SO_2NH$($C_1$-$C_6$-Alkyl), $SO_2N$($C_1$-$C_6$-Alkyl)$_2$, COOH, CO-$NH_2$, CO-NH-($C_1$-$C_6$-Alkyl), CO-N($C_1$-$C_6$-Alkyl)$_2$, CO-NH($C_5$-$C_{12}$- Heteroaryl), NH-CO($C_1$-$C_6$-Alkyl), $CH_2$-NH-CO($C_1$-$C_6$-Alkyl), NH-CO($C_5$-$C_{12}$-Heteroaryl) oder ein -S(O)$_r$-$CH_3$, wobei r für 0 - 2 steht,
oder zwei benachbarte Positionen durch -O-$CH_2$-O- oder -O-C($CH_3$)$_2$- O- substituiert sein können, oder
ein $C_5$-$C_{16}$-Heteroaryl,
welches gegebenenfalls ein- oder mehrfach, gleich oder verschieden substituiert sein kann mit Halogen, $C_1$-$C_6$-Alkyl, $C_3$-$C_6$-Cycloalkyl, $C_1$-$C_6$-Acyl, $C_1$-$C_6$-Alkoxy, $C_6$-$C_{12}$-Aryl, $C_5$- $C_{12}$-Heteroaryl, Hydroxy, $CH_2$-OH, Cyano, $CH_2$-CN, Amino, $CO_2$-($C_1$-$C_6$-Alkyl), N-($C_1$-$C_6$-Alkyl)$_2$, $NHSO_2CH_3$, $SO_2NH_2$, $SO_2NH$($C_1$-$C_6$-Alkyl), $SO_2N$($C_1$-$C_6$-Alkyl)$_2$, COOH, CO-$NH_2$, CO-NH-($C_1$-$C_6$-Alkyl), CO-N($C_1$-$C_6$-Alkyl)$_2$, CO-NH($C_5$-$C_{12}$- Heteroaryl), NH-CO($C_1$-$C_6$-Alkyl), $CH_2$-NH-CO($C_1$-$C_6$-Alkyl), NH-CO($C_5$-$C_{12}$-Heteroaryl) oder ein -S(O)$_r$-$CH_3$, wobei r für 0 - 2 steht, oder
ein $C_3$-$C_6$-Cycloalkyl,
welches gegebenenfalls ein- oder mehrfach, gleich oder verschieden mit Halogen, $C_1$-$C_6$-Alkyl, Hydroxy, Cyano, $CO_2$-($C_1$-$C_6$-Alkyl), $C_1$-$C_6$-Acyl, N-($C_1$-$C_6$-Alkyl)$_2$, COOH, CO- $NH_2$, CO-NH($C_1$-$C_6$-Alkyl), CO-N ($C_1$-$C_6$-Alkyl)$_2$ oder $C_1$-$C_6$- Alkoxy substituiert sein kann,

$R^2$ ein Wasserstoff, Halogen, Amino, eine -S(O)$_p$-$C_1$-$C_6$-Alkylgruppe, wobei p für 0 - 2 steht,
ein $SO_2NH_2$, eine -$SO_2NH$-$C_1$-$C_6$-Alkylgruppe, eine -NH-$SO_2$-$C_1$- $C_6$-Alkylgruppe, $C_1$-$C_6$-Acyl-, NH-CO-$NH_2$, -O-CO-NH($C_1$-$C_6$-Alkyl), -O-CO-N($C_1$-$C_6$-Alkyl)$_2$ oder NH-CO-$C_1$-$C_6$-Alkylrest, Hydroxy, Cyano, O-CO-($C_1$-$C_6$-Alkyl), $CO_2$-($C_1$-$C_6$-Alkyl), CO-NH-($C_1$-$C_6$- Alkyl), CO-N($C_1$-$C_6$-Alkyl)$_2$, CO-NH($C_5$-$C_{12}$-Heteroaryl), NH-($C_1$-$C_6$- Alkyl), N-($C_1$-$C_6$-Alkyl)$_2$, oder ein $C_1$-$C_6$-Alkyl,
welches gegebenenfalls ein- oder mehrfach, gleich oder verschieden substituiert sein kann mit COOH, CO-$NH_2$, CO- NH($C_1$-$C_6$-Alkyl) oder mit CO-N($C_1$-$C_6$-Alkyl)$_2$, $C_1$-$C_6$-Acyl, $C_1$-$C_6$-Alkoxy, Hydroxy, Cyano, $CO_2$-($C_1$-$C_6$-Alkyl), N-($C_1$-$C_6$- Alkyl)$_2$, $C_5$-$C_{12}$-Heteroaryl,
welches gegebenenfalls mehrfach substituiert ist, $C_6$-$C_{12}$-Aryl,
welches gegebenenfalls mehrfach substituiert ist,
oder
ein $C_1$-$C_6$-Alkoxy,
welches gegebenenfalls ein- oder mehrfach, gleich oder verschieden substituiert sein kann mit Hydroxy, Cyano, $CO_2$-($C_1$-$C_6$-Alkyl), N-($C_1$-$C_6$-Alkyl)$_2$, NH-$C_3$-$C_6$-Cycloalkyl, COOH, CO-$NH_2$, CO-NH($C_1$-$C_6$-Alkyl) oder mit CO-N($C_1$-$C_6$- Alkyl)$_2$,
ein O-$C_6$-$C_{12}$-Aryl,
welches gegebenenfalls ein- oder mehrfach, gleich oder verschieden mit Halogen, Hydroxy, Cyano, COOH, CO- $NH_2$, $C_1$-$C_6$-Alkyl, $C_3$-$C_6$-Cycloalkyl, $C_1$-$C_6$-Acyl oder $C_1$-$C_6$- Alkoxy substituiert sein kann, oder
ein O-$C_5$-$C_{12}$-Heteroaryl,
welches gegebenenfalls ein- oder mehrfach, gleich oder verschieden mit Halogen, Hydroxy, Cyano, COOH, CO- $NH_2$, $C_1$-$C_6$-Alkyl, $C_3$-$C_6$-Cycloalkyl, $C_1$-$C_6$-Acyl oder $C_1$-$C_6$- Alkoxy substituiert sein kann, oder
ein $C_6$-$C_{12}$-Aryl,
welches gegebenenfalls ein- oder mehrfach, gleich oder verschieden substituiert sein kann mit Halogen, $C_1$-$C_6$-Alkyl, $C_3$-$C_6$-Cycloalkyl, $C_1$-$C_6$-Acyl, $C_1$-$C_6$-Alkoxy, $C_6$-$C_{12}$-Aryl, $C_5$- $C_{12}$-Heteroaryl, Hydroxy, $CH_2$-OH, Cyano, $CH_2$-CN, Amino, $CO_2$-($C_1$-$C_6$-Alkyl), N-($C_1$-$C_6$-Alkyl)$_2$, $NHSO_2CH_3$, $SO_2NH_2$, $SO_2NH$($C_1$-$C_6$-Alkyl), $SO_2N$($C_1$-$C_6$-Alkyl)$_2$, COOH, CO-$NH_2$, CO-NH($C_1$-$C_6$-Alkyl), CO-N($C_1$-$C_6$-Alkyl)$_2$, CO-NH($C_5$-$C_{12}$- Heteroaryl), NH-CO($C_1$-$C_6$-Alkyl), $CH_2$-NH-CO($C_1$-$C_6$-Alkyl), NH-CO($C_5$-$C_{12}$-Heteroaryl) oder ein -S(O)$_r$-$CH_3$, wobei r für 0 - 2 steht, oder zwei benachbarte Positionen durch -O-$CH_2$-O- oder -O-C($CH_3$)$_2$- O- substituiert sein können, oder
ein $C_5$-$C_{16}$-Heteroaryl,
welches gegebenenfalls ein- oder mehrfach, gleich oder verschieden substituiert sein kann mit Halogen, $C_1$-$C_6$-Alkyl, $C_3$-$C_6$-Cycloalkyl, $C_1$-$C_6$-Acyl, $C_1$-$C_6$-Alkoxy, $C_6$-$C_{12}$-Aryl, $C_5$- $C_{12}$-Heteroaryl, Hydroxy, $CH_2$-OH, Cyano, $CH_2$-CN, Amino, $CO_2$-($C_1$-$C_6$-Alkyl), N-($C_1$-$C_6$-Alkyl)$_2$, $NHSO_2CH_3$, $SO_2NH_2$, $SO_2NH$($C_1$-$C_6$-Alkyl), $SO_2N$($C_1$-$C_6$-Alkyl)$_2$, COOH, CO-$NH_2$, CO-NH-($C_1$-$C_6$-Alkyl), CO-N($C_1$-$C_6$-Alkyl)$_2$, CO-NH($C_5$-$C_{12}$- Heteroaryl), NH-CO($C_1$-$C_6$-Alkyl), $CH_2$-NH-CO($C_1$-$C_6$-Alkyl), NH-CO($C_5$-$C_{12}$-Heteroaryl) oder ein -S(O)$_r$-$CH_3$, wo-

bei r für 0 - 2 steht, oder
ein $C_3$-$C_6$-Cycloalkyl,
welches gegebenenfalls ein- oder mehrfach, gleich oder verschieden mit Halogen, $C_1$-$C_6$-Alkyl, Hydroxy, Cyano, $CO_2$-($C_1$-$C_6$-Alkyl), $C_1$-$C_6$-Acyl, N-($C_1$-$C_6$-Alkyl)$_2$, COOH, CO- $NH_2$, CO-NH($C_1$-$C_6$-Alkyl), CO-N ($C_1$-$C_6$-Alkyl)$_2$ oder $C_1$-$C_6$- Alkoxy substituiert sein kann,

$R^3$ ein $C_6$-$C_{12}$-Aryl,
welches gegebenenfalls ein- oder mehrfach, gleich oder verschieden mit Halogen, $C_1$-$C_6$-Alkyl, $C_1$-$C_6$-Alkoxy, Hydroxy, $C_1$-$C_6$-Alkylen-OH, $C_1$-$C_6$-Alkylen-$NR^5R^6$, Cyano, $CO_2$-($C_1$-$C_6$-Alkyl), N-($C_1$-$C_6$-Alkyl)$_2$, CO-NH-($C_1$-$C_6$-Alkyl), CO-NH-($C_1$-$C_6$-Alkylen-OH), CO-NH-($C_1$-$C_6$-Alkylen-$NR^5R^6$), CO-N($C_2$-$C_6$-Alkyl)$_2$ oder $C_1$-$C_6$-Acyl,
welches gegebenenfalls ein- oder mehrfach substituiert ist,
oder
mit zwei benachbarten Positionen durch -O-$CH_2$-O-, -O- $CH_2$-$CH_2$-O-, oder -O-C($CH_3$)$_2$-O- substituiert sein kann, oder ein $C_5$-$C_{16}$-Heteroaryl,
welches gegebenenfalls ein- oder mehrfach, gleich oder verschieden substituiert sein kann mit Halogen, $C_1$-$C_6$-Alkyl, $C_1$-$C_6$-Acyl, $C_1$-$C_6$-Alkoxy, Hydroxy, Cyano, $CO_2$-($C_1$-$C_6$- Alkyl), N-($C_1$-$C_6$-Alkyl)$_2$, CO-NH-($C_1$-$C_6$-Alkylen-OH), CO- NH-($C_1$-$C_6$-Alkylen-$NR^5R^6$), CO-NH($C_1$-$C_6$-Alkyl) oder CO- N($C_1$-$C_6$-Alkyl)$_2$,
oder
ein $C_3$-$C_6$-Cycloalkyl,
welches gegebenenfalls ein- oder mehrfach, gleich oder verschieden substituiert sein kann mit Halogen, $CF_3$, Hydroxy, Cyano, $CO_2$-($C_1$-$C_6$-Alkyl), $C_1$-$C_6$-Alkyl, $C_1$-$C_6$-Acyl, N-($C_1$-$C_6$-Alkyl)$_2$, CO-NH-($C_1$-$C_6$-Alkylen-OH), CO-NH-($C_1$- $C_6$-Alkylen-$NR^5R^6$), CO-NH($C_1$-$C_6$-Alkyl) oder CO-N($C_1$-$C_6$- Alkyl)$_2$ oder $C_1$-$C_6$-Alkoxy,

$R^4$ ein Wasserstoff,
ein $C_1$-$C_6$-Alkyl,
welches gegebenenfalls ein- oder mehrfach, gleich oder verschieden substituiert sein kann mit einer -S (O)$_p$-$C_1$-$C_6$- Alkylgruppe oder einer -S(O)$_p$-$C_3$-$C_6$-Cycloalkylgruppe, wobei p für 0 - 2 steht, Hydroxy, Cyano, COOH, CO-$NH_2$, $SO_2NH_2$, $SO_2NH$-($C_1$-$C_6$-Alkyl), $C_3$-$C_6$-Cycloalkyl, $C_1$-$C_6$-Acyl, $C_1$-$C_6$-Alkoxy, $CO_2$-($C_1$-$C_6$-Alkyl), N-($C_1$-$C_6$-Alkyl)$_2$, NH-($C_1$-$C_6$-Alkyl), NH-($C_3$-$C_6$-Cycloalkyl), CO-NH($C_1$-$C_6$-Alkyl) oder mit CO-N($C_1$-$C_6$-Alkyl)$_2$, $C_6$-$C_{12}$-Aryl,
welches gegebenenfalls mehrfach substituiert ist, $C_5$-$C_{12}$-Heteroaryl,
welches gegebenenfalls mehrfach substituiert ist,
oder
ein $C_3$-$C_6$-Cycloalkyl,
welches gegebenenfalls ein- oder mehrfach, gleich oder verschieden mit Hydroxy, $CH_2$-OH, Cyano, COOH, CO-$NH_2$, $SO_2NH_2$, $C_1$-$C_6$-Alkyl, $C_1$-$C_6$-Acyl oder $C_1$-$C_6$-Alkoxy substituiert sein kann, oder
ein $C_6$-$C_{12}$-Aryl,
welches gegebenenfalls ein- oder mehrfach, gleich oder verschieden mit Halogen, Hydroxy, $C_1$-$C_6$-Alkyl, $C_1$-$C_6$-Acyl, $C_1$-$C_6$-Alkoxy, N-($C_1$-$C_6$-Alkyl)$_2$, NH-($C_1$-$C_6$-Alkyl) oder Cyano substituiert sein kann, oder
ein $C_5$-$C_{12}$-Heteroaryl, welches gegebenenfalls ein- oder mehrfach, gleich oder verschieden mit Halogen, Amino, Hydroxy, Cyano, COOH, $C_1$-$C_6$-Alkyl, $C_1$-$C_6$-Acyl, $C_1$-$C_6$-Alkoxy, N-($C_1$-$C_6$-Alkyl)$_2$ oder NH-($C_1$-$C_6$-Alkyl) substituiert sein kann,

$R^5$, $R^6$ unabhängig voneinander Wasserstoff oder ein $C_1$-$C_6$-Alkyl, und

$X^1$ ein Stickstoff, wenn $X^2$ und $X^3$ eine CH-Gruppe sind, oder

$X^2$ ein Stickstoff, wenn $X^1$ und $X^3$ eine CH-Gruppe sind, oder

$X^3$ ein Stickstoff, wenn $X^1$ und $X^2$ eine CH-Gruppe sind,

bedeuten,
sowie deren Stereoisomere, Diastereomere, Enantiomere und Salze.

Ebenfalls bevorzugt sind solche Verbindungen der allgemeinen Formel I, wobei

A ein Phenyl oder ein monocyclischer $C_5$-$C_7$-Heteroarylrest, der gegebenenfalls ein- oder mehrfach mit $R^1$ und/ oder $R^2$ substituiert sein kann,

$R^1$ ein Wasserstoff, Halogen, Amino, eine -$S(O)_p$-$C_1$-$C_6$-Alkylgruppe, wobei p für 0 - 2 steht, ein $SO_2NH_2$, eine -$SO_2NH$-$C_1$-$C_6$-Alkylgruppe, eine -NH-$SO_2$-$C_1$- $C_6$-Alkylgruppe, $C_1$-$C_6$-Acyl-, NH-CO-$NH_2$, -O-CO-NH ($C_1$-$C_6$-Alkyl),- O-CO-N($C_1$-$C_6$-Alkyl)$_2$, NH-CO-$C_1$-$C_6$-Alkylrest, Hydroxy, Cyano, O-CO-($C_1$-$C_6$-Alkyl), $CO_2$-($C_1$-$C_6$-Alkyl), CO-NH-($C_1$-$C_6$-Alkyl), CO- N($C_1$-$C_6$-Alkyl)$_2$, CO-NH($C_5$-$C_{12}$-Heteroaryl), NH-($C_1$-$C_6$-Alkyl), N- ($C_1$-$C_6$-Alkyl)$_2$, oder

ein $C_1$-$C_6$-Alkyl,

welches gegebenenfalls ein- oder mehrfach, gleich oder verschieden substituiert sein kann mit COOH, CO-$NH_2$, CO- NH-($C_1$-$C_6$-Alkyl), CO-N($C_1$-$C_6$-Alkyl)$_2$, $C_1$-$C_6$-Acyl, $C_1$-$C_6$- Alkoxy, Hydroxy, Cyano, $CO_2$-($C_1$-$C_6$-Alkyl), N-($C_1$-$C_6$- Alkyl)$_2$, $C_5$-$C_{12}$-Heteroaryl, welches gegebenenfalls mehrfach substituiert ist, $C_6$-$C_{12}$-Aryl, welches gegebenenfalls mehrfach substituiert ist,

oder

ein $C_1$-$C_6$-Alkoxy,

welches gegebenenfalls ein- oder mehrfach, gleich oder verschieden substituiert sein kann mit Hydroxy, Cyano, $CO_2$-($C_1$-$C_6$-Alkyl), N-($C_1$-$C_6$-Alkyl)$_2$, NH-$C_3$-$C_6$-Cycloalkyl, COOH, CO-$NH_2$, CO-NH($C_1$-$C_6$-Alkyl) oder mit CO-N($C_1$-$C_6$- Alkyl)$_2$, oder

ein O-$C_6$-$C_{12}$-Aryl,

welches gegebenenfalls ein- oder mehrfach, gleich oder verschieden mit Halogen, Hydroxy, Cyano, COOH, CO- $NH_2$, $C_1$-$C_6$-Alkyl, $C_3$-$C_6$-Cycloalkyl, $C_1$-$C_6$-Acyl oder $C_1$-$C_6$- Alkoxy substituiert sein kann, oder

ein O-$C_5$-$C_{12}$-Heteroaryl,

welches gegebenenfalls ein- oder mehrfach, gleich oder verschieden mit Halogen, Hydroxy, Cyano, COOH, CO- $NH_2$, $C_1$-$C_6$-Alkyl, $C_3$-$C_6$-Cycloalkyl, $C_1$-$C_6$-Acyl oder $C_1$-$C_6$- Alkoxy substituiert sein kann, oder

ein $C_6$-$C_{12}$-Aryl,

welches gegebenenfalls ein- oder mehrfach, gleich oder verschieden substituiert sein kann mit Halogen, $C_1$-$C_6$-Alkyl, $C_3$-$C_6$-Cycloalkyl, $C_1$-$C_6$-Acyl, $C_1$-$C_6$-Alkoxy, $C_6$-$C_{12}$-Aryl, $C_5$- $C_{12}$-Heteroaryl, Hydroxy, $CH_2$-OH, Cyano, $CH_2$-CN, Amino, $CO_2$-($C_1$-$C_6$-Alkyl), N-($C_1$-$C_6$-Alkyl)$_2$, $NHSO_2CH_3$, $SO_2NH_2$, $SO_2NH$($C_1$-$C_6$-Alkyl), $SO_2N$($C_1$-$C_6$-Alkyl)$_2$, COOH, CO-$NH_2$, CO-NH-($C_1$-$C_6$-Alkyl), CO-N($C_1$-$C_6$-Alkyl)$_2$, CO-NH($C_5$-$C_{12}$- Heteroaryl), NH-CO($C_1$-$C_6$-Alkyl), $CH_2$-NH-CO($C_1$-$C_6$-Alkyl), NH-CO($C_5$-$C_{12}$-Heteroaryl) oder ein -$S(O)_r$-$CH_3$, wobei r für 0 - 2 steht, oder

zwei benachbarte Positionen durch -O-$CH_2$-O- oder -O-C($CH_3$)$_2$- O- substituiert sein können, oder

ein $C_5$-$C_{16}$-Heteroaryl,

welches gegebenenfalls ein- oder mehrfach, gleich oder verschieden substituiert sein kann mit Halogen, $C_1$-$C_6$-Alkyl, $C_3$-$C_6$-Cycloalkyl, $C_1$-$C_6$-Acyl, $C_1$-$C_6$-Alkoxy, $C_6$-$C_{12}$-Aryl, $C_5$- $C_{12}$-Heteroaryl, Hydroxy, $CH_2$-OH, Cyano, $CH_2$-CN, Amino, $CO_2$-($C_1$-$C_6$-Alkyl), N-($C_1$-$C_6$-Alkyl)$_2$, $NHSO_2CH_3$, $SO_2NH_2$, $SO_2NH$($C_1$-$C_6$-Alkyl), $SO_2N$($C_1$-$C_6$-Alkyl)$_2$, COOH, CO-$NH_2$, CO-NH-($C_1$-$C_6$-Alkyl), CO-N($C_1$-$C_6$-Alkyl)$_2$, CO-NH($C_5$-$C_{12}$- Heteroaryl), NH-CO($C_1$-$C_6$-Alkyl), $CH_2$-NH-CO($C_1$-$C_6$-Alkyl), NH-CO($C_5$-$C_{12}$-Heteroaryl) oder ein -$S(O)_r$-$CH_3$, wobei r für 0 - 2 steht, oder

ein $C_3$-$C_6$-Cycloalkyl,

welches gegebenenfalls ein- oder mehrfach, gleich oder verschieden mit Halogen, $C_1$-$C_6$-Alkyl, Hydroxy, Cyano, $CO_2$-($C_1$-$C_6$-Alkyl), $C_1$-$C_6$-Acyl, N-($C_1$-$C_6$-Alkyl)$_2$, COOH, CO- $NH_2$, CO-NH($C_1$-$C_6$-Alkyl), CO-N ($C_1$-$C_6$-Alkyl)$_2$ oder $C_1$-$C_6$- Alkoxy substituiert sein kann,

$R^2$ ein Wasserstoff, Halogen, $C_1$-$C_6$-Alkyl, $C_1$-$C_6$-Alkoxy, $C_1$-$C_6$-Acyl-, $C_6$-$C_{12}$-Aryl, $C_5$-$C_{16}$-Heteroaryl, $C_3$-$C_6$-Cycloalkyl, NH-CO-$NH_2$, -O- CO-NH($C_1$-$C_6$-Alkyl), -O-CO-N($C_1$-$C_6$-Alkyl)$_2$ , NH-CO-$C_1$-$C_6$- Alkylrest, Hydroxy, Cyano, O-CO-($C_1$-$C_6$-Alkyl), $CO_2$-($C_1$-$C_6$-Alkyl), CO-NH-($C_1$-$C_6$-Alkyl), CO-N($C_1$-$C_6$-Alkyl)$_2$, CO-NH($C_5$-$C_{12}$-Heteroaryl), NH-($C_1$-$C_6$-Alkyl), N-($C_1$-$C_6$-Alkyl)$_2$, O-$C_6$-$C_{12}$-Aryl, O- $C_5$-$C_{12}$-Heteroaryl, oder

$R^3$ ein $C_6$-$C_{12}$-Aryl,

welches gegebenenfalls ein- oder mehrfach, gleich oder verschieden substituiert sein kann mit Halogen, $C_1$-$C_6$-Alkyl, $C_1$-$C_6$-Alkoxy, Hydroxy, $C_1$-$C_6$-Alkylen-OH, $C_1$-$C_6$-Alkylen- $NR^5R^6$, Cyano, $CO_2$-($C_1$-$C_6$-Alkyl), N-($C_1$-$C_6$-Alkyl)$_2$, CO-NH- ($C_1$-$C_6$-Alkyl), CO-NH-($C_1$-$C_6$-Alkylen-OH), CO-NH-($C_1$-$C_6$- Alkylen-$NR^5R^6$), CO-N($C_1$-$C_6$-Alkyl)$_2$ oder $C_1$-$C_6$-Acyl,

welches gegebenenfalls ein- oder mehrfach substituiert ist,

oder

mit zwei benachbarten Positionen durch -O-$CH_2$-O-, -O- $CH_2$-$CH_2$-O-, oder -O-C($CH_3$)$_2$-O- substituiert sein kann, oder

ein $C_5$-$C_{16}$-Heteroaryl,

welches gegebenenfalls ein- oder mehrfach, gleich oder verschieden substituiert sein kann mit Halogen, $C_1$-$C_6$-Alkyl, $C_1$-$C_6$-Acyl, $C_1$-$C_6$-Alkoxy, Hydroxy, Cyano, $CO_2$-($C_1$-$C_6$- Alkyl), N-($C_1$-$C_6$-Alkyl)$_2$, CO-

NH-($C_1$-$C_6$-Alkylen-OH), CO- NH-($C_1$-$C_6$-Alkylen-$NR^5R^6$), CO-NH($C_1$-$C_6$-Alkyl) oder CO- N($C_1$-$C_6$-Alkyl)$_2$ oder
ein $C_3$-$C_6$-Cycloalkyl,
welches gegebenenfalls ein- oder mehrfach, gleich oder verschieden mit Halogen, $CF_3$, Hydroxy, Cyano, $CO_2$-($C_1$-$C_6$-Alkyl), $C_1$-$C_6$-Alkyl, $C_1$-$C_6$-Acyl, N-($C_1$-$C_6$-Alkyl)$_2$, CO-NH-($C_1$-$C_6$-Alkylen-OH), CO-NH-($C_1$-$C_6$-Alkylen-$NR^5R^6$), CO- NH($C_1$-$C_6$-Alkyl) oder CO-N($C_1$-$C_6$-Alkyl)$_2$ oder $C_1$-$C_6$-Alkoxy substituiert sein kann,

$R^4$ ein Wasserstoff, ein $C_1$-$C_6$-Alkyl, welches gegebenenfalls ein- oder mehrfach, gleich oder verschieden substituiert sein kann mit einer -S(O)$_p$-$C_1$-$C_6$- Alkylgruppe oder einer -S(O)$_p$-$C_3$-$C_6$-Cycloalkylgruppe, wobei p für 0 - 2 steht, Hydroxy, Cyano, COOH, CO-$NH_2$, $SO_2NH_2$, $SO_2$NH-($C_1$-$C_6$-Alkyl), $C_3$-$C_6$-Cycloalkyl, $C_1$-$C_6$- Acyl, $C_1$-$C_6$-Alkoxy, $CO_2$-($C_1$-$C_6$-Alkyl), N-($C_1$-$C_6$-Alkyl)$_2$, NH- ($C_1$-$C_6$-Alkyl), NH-($C_3$-$C_6$-Cycloalkyl), CO-NH ($C_1$-$C_6$-Alkyl) oder mit CO-N($C_1$-$C_6$-Alkyl)$_2$, $C_6$-$C_{12}$-Aryl,
welches gegebenenfalls mehrfach substituiert ist, $C_5$-$C_{12}$-Heteroaryl,
welches gegebenenfalls mehrfach substituiert ist, oder
ein $C_3$-$C_6$-Cycloalkyl,
welches gegebenenfalls ein- oder mehrfach, gleich oder verschieden mit Hydroxy, $CH_2$-OH, Cyano, COOH, CO-$NH_2$, $SO_2NH_2$, $C_1$-$C_6$-Alkyl, $C_1$-$C_6$-Acyl oder $C_1$-$C_6$-Alkoxy substituiert sein kann, oder
ein $C_6$-$C_{12}$-Aryl,
welches gegebenenfalls ein- oder mehrfach, gleich oder verschieden mit Halogen, Hydroxy, $C_1$-$C_6$-Alkyl, $C_1$-$C_6$-Acyl, $C_1$-$C_6$-Alkoxy, N-($C_1$-$C_6$-Alkyl)$_2$, NH-($C_1$-$C_6$-Alkyl) oder Cyano substituiert sein kann, oder
ein $C_5$-$C_{12}$-Heteroaryl,
welches gegebenenfalls ein- oder mehrfach, gleich oder verschieden mit Halogen, Amino, Hydroxy, Cyano, COOH, $C_1$-$C_6$-Alkyl, $C_1$-$C_6$-Acyl, $C_1$-$C_6$-Alkoxy, N-($C_1$-$C_6$-Alkyl)$_2$ oder NH-($C_1$-$C_6$-Alkyl) substituiert sein kann,

$R^5$, $R^6$ unabhängig voneinander Wasserstoff oder ein $C_1$-$C_6$-Alkyl, und

$X^1$ ein Stickstoff, wenn $X^2$ und $X^3$ eine CH-Gruppe sind, oder

$X^2$ ein Stickstoff, wenn $X^1$ und $X^3$ eine CH-Gruppe sind, oder

$X^3$ ein Stickstoff, wenn $X^1$ und $X^2$ eine CH-Gruppe sind,

bedeuten,
sowie deren Stereoisomere, Diastereomere, Enantiomere und Salze.

Ebenfalls bevorzugt sind solche Verbindungen der allgemeinen Formel I, wobei

A ein Phenyl oder ein monocyclischer $C_5$-$C_7$-Heteroarylrest, der gegebenenfalls ein- oder mehrfach mit $R^1$ und/ oder $R^2$ substituiert sein kann,

$R^1$ ein Wasserstoff, Halogen, Amino, eine -S(O)$_p$-$C_1$-$C_6$-Alkylgruppe, wobei p für 0 - 2 steht,
ein $SO_2NH_2$, eine -$SO_2$NH-$C_1$-$C_6$-Alkylgruppe, eine -NH-$SO_2$-$C_1$- $C_6$-Alkylgruppe, $C_1$-$C_6$-Acyl-, NH-CO-$NH_2$, -O-CO-NH($C_1$-$C_6$-Alkyl), -O-CO-N($C_1$-$C_6$-Alkyl)$_2$, NH-CO-$C_1$-$C_6$-Alkylrest, Hydroxy, Cyano, O-CO-($C_1$-$C_6$-Alkyl), $CO_2$-($C_1$-$C_6$-Alkyl), CO-NH-($C_1$-$C_6$-Alkyl), CO-N($C_1$-$C_6$-Alkyl)$_2$, CO-NH($C_5$-$C_{12}$-Heteroaryl), NH-($C_1$-$C_6$-Alkyl), N- ($C_1$-$C_6$-Alkyl)$_2$, oder
ein $C_1$-$C_6$-Alkyl,
welches gegebenenfalls ein- oder mehrfach, gleich oder verschieden substituiert sein kann mit COOH, CO-$NH_2$, CO- NH-($C_1$-$C_6$-Alkyl), CO-N($C_1$-$C_6$-Alkyl)$_2$, $C_1$-$C_6$-Acyl, $C_1$-$C_6$- Alkoxy, Hydroxy, Cyano, $CO_2$-($C_1$-$C_6$-Alkyl), N-($C_1$-$C_6$- Alkyl)$_2$, $C_5$-$C_{12}$-Heteroaryl,
welches gegebenenfalls mehrfach substituiert ist, $C_6$-$C_{12}$-Aryl,
welches gegebenenfalls mehrfach substituiert ist,
oder
ein $C_1$-$C_6$-Alkoxy,
welches gegebenenfalls ein- oder mehrfach, gleich oder verschieden substituiert sein kann mit Hydroxy, Cyano, $CO_2$-($C_1$-$C_6$-Alkyl), N-($C_1$-$C_6$-Alkyl)$_2$, NH-$C_3$-$C_6$-Cycloalkyl, COOH, CO-$NH_2$, CO-NH($C_1$-$C_6$-Alkyl) oder mit CO-N($C_1$-$C_6$- Alkyl)$_2$, oder
ein O-$C_6$-$C_{12}$-Aryl,
welches gegebenenfalls ein- oder mehrfach, gleich oder verschieden mit Halogen, Hydroxy, Cyano, COOH, CO- $NH_2$, $C_1$-$C_6$-Alkyl, $C_3$-$C_6$-Cycloalkyl, $C_1$-$C_6$-Acyl oder $C_1$-$C_6$- Alkoxy substituiert sein kann, oder

ein $O-C_5-C_{12}$-Heteroaryl,
welches gegebenenfalls ein- oder mehrfach, gleich oder verschieden mit Halogen, Hydroxy, Cyano, COOH, CO- $NH_2$, $C_1-C_6$-Alkyl, $C_3-C_6$-Cycloalkyl, $C_1-C_6$-Acyl oder $C_1-C_6$- Alkoxy substituiert sein kann, oder
ein $C_6-C_{12}$-Aryl,
welches gegebenenfalls ein- oder mehrfach, gleich oder verschieden substituiert sein kann mit Halogen, $C_1-C_6$-Alkyl, $C_3-C_6$-Cycloalkyl, $C_1-C_6$-Acyl, $C_1-C_6$-Alkoxy, $C_6-C_{12}$-Aryl, $C_5$- $C_{12}$-Heteroaryl, Hydroxy, $CH_2$-OH, Cyano, $CH_2$-CN, Amino, $CO_2$-($C_1-C_6$-Alkyl), N-($C_1-C_6$-Alkyl)$_2$, $NHSO_2CH_3$, $SO_2NH_2$, $SO_2NH$($C_1-C_6$-Alkyl), $SO_2N$($C_1-C_6$-Alkyl)$_2$, COOH, CO-$NH_2$, CO-NH-($C_1-C_6$-Alkyl), CO-N($C_1-C_6$-Alkyl)$_2$, CO-NH($C_5-C_{12}$- Heteroaryl), NH-CO($C_1-C_6$-Alkyl), $CH_2$-NH-CO($C_1-C_6$-Alkyl), NH-CO($C_5-C_{12}$-Heteroaryl) oder ein -S(O)$_r$-$CH_3$, wobei r für 0 - 2 steht, oder
zwei benachbarte Positionen durch -O-$CH_2$-O- oder -O-C($CH_3$)$_2$- O- substituiert sein können, oder
ein $C_5-C_{12}$-Heteroaryl,
welches gegebenenfalls ein- oder mehrfach, gleich oder verschieden substituiert sein kann mit Halogen, $C_1-C_6$-Alkyl, $C_3-C_6$-Cycloalkyl, $C_1-C_6$-Acyl, $C_1-C_6$-Alkoxy, $C_6-C_{12}$-Aryl, $C_5$- $C_{12}$-Heteroaryl, Hydroxy, $CH_2$-OH, Cyano, $CH_2$-CN, Amino, $CO_2$-($C_1-C_6$-Alkyl), N-($C_1-C_6$-Alkyl)$_2$, $NHSO_2CH_3$, $SO_2NH_2$, $SO_2NH$($C_1-C_6$-Alkyl), $SO_2N$($C_1-C_6$-Alkyl)$_2$, COOH, CO-$NH_2$, CO-NH-($C_1-C_6$-Alkyl), CO-N($C_1-C_6$-Alkyl)$_2$, CO-NH($C_5-C_{12}$- Heteroaryl), NH-CO($C_1-C_6$-Alkyl), $CH_2$-NH-CO($C_1-C_6$-Alkyl), NH-CO($C_5-C_{12}$-Heteroaryl) oder ein -S(O)$_r$-$CH_3$, wobei r für 0 - 2 steht, oder
ein $C_3-C_6$-Cycloalkyl,
welches gegebenenfalls ein- oder mehrfach, gleich oder verschieden mit Halogen, $C_1-C_6$-Alkyl, Hydroxy, Cyano, $CO_2$-($C_1-C_6$-Alkyl), $C_1-C_6$-Acyl, N-($C_1-C_6$-Alkyl)$_2$, COOH, CO- $NH_2$, CO-NH($C_1-C_6$-Alkyl), CO-N ($C_1-C_6$-Alkyl)$_2$ oder $C_1-C_6$- Alkoxy substituiert sein kann,

$R^2$ ein Wasserstoff, Halogen, $C_1-C_6$-Acyl-, NH-CO-$NH_2$, -O-CO- NH($C_1-C_6$-Alkyl), -O-CO-N($C_1-C_6$-Alkyl)$_2$, ein NH-CO-$C_1-C_6$- Alkylrest, Hydroxy, Cyano, O-CO-($C_1-C_6$-Alkyl), $CO_2$-($C_1-C_6$-Alkyl), CO-NH-($C_1-C_6$-Alkyl), CO-N($C_1-C_6$-Alkyl)$_2$, CO-NH($C_5-C_{12}$- Heteroaryl), NH-($C_1-C_6$-Alkyl), N-($C_1-C_6$-Alkyl)$_2$, $C_1-C_6$-Alkyl, $C_1-C_6$- Alkoxy, O-$C_6-C_{12}$-Aryl, O-$C_5-C_{12}$-Heteroaryl, $C_6-C_{12}$-Aryl, $C_5-C_{16}$- Heteroaryl, $C_3-C_6$-Cycloalkyl,

$R^3$ ein $C_6-C_{12}$-Aryl, welches gegebenenfalls ein- oder mehrfach, gleich oder verschieden substituiert sein kann mit Halogen, $C_1-C_6$-Alkyl, $C_1-C_6$-Alkoxy, Hydroxy, $C_1-C_6$-Alkylen-OH, $C_1-C_6$-Alkylen- $NR^5R^6$, Cyano, $CO_2$-($C_1-C_6$-Alkyl), N-($C_1-C_6$-Alkyl)$_2$, CO-NH- ($C_1-C_6$-Alkyl), CO-NH-($C_1-C_6$-Alkylen-OH), CO-NH-($C_1-C_6$- Alkylen-$NR^5R^6$), CO-N($C_1-C_6$-Alkyl)$_2$ oder $C_1-C_6$-Acyl,
welches gegebenenfalls ein- oder mehrfach substituiert ist,
oder
mit zwei benachbarten Positionen durch -O-$CH_2$-O-, -O- $CH_2$-$CH_2$-O-, oder -O-C($CH_3$)$_2$-O- substituiert sein kann, oder
ein $C_5-C_{12}$-Heteroaryl,
welches gegebenenfalls ein- oder mehrfach, gleich oder verschieden mit Halogen, $C_1-C_6$-Alkyl, $C_1-C_6$-Acyl, $C_1-C_6$- Alkoxy, Hydroxy, Cyano, $CO_2$-($C_1-C_6$-Alkyl), N-($C_1-C_6$- Alkyl)$_2$, CO-NH-($C_1-C_6$-Alkylen-OH), CO-NH-($C_1-C_6$-Alkylen- $NR^5R^6$), CO-NH($C_1-C_6$-Alkyl) oder CO-N($C_1-C_6$-Alkyl)$_2$ substituiert sein kann,

$R^4$ ein Wasserstoff,
ein $C_1-C_6$-Alkyl,
welches gegebenenfalls ein- oder mehrfach, gleich oder verschieden substituiert sein kann mit einer -S (O)$_p$-$C_1-C_6$- Alkylgruppe oder -S(O)$_p$-$C_3-C_6$-Cycloalkylgruppe, wobei p für 0 - 2 steht, Hydroxy, Cyano, COOH, CO-$NH_2$, $SO_2NH_2$, $SO_2NH$-($C_1-C_6$-Alkyl), $C_3-C_6$-Cycloalkyl, $C_1-C_6$-Acyl, $C_1-C_6$- Alkoxy, $CO_2$-($C_1-C_6$-Alkyl), N-($C_1-C_6$-Alkyl)$_2$, NH-($C_1-C_6$- Alkyl), NH-($C_3-C_6$-Cycloalkyl), CO-NH($C_1-C_6$-Alkyl) oder mit CO-N($C_1-C_6$-Alkyl)$_2$, oder $C_6-C_{12}$-Aryl, welches gegebenenfalls mehrfach substituiert ist, $C_5-C_{12}$-Heteroaryl,
welches gegebenenfalls mehrfach substituiert ist,
oder
ein $C_3-C_6$-Cycloalkyl,
welches gegebenenfalls ein- oder mehrfach, gleich oder verschieden mit Hydroxy, $CH_2$-OH, Cyano, COOH, CO-$NH_2$, $SO_2NH_2$, $C_1-C_6$-Alkyl, $C_1-C_6$-Acyl oder $C_1-C_6$-Alkoxy substituiert sein kann, oder
ein $C_6-C_{12}$-Aryl,
welches gegebenenfalls ein- oder mehrfach, gleich oder verschieden mit Halogen, Hydroxy, $C_1-C_6$-Alkyl, $C_1-C_6$-Acyl, $C_1-C_6$-Alkoxy, N-($C_1-C_6$-Alkyl)$_2$, NH-($C_1-C_6$-Alkyl) oder Cyano substituiert sein kann, oder
ein $C_5-C_{12}$-Heteroaryl,
welches gegebenenfalls ein- oder mehrfach, gleich oder verschieden mit Halogen, Amino, Hydroxy, Cyano,

COOH, $C_1$-$C_6$-Alkyl, $C_1$-$C_6$-Acyl, $C_1$-$C_6$-Alkoxy, N-($C_1$-$C_6$-Alkyl)$_2$ oder NH-($C_1$-$C_6$-Alkyl) substituiert sein kann,

$R^5$, $R^6$ unabhängig voneinander Wasserstoff oder ein $C_1$-$C_6$-Alkyl, und

$X^1$ ein Stickstoff, wenn $X^2$ und $X^3$ eine CH-Gruppe sind, oder

$X^2$ ein Stickstoff, wenn $X^1$ und $X^3$ eine CH-Gruppe sind, oder

$X^3$ ein Stickstoff, wenn $X^1$ und $X^2$ eine CH-Gruppe sind,

bedeuten,
sowie deren Stereoisomere, Diastereomere, Enantiomere und Salze.
Ebenfalls bevorzugt sind solche Verbindungen der allgemeinen Formel I, wobei

A ein Phenyl oder ein monocyclischer $C_5$-$C_7$-Heteroarylrest, der gegebenenfalls ein- oder mehrfach mit $R^1$ und/oder $R^2$ substituiert sein kann,

$R^1$ ein Wasserstoff, Halogen, eine -S(O)$_p$-$C_1$-$C_6$-Alkylgruppe, wobei p für 0 - 2 steht,
ein $SO_2NH_2$, eine -$SO_2$NH-$C_1$-$C_6$-Alkylgruppe, eine -NH-$SO_2$-$C_1$- $C_6$-Alkylgruppe, oder
ein $C_1$-$C_6$-Acyl-, Hydroxy, Cyano, CO-NH-($C_1$-$C_6$-Alkyl), CO-N($C_1$- $C_6$-Alkyl)$_2$, CO-NH($C_5$-$C_{12}$-Heteroaryl), NH-($C_1$-$C_6$-Alkyl), N-($C_1$-$C_6$- Alkyl)$_2$, oder
ein $C_1$-$C_6$-Alkyl,
welches gegebenenfalls ein- oder mehrfach, gleich oder verschieden substituiert sein kann mit $C_1$-$C_6$-Alkoxy, Hydroxy, Cyano, N-($C_1$-$C_6$-Alkyl)$_2$, $C_5$-$C_{12}$-Heteroaryl, welches gegebenenfalls ein oder mehrfach, gleich oder verschieden mit Halogen oder $C_1$-$C_6$-Alkyl substituiert ist,
$C_6$-$C_{12}$-Aryl,
welches gegebenenfalls ein- oder mehrfach, gleich oder verschieden mit Halogen oder $C_1$-$C_6$-Alkyl substituiert ist, oder
ein $C_1$-$C_6$-Alkoxy, oder
ein O-$C_6$-$C_{12}$-Aryl, welches gegebenenfalls ein- oder mehrfach, gleich oder verschieden substituiert sein kann mit Halogen oder $C_1$-$C_6$- Alkyl, $C_3$-$C_6$-Cycloalkyl oder $C_1$-$C_6$-Alkoxy, oder
ein O-$C_5$-$C_{12}$-Heteroaryl,
welches gegebenenfalls ein- oder mehrfach, gleich oder verschieden substituiert sein kann mit Halogen oder $C_1$-$C_6$- Alkyl, $C_3$-$C_6$-Cycloalkyl oder $C_1$-$C_6$-Alkoxy, oder
ein Phenyl,
welches gegebenenfalls ein- oder mehrfach, gleich oder verschieden substituiert sein kann mit Halogen, $C_1$-$C_6$-Alkyl, $C_3$-$C_6$-Cycloalkyl, $C_1$-$C_6$-Acyl, $C_1$-$C_6$-Alkoxy, $C_6$-$C_{12}$-Aryl, $C_5$- $C_{12}$-Heteroaryl, Hydroxy, $CH_2$-OH, Cyano, $CH_2$-CN, Amino, $CO_2$-($C_1$-$C_6$-Alkyl), N-($C_1$-$C_6$-Alkyl)$_2$, $NHSO_2CH_3$, $SO_2NH_2$, $SO_2$NH($C_1$-$C_6$-Alkyl), $SO_2$N($C_1$-$C_6$-Alkyl)$_2$, COOH, CO-$NH_2$, CO-NH-($C_1$-$C_6$-Alkyl), CO-N($C_1$-$C_6$-Alkyl)$_2$, CO-NH($C_5$-$C_{12}$- Heteroaryl), NH-CO($C_1$-$C_6$-Alkyl), $CH_2$-NH-CO($C_1$-$C_6$-Alkyl), NH-CO($C_5$-$C_{12}$-Heteroaryl) oder ein -S(O)$_r$-$CH_3$, wobei r für 0 - 2 steht, oder
zwei benachbarte Positionen durch -O-$CH_2$-O- oder -O-C($CH_3$)$_2$- O- substituiert sein können, oder
ein monocyclischer $C_5$-$C_7$-Heteroarylrest,
welcher gegebenenfalls ein- oder mehrfach, gleich oder verschieden substituiert sein kann mit Halogen, mit $C_1$-$C_6$- Alkyl, $C_3$-$C_6$-Cycloalkyl, $C_1$-$C_6$-Acyl, $C_1$-$C_6$-Alkoxy, $C_6$-$C_{12}$- Aryl, $C_5$-$C_{12}$-Heteroaryl, Hydroxy, $CH_2$-OH, Cyano, $CH_2$-CN, Amino, $CO_2$-($C_1$-$C_6$-Alkyl), N-($C_1$-$C_6$-Alkyl)$_2$, $NHSO_2CH_3$, $SO_2NH_2$, $SO_2$NH($C_1$-$C_6$-Alkyl), $SO_2$N($C_1$-$C_6$-Alkyl)$_2$, COOH, CO-$NH_2$, CO-NH($C_1$-$C_6$-Alkyl), CO-N($C_1$-$C_6$-Alkylh, CO- NH($C_5$-$C_{12}$-Heteroaryl), NH-CO($C_1$-$C_6$-Alkyl), $CH_2$-NH- CO($C_1$-$C_6$-Alkyl), NH-CO($C_5$-$C_{12}$-Heteroaryl) oder ein -S(O)$_r$- $CH_3$, wobei r für 0 - 2 steht, oder
ein $C_3$-$C_6$-Cycloalkyl,
welches gegebenenfalls ein- oder mehrfach, gleich oder verschieden mit Halogen, mit $C_1$-$C_6$-Alkyl, Hydroxy, Cyano, $CO_2$-($C_1$-$C_6$-Alkyl), $C_1$-$C_6$-Acyl, N-($C_1$-$C_6$-Alkyl)$_2$, COOH, CO- $NH_2$, CO-NH($C_1$-$C_6$-Alkyl), CO-N ($C_1$-$C_6$-Alkyl)$_2$ oder $C_1$-$C_6$- Alkoxy substituiert sein kann,

$R^2$ ein Wasserstoff,

$R^3$ ein $C_6$-$C_{12}$-Aryl,
welches gegebenenfalls ein- oder mehrfach, gleich oder verschieden substituiert sein kann mit Halogen,

$C_1$-$C_6$-Alkyl, $C_1$-$C_6$-Alkoxy, Hydroxy, $C_1$-$C_6$-Alkylen-OH, $C_1$-$C_6$-Alkylen- $NR^5R^6$, Cyano, $CO_2$-($C_1$-$C_6$-Alkyl), N-($C_1$-$C_6$-Alkyl)$_2$, CO-NH- ($C_1$-$C_6$-Alkyl), CO-NH-($C_1$-$C_6$-Alkylen-OH), CO-NH-($C_1$-$C_6$- Alkylen-$NR^5R^6$), CO-N ($C_1$-$C_6$-Alkyl)$_2$ oder $C_1$-$C_6$-Acyl,
welches gegebenenfalls ein- oder mehrfach substituiert ist,
oder
mit zwei benachbarten Positionen durch -O-$CH_2$-O-, -O- $CH_2$-$CH_2$-O-, oder -O-C($CH_3$)$_2$-O- substituiert sein kann, oder
ein $C_5$-$C_{12}$-Heteroaryl,
welches gegebenenfalls ein- oder mehrfach, gleich oder verschieden mit Halogen, $C_1$-$C_6$-Alkyl, $C_1$-$C_6$-Acyl, $C_1$-$C_6$- Alkoxy, Hydroxy, Cyano, $CO_2$-($C_1$-$C_6$-Alkyl), N-($C_1$-$C_6$- Alkyl)$_2$, CO-NH-($C_1$-$C_6$-Alkylen-OH), CO-NH-($C_1$-$C_6$-Alkylen- $NR^5R^6$), CO-NH($C_1$-$C_6$-Alkyl) oder CO-N($C_1$-$C_6$-Alkyl)$_2$ substituiert sein kann,

$R^4$ ein Wasserstoff,
ein $C_1$-$C_6$-Alkyl,
welches gegebenenfalls ein- oder mehrfach, gleich oder verschieden substituiert sein kann mit einer -S (O)$_p$-$C_1$-$C_6$- Alkylgruppe oder -S(O)$_p$-$C_3$-$C_6$-Cycloalkylgruppe, wobei p für 0 - 2 steht, Hydroxy, Cyano, COOH, CO-$NH_2$, $SO_2NH_2$, $SO_2$NH-($C_1$-$C_6$-Alkyl), $C_3$-$C_6$-Cycloalkyl, $C_1$-$C_6$-Acyl, $C_1$-$C_6$- Alkoxy, $CO_2$-($C_1$-$C_6$-Alkyl), N-($C_1$-$C_6$-Alkyl)$_2$, NH-($C_1$-$C_6$- Alkyl), NH-($C_3$-$C_6$-Cycloalkyl), CO-NH($C_1$-$C_6$-Alkyl) oder mit CO-N($C_1$-$C_6$-Alkyl)$_2$, $C_6$-$C_{12}$-Aryl,
welches gegebenenfalls mehrfach substituiert ist, $C_5$-$C_{12}$-Heteroaryl,
welches gegebenenfalls mehrfach substituiert ist, oder
ein $C_3$-$C_6$-Cycloalkyl,
welches gegebenenfalls ein- oder mehrfach, gleich oder verschieden mit Hydroxy, $CH_2$-OH, Cyano, COOH, CO-$NH_2$, $SO_2NH_2$, $C_1$-$C_6$-Alkyl, $C_1$-$C_6$-Acyl oder $C_1$-$C_6$-Alkoxy substituiert sein kann, oder
ein $C_6$-$C_{12}$-Aryl,
welches gegebenenfalls ein- oder mehrfach, gleich oder verschieden mit Halogen, Hydroxy, $C_1$-$C_6$-Alkyl, $C_1$-$C_6$-Acyl, $C_1$-$C_6$-Alkoxy, N-($C_1$-$C_6$-Alkyl)$_2$, NH-($C_1$-$C_6$-Alkyl) oder Cyano substituiert sein kann, oder
ein $C_5$-$C_{12}$-Heteroaryl,
welches gegebenenfalls ein- oder mehrfach, gleich oder verschieden mit Halogen, Amino, Hydroxy, Cyano, COOH, $C_1$-$C_6$-Alkyl, $C_1$-$C_6$-Acyl, $C_1$-$C_6$-Alkoxy, N-($C_1$-$C_6$-Alkyl)$_2$ oder NH-($C_1$-$C_6$-Alkyl) substituiert sein kann,

$R^5$ $R^6$ unabhängig voneinander Wasserstoff oder ein $C_1$-$C_6$-Alkyl, und

$X^2$ ein Stickstoff, wenn $X^1$ und $X^3$ eine CH-Gruppe sind, oder

$X^3$ ein Stickstoff, wenn $X^1$ und $X^2$ eine CH-Gruppe sind,

bedeuten,
sowie deren Stereoisomere, Diastereomere, Enantiomere und Salze.

Ebenfalls bevorzugt sind solche Verbindungen der allgemeinen Formel I, wobei

A ein Phenyl-, Thienyl- oder Pyridinyl-Rest, der gegebenenfalls ein- oder zweifach mit $R^1$ und/oder $R^2$ substituiert sein kann,

$R^1$ ein Wasserstoff, Halogen, $C_1$-$C_6$-Alkoxy
ein $C_1$-$C_6$-Alkyl,
welches gegebenenfalls ein- oder mehrfach, gleich oder verschieden substituiert sein kann mit $C_1$-$C_6$-Alkoxy, Hydroxy, Cyano, N-($C_1$-$C_6$-Alkyl)$_2$, Phenyl, ein O-$C_6$-$C_{12}$-Aryl oder ein O-$C_5$-$C_{12}$-Heteroaryl, oder
ein Phenyl,
welches gegebenenfalls ein- oder zweifach, gleich oder verschieden substituiert sein kann mit Halogen, $C_1$-$C_6$-Alkyl, $C_3$-$C_6$-Cycloalkyl, $C_1$-$C_6$-Acyl, $C_1$-$C_6$-Alkoxy, $C_6$-$C_{12}$-Aryl, $C_5$- $C_{12}$-Heteroaryl, Hydroxy, $CH_2$-OH, Cyano, $CH_2$-CN, Amino, $CO_2$-($C_1$-$C_6$-Alkyl), N-($C_1$-$C_6$-Alkyl)$_2$, $NHSO_2CH_3$, $SO_2NH_2$, $SO_2$NH($C_1$-$C_6$-Alkyl), $SO_2$N($C_1$-$C_6$-Alkyl)$_2$, COOH, CO-$NH_2$, CO-NH-($C_1$-$C_6$-Alkyl), CO-N($C_1$-$C_6$-Alkyl)$_2$, CO-NH($C_5$-$C_{12}$- Heteroaryl), NH-CO($C_1$-$C_6$-Alkyl), $CH_2$-NH-CO($C_1$-$C_6$-Alkyl), NH-CO($C_5$-$C_{12}$-Heteroaryl) oder ein -S(O)$_r$-$CH_3$, wobei r für 0 - 2 steht, oder zwei benachbarte Positionen durch -O-$CH_2$-O- oder -O-C($CH_3$)$_2$- O- substituiert sein können, oder
ein Thienyl- oder Pyridinyl Rest,
welcher gegebenenfalls ein- oder zweifach, gleich oder verschieden substituiert sein kann mit Halogen, mit

$C_1$-$C_6$- Alkyl, $C_3$-$C_6$-Cycloalkyl, $C_1$-$C_6$-Acyl, $C_1$-$C_6$-Alkoxy, $C_6$-$C_{12}$- Aryl, $C_5$-$C_{12}$-Heteroaryl, Hydroxy, $CH_2$-OH, Cyano, $CH_2$-CN, Amino, $CO_2$-($C_1$-$C_6$-Alkyl), N-($C_1$-$C_6$-Alkyl)$_2$, $NHSO_2CH_3$, $SO_2NH_2$, $SO_2NH$($C_1$-$C_6$-Alkyl), $SO_2N$($C_1$-$C_6$-Alkyl)$_2$, COOH, CO-$NH_2$, CO-NH($C_1$-$C_6$-Alkyl), CO-N($C_1$-$C_6$-Alkyl)$_2$, CO- NH($C_5$-$C_{12}$-Heteroaryl), NH-CO($C_1$-$C_6$-Alkyl), $CH_2$-NH- CO($C_1$-$C_6$-Alkyl), NH-CO($C_5$-$C_{12}$-Heteroaryl) oder ein -$S(O)_r$- $CH_3$, wobei r für 0 - 2 steht, oder
ein $C_3$-$C_6$-Cycloalkyl,
welches gegebenenfalls ein- oder mehrfach, gleich oder verschieden mit Halogen, mit $C_1$-$C_6$-Alkyl, Hydroxy, Cyano, $CO_2$-($C_1$-$C_6$-Alkyl), $C_1$-$C_6$-Acyl, N-($C_1$-$C_6$-Alkyl)$_2$, COOH, CO- $NH_2$, CO-NH($C_1$-$C_6$-Alkyl), CO-N ($C_1$-$C_6$-Alkyl)$_2$ oder $C_1$-$C_6$- Alkoxy substituiert sein kann,

$R^2$ ein Wasserstoff,

$R^3$ ein $C_6$-$C_{12}$-Aryl,
welches gegebenenfalls ein- oder zweifach, gleich oder verschieden substituiert sein kann mit Halogen, $C_1$-$C_6$-Alkyl, $C_1$-$C_6$-Alkoxy, Hydroxy, $C_1$-$C_6$-Alkylen-OH, $C_1$-$C_6$-Alkylen- $NR^5R^6$, Cyano, $CO_2$-($C_1$-$C_6$-Alkyl), N-($C_1$-$C_6$-Alkyl)$_2$, CO-NH- ($C_1$-$C_6$-Alkyl), CO-NH-($C_1$-$C_6$-Alkylen-OH), CO-NH-($C_1$-$C_6$- Alkylen-$NR^5R^6$), CO-N($C_1$-$C_6$-Alkyl)$_2$ oder $C_1$-$C_6$-Acyl,
welches gegebenenfalls ein- oder mehrfach substituiert ist,
oder
mit zwei benachbarten Positionen durch -O-$CH_2$-O-, -O- $CH_2$-$CH_2$-O-, oder -O-C$(CH_3)_2$-O- substituiert sein kann, oder
ein $C_5$-$C_{12}$-Heteroaryl,
welches gegebenenfalls ein- oder mehrfach, gleich oder verschieden substituiert sein kann mit Halogen, $C_1$-$C_6$-Alkyl, $C_1$-$C_6$-Acyl, $C_1$-$C_6$-Alkoxy, Hydroxy, Cyano, $CO_2$-($C_1$-$C_6$- Alkyl), N-($C_1$-$C_6$-Alkyl)$_2$, CO-NH-($C_1$-$C_6$-Alkylen-OH), CO- NH-($C_1$-$C_6$-Alkylen-$NR^5R^6$), CO-NH($C_1$-$C_6$-Alkyl) oder CO- N($C_1$-$C_6$-Alkyl)$_2$,

$R^4$ ein Wasserstoff,
ein $C_1$-$C_6$-Alkyl,
welches gegebenenfalls ein- oder mehrfach, gleich oder verschieden substituiert sein kann mit einer -S $(O)_p$-$C_1$-$C_6$- Alkylgruppe oder -$S(O)_p$-$C_3$-$C_6$-Cycloalkylgruppe, wobei p für 0 - 2 steht, Hydroxy, Cyano, COOH, CO-$NH_2$, $SO_2NH_2$, $SO_2NH$-($C_1$-$C_6$-Alkyl), $C_3$-$C_6$-Cycloalkyl, $C_1$-$C_6$-Acyl, $C_1$-$C_6$- Alkoxy, $CO_2$-($C_1$-$C_6$-Alkyl), N-($C_1$-$C_6$-Alkyl)$_2$, NH-($C_1$-$C_6$- Alkyl), NH-($C_3$-$C_6$-Cycloalkyl), CO-NH($C_1$-$C_6$-Alkyl) oder mit CO-N($C_1$-$C_6$-Alkyl)$_2$, $C_6$-$C_{12}$-Aryl, welches gegebenenfalls mehrfach substituiert ist, $C_5$-$C_{12}$-Heteroaryl,
welches gegebenenfalls mehrfach substituiert ist,
oder
ein $C_3$-$C_6$-Cycloalkyl,
welches gegebenenfalls ein- oder mehrfach, gleich oder verschieden mit Hydroxy, $CH_2$-OH, Cyano, COOH, CO-$NH_2$, $SO_2NH_2$, $C_1$-$C_6$-Alkyl, $C_1$-$C_6$-Acyl oder $C_1$-$C_6$-Alkoxy substituiert sein kann, oder
ein $C_6$-$C_{12}$-Aryl,
welches gegebenenfalls ein- oder mehrfach, gleich oder verschieden mit Halogen, Hydroxy, $C_1$-$C_6$-Alkyl, $C_1$-$C_6$-Acyl, $C_1$-$C_6$-Alkoxy, N-($C_1$-$C_6$-Alkyl)$_2$, NH-($C_1$-$C_6$-Alkyl) oder Cyano substituiert sein kann, oder
ein $C_5$-$C_{12}$-Heteroaryl,
welches gegebenenfalls ein- oder mehrfach, gleich oder verschieden mit Halogen, Amino, Hydroxy, Cyano, COOH, $C_1$-$C_6$-Alkyl, $C_1$-$C_6$-Acyl, $C_1$-$C_6$-Alkoxy, N-($C_1$-$C_6$-Alkyl)$_2$ oder NH-($C_1$-$C_6$-Alkyl) substituiert sein kann,

$R^5$, $R^6$ unabhängig voneinander Wasserstoff oder ein $C_1$-$C_6$-Alkyl, und

$X^2$ ein Stickstoff, wenn $X^1$ und $X^3$ eine CH-Gruppe sind, oder

$X^3$ ein Stickstoff, wenn $X^1$ und $X^2$ eine CH-Gruppe sind,

bedeuten,
sowie deren Stereoisomere, Diastereomere, Enantiomere und Salze.

Ebenfalls bevorzugt sind solche Verbindungen der allgemeinen Formel I, wobei

A ein Phenyl-, Thienyl- oder Pyridinyl-Rest, der gegebenenfalls ein- oder zweifach mit $R^1$ und/oder $R^2$ substituiert sein kann,

$R^1$ ein Wasserstoff, Halogen, $C_1$-$C_6$-Alkoxy,
ein $C_1$-$C_6$-Alkyl,
welches gegebenenfalls ein- oder mehrfach, gleich oder verschieden substituiert sein kann mit $C_1$-$C_6$-Alkoxy, Hydroxy, Cyano, N-($C_1$-$C_6$-Alkyl)$_2$, Phenyl, ein O-$C_6$-$C_{12}$-Aryl oder ein O-$C_5$-$C_{12}$-Heteroaryl, oder
ein Phenyl,
welches gegebenenfalls ein- oder zweifach, gleich oder verschieden substituiert sein kann mit Halogen, $C_1$-$C_6$-Alkyl, $C_3$-$C_6$-Cycloalkyl, $C_1$-$C_6$-Acyl, $C_1$-$C_6$-Alkoxy, $C_6$-$C_{12}$-Aryl, $C_5$- $C_{12}$-Heteroaryl, Hydroxy, $CH_2$-OH, Cyano, $CH_2$-CN, Amino, $CO_2$-($C_1$-$C_6$-Alkyl), N-($C_1$-$C_6$-Alkyl)$_2$, $NHSO_2CH_3$, $SO_2NH_2$, $SO_2NH$($C_1$-$C_6$-Alkyl), $SO_2N$($C_1$-$C_6$-Alkyl)$_2$, COOH, CO-$NH_2$, CO-NH-($C_1$-$C_6$-Alkyl), CO-N($C_1$-$C_6$-Alkyl)$_2$, CO-NH($C_5$-$C_{12}$- Heteroaryl), NH-CO($C_1$-$C_6$-Alkyl), $CH_2$-NH-CO($C_1$-$C_6$-Alkyl), NH-CO($C_5$-$C_{12}$-Heteroaryl) oder ein -S(O)$_r$-$CH_3$, wobei r für 0 - 2 steht, oder
zwei benachbarte Positionen durch -O-$CH_2$-O- oder -O-C($CH_3$)$_2$- O- substituiert sein können, oder
ein Thienyl- oder Pyridinyl- Rest,
welcher gegebenenfalls ein- oder zweifach, gleich oder verschieden substituiert sein kann mit Halogen, mit $C_1$-$C_6$- Alkyl, $C_3$-$C_6$-Cycloalkyl, $C_1$-$C_6$-Acyl, $C_1$-$C_6$-Alkoxy, $C_6$-$C_{12}$- Aryl, $C_5$-$C_{12}$-Heteroaryl, Hydroxy, $CH_2$-OH, Cyano, $CH_2$-CN, Amino, $CO_2$-($C_1$-$C_6$-Alkyl), N-($C_1$-$C_6$-Alkyl)$_2$, $NHSO_2CH_3$, $SO_2NH_2$, $SO_2NH$($C_1$-$C_6$-Alkyl), $SO_2N$($C_1$-$C_6$-Alkyl)$_2$, COOH, CO-$NH_2$, CO-NH($C_1$-$C_6$-Alkyl), CO-N($C_1$-$C_6$-Alkyl)$_2$, CO- NH($C_5$-$C_{12}$-Heteroaryl), NH-CO($C_1$-$C_6$-Alkyl), $CH_2$-NH- CO($C_1$-$C_6$-Alkyl), NH-CO($C_5$-$C_{12}$-Heteroaryl) oder ein -S(O)$_r$- $CH_3$, wobei r für 0 - 2 steht, oder
ein $C_3$-$C_6$-Cycloalkyl,
welches gegebenenfalls ein- oder mehrfach, gleich oder verschieden mit Halogen, mit $C_1$-$C_6$-Alkyl, Hydroxy, Cyano, $CO_2$-($C_1$-$C_6$-Alkyl), $C_1$-$C_6$-Acyl, N-($C_1$-$C_6$-Alkyl)$_2$, COOH, CO- $NH_2$, CO-NH($C_1$-$C_6$-Alkyl), CO-N ($C_1$-$C_6$-Alkyl)$_2$ oder $C_1$-$C_6$- Alkoxy substituiert sein kann,

$R^2$ ein Wasserstoff,

$R^3$ ein Phenyl,
welches gegebenenfalls ein- oder zweifach, gleich oder verschieden substituiert sein kann mit Halogen, $C_1$-$C_6$-Alkyl, $C_1$-$C_6$-Alkoxy, Hydroxy, $C_1$-$C_6$-Alkylen-OH, $C_1$-$C_6$-Alkylen- $NR^5R^6$, Cyano, $CO_2$-($C_1$-$C_6$-Alkyl), N-($C_1$-$C_6$-Alkyl)$_2$, CO-NH- ($C_1$-$C_6$-Alkyl), CO-NH-($C_1$-$C_6$-Alkylen-OH), CO-NH-($C_1$-$C_6$- Alkylen-$NR^5R^6$), CO-N($C_1$-$C_6$-Alkyl)$_2$ oder $C_1$-$C_6$-Acyl, welches gegebenenfalls ein- oder mehrfach substituiert ist, oder
mit zwei benachbarten Positionen durch -O-$CH_2$-O-, -O- $CH_2$-$CH_2$-O-, oder -O-C($CH_3$)$_2$-O- substituiert sein kann,
oder
ein Thiophenyl-, Furanyl-, oder Pyridinyl-Rest,
welcher gegebenenfalls ein- oder mehrfach, gleich oder verschieden substituiert sein kann mit Halogen, $C_1$-$C_6$-Alkyl, $C_1$-$C_6$-Acyl, $C_1$-$C_6$-Alkoxy, Hydroxy, Cyano, $CO_2$-($C_1$-$C_6$- Alkyl), N-($C_1$-$C_6$-Alkylh, CO-NH-($C_1$-$C_6$-Alkylen-OH), CO- NH-($C_1$-$C_6$-Alkylen-$NR^5R^6$), CO-NH($C_1$-$C_6$-Alkyl) oder CO- N($C_1$-$C_6$-Alkyl)$_2$,

$R^4$ ein Wasserstoff,
ein $C_1$-$C_6$-Alkyl,
welches gegebenenfalls ein- oder mehrfach, gleich oder verschieden substituiert sein kann mit einer -S (O)$_p$-$C_1$-$C_6$- Alkylgruppe oder -S(O)$_p$-$C_3$-$C_6$-Cycloalkylgruppe, wobei p für 0 - 2 steht, Hydroxy, Cyano, COOH, CO-$NH_2$, $SO_2NH_2$, $SO_2NH$-($C_1$-$C_6$-Alkyl), $C_3$-$C_6$-Cycloalkyl, $C_1$-$C_6$-Acyl, $C_1$-$C_6$- Alkoxy, $CO_2$-($C_1$-$C_6$-Alkyl), N-($C_1$-$C_6$-Alkyl)$_2$, NH-($C_1$-$C_6$- Alkyl), NH-($C_3$-$C_6$-Cycloalkyl), CO-NH($C_1$-$C_6$-Alkyl) oder mit CO-N($C_1$-$C_6$-Alkyl)$_2$, $C_6$-$C_{12}$-Aryl,
welches gegebenenfalls mehrfach substituiert ist, $C_5$-$C_{12}$-Heteroaryl,
welches gegebenenfalls mehrfach substituiert ist,
oder
ein $C_3$-$C_6$-Cycloalkyl,
welches gegebenenfalls ein- oder mehrfach, gleich oder verschieden mit Hydroxy, $CH_2$-OH, Cyano, COOH, CO-$NH_2$, $SO_2NH_2$, $C_1$-$C_6$-Alkyl, $C_1$-$C_6$-Acyl oder $C_1$-$C_6$-Alkoxy substituiert sein kann, oder
ein $C_6$-$C_{12}$-Aryl,
welches gegebenenfalls ein- oder mehrfach, gleich oder verschieden mit Halogen, Hydroxy, $C_1$-$C_6$-Alkyl, $C_1$-$C_6$-Acyl, $C_1$-$C_6$-Alkoxy, N-($C_1$-$C_6$-Alkyl)$_2$, NH-($C_1$-$C_6$-Alkyl) oder Cyano substituiert sein kann, oder
ein $C_5$-$C_{12}$-Heteroaryl,
welches gegebenenfalls ein- oder mehrfach, gleich oder verschieden mit Halogen, Amino, Hydroxy, Cyano, COOH, $C_1$-$C_6$-Alkyl, $C_1$-$C_6$-Acyl, $C_1$-$C_6$-Alkoxy, N-($C_1$-$C_6$-Alkyl)$_2$ oder NH-($C_1$-$C_6$-Alkyl) substituiert sein kann,

$R^5$, $R^6$ unabhängig voneinander Wasserstoff oder ein $C_1$-$C_6$-Alkyl, und

$X^2$ ein Stickstoff, wenn $X^1$ und $X^3$ eine CH-Gruppe sind, oder

$X^3$ ein Stickstoff, wenn $X^1$ und $X^2$ eine CH-Gruppe sind,

bedeuten,
sowie deren Stereoisomere, Diastereomere, Enantiomere und Salze.

Ebenfalls bevorzugt sind solche Verbindungen der allgemeinen Formel I, wobei

A ein Phenyl-, Thienyl- oder Pyridinyl-Rest, der gegebenenfalls ein- oder zweifach mit $R^1$ und/ oder $R^2$ substituiert sein kann,

$R^1$ ein Wasserstoff, Halogen, $C_1$-$C_4$-Alkoxy
ein $C_1$-$C_4$-Alkyl,
welches gegebenenfalls ein- oder mehrfach, gleich oder verschieden substituiert sein kann mit $C_1$-$C_4$-Alkoxy, Hydroxy, Cyano, N-($C_1$-$C_4$-Alkyl)$_2$, Phenyl,
ein O-$C_6$-$C_{12}$-Aryl oder einO-$C_5$-$C_{12}$-Heteroaryl, oder ein Phenyl,
welches gegebenenfalls ein- oder zweifach, gleich oder verschieden substituiert sein kann mit Halogen, $C_1$-$C_4$-Alkyl, $C_3$-$C_6$-Cycloalkyl, $C_1$-$C_4$-Acyl, $C_1$-$C_4$-Alkoxy, $C_6$-$C_{12}$-Aryl, $C_5$- $C_{12}$-Heteroaryl, Hydroxy, $CH_2$-OH, Cyano, $CH_2$-CN, Amino, $CO_2$-($C_1$-$C_4$-Alkyl), N-($C_1$-$C_4$-Alkyl)$_2$, $NHSO_2CH_3$, $SO_2NH_2$, $SO_2NH$($C_1$-$C_4$-Alkyl), $SO_2N$($C_1$-$C_4$-Alkyl)$_2$, COOH, CO-$NH_2$, CO-NH-($C_1$-$C_4$-Alkyl), CO-N($C_1$-$C_4$-Alkyl)$_2$, CO-NH($C_5$-$C_{12}$- Heteroaryl), NH-CO($C_1$-$C_4$-Alkyl), $CH_2$-NH-CO($C_1$-$C_4$-Alkyl), NH-CO($C_5$-$C_{12}$-Heteroaryl) oder ein -S(O),-$CH_3$, wobei r für 0 - 2 steht, oder
zwei benachbarte Positionen durch -O-$CH_2$-O- oder -O-C($CH_3$)$_2$- O- substituiert sein können, oder
ein Thienyl- oder Pyridinyl- Rest,
welcher gegebenenfalls ein- oder zweifach, gleich oder verschieden substituiert sein kann mit Halogen, mit $C_1$-$C_4$- Alkyl, $C_3$-$C_6$-Cycloalkyl, $C_1$-$C_4$-Acyl, $C_1$-$C_4$-Alkoxy, $C_6$-$C_{12}$- Aryl, $C_5$-$C_{12}$-Heteroaryl, Hydroxy, $CH_2$-OH, Cyano, $CH_2$-CN, Amino, $CO_2$-($C_1$-$C_4$-Alkyl), N-($C_1$-$C_4$-Alkyl)$_2$, $NHSO_2CH_3$, $SO_2NH_2$, $SO_2NH$($C_1$-$C_4$-Alkyl), $SO_2N$($C_1$-$C_4$-Alkyl)$_2$, COOH, CO-$NH_2$, CO-NH($C_1$-$C_4$-Alkyl), CO-N($C_1$-$C_4$-Alkyl)$_2$, CO- NH($C_5$-$C_{12}$-Heteroaryl), NH-CO($C_1$-$C_4$-Alkyl), $CH_2$-NH- CO($C_1$-$C_4$-Alkyl), NH-CO($C_5$-$C_{12}$-Heteroaryl) oder ein -S(O)$_r$- $CH_3$, wobei r für 0 - 2 steht, oder
ein $C_3$-$C_6$-Cycloalkyl,
welches gegebenenfalls ein- oder mehrfach, gleich oder verschieden mit Halogen, mit $C_1$-$C_4$-Alkyl, Hydroxy, Cyano, $CO_2$-($C_1$-$C_4$-Alkyl), $C_1$-$C_4$-Acyl, N-($C_1$-$C_4$-Alkyl)$_2$, COOH, CO- $NH_2$, CO-NH($C_1$-$C_4$-Alkyl), CO-N ($C_1$-$C_4$-Alkyl)$_2$ oder $C_1$-$C_4$- Alkoxy substituiert sein kann,

$R^2$ ein Wasserstoff,

$R^3$ ein Phenyl,
welches gegebenenfalls ein- oder zweifach, gleich oder verschieden substituiert sein kann mit Halogen, $C_1$-$C_4$-Alkyl, $C_1$-$C_4$-Alkoxy, Hydroxy, $C_1$-$C_4$-Alkylen-OH, $C_1$-$C_4$-Alkylen- $NR^5R^6$, Cyano, $CO_2$-($C_1$-$C_4$-Alkyl), N-($C_1$-$C_4$-Alkyl)$_2$, CO-NH- ($C_1$-$C_4$-Alkyl), CO-NH-($C_1$-$C_4$-Alkylen-OH), CO-NH-($C_1$-$C_4$- Alkylen-$NR^5R^6$), CO-N($C_1$-$C_4$-Alkyl)$_2$ oder $C_1$-$C_4$-Acyl,
welches gegebenenfalls ein- oder mehrfach substituiert ist,
oder
mit zwei benachbarten Positionen durch -O-$CH_2$-O-, -O- $CH_2$-$CH_2$-O-, oder -O-C($CH_3$)$_2$-O- substituiert sein kann,
oder
ein Thiophenyl-, Furanyl-, oder Pyridinyl-Rest,
welcher gegebenenfalls ein- oder mehrfach, gleich oder verschieden substituiert sein kann mit Halogen, $C_1$-$C_4$-Alkyl, $C_1$-$C_4$-Acyl, $C_1$-$C_4$-Alkoxy, Hydroxy, Cyano, $CO_2$-($C_1$-$C_4$- Alkyl), N-($C_1$-$C_4$-Alkyl)$_2$, CO-NH-($C_1$-$C_4$-Alkylen-OH), CO- NH-($C_1$-$C_4$-Alkylen-$NR^5R^6$), CO-NH($C_1$-$C_4$-Alkyl) oder CO- N($C_1$-$C_4$-Alkyl)$_2$,

$R^4$ ein Wasserstoff,
ein $C_1$-$C_4$-Alkyl,
welches gegebenenfalls ein- oder mehrfach, gleich oder verschieden substituiert sein kann mit einer -S (O)$_p$-$C_1$-$C_4$- Alkylgruppe oder -S(O)$_p$-$C_3$-$C_6$-Cycloalkylgruppe, wobei p für 0 - 2 steht, Hydroxy, Cyano, COOH,

$CO-NH_2$, $SO_2NH_2$, $SO_2NH-(C_1-C_4-Alkyl)$, $C_3-C_6$-Cycloalkyl, $C_1-C_4$-Acyl, $C_1-C_4$- Alkoxy, $CO_2-(C_1-C_4-Alkyl)$, $N-(C_1-C_4-Alkyl)_2$, $NH-(C_1-C_4$- Alkyl), $NH-(C_3-C_6$-Cycloalkyl), $CO-NH(C_1-C_4$-Alkyl) oder mit $CO-N(C_1-C_4$-Alkyl$)_2$, $C_6-C_{12}$-Aryl,
welches gegebenenfalls mehrfach substituiert ist, $C_5-C_{12}$-Heteroaryl,
welches gegebenenfalls mehrfach substituiert ist,
oder
ein $C_3-C_6$-Cycloalkyl,
welches gegebenenfalls ein- oder mehrfach, gleich oder verschieden mit Hydroxy, $CH_2$-OH, Cyano, COOH, $CO-NH_2$, $SO_2NH_2$, $C_1-C_4$-Alkyl, $C_1-C_4$-Acyl oder $C_1-C_4$-Alkoxy substituiert sein kann, oder
ein $C_6-C_{12}$-Aryl,
welches gegebenenfalls ein- oder mehrfach, gleich oder verschieden mit Halogen, Hydroxy, $C_1-C_4$-Alkyl, $C_1-C_4$-Acyl, $C_1-C_4$-Alkoxy, $N-(C_1-C_4-Alkyl)_2$, $NH-(C_1-C_4$-Alkyl) oder Cyano substituiert sein kann, oder
ein $C_5-C_{12}$-Heteroaryl,
welches gegebenenfalls ein- oder mehrfach, gleich oder verschieden mit Halogen, Amino, Hydroxy, Cyano, COOH, $C_1-C_4$-Alkyl, $C_1-C_4$-Acyl, $C_1-C_4$-Alkoxy, $N-(C_1-C_4-Alkyl)_2$ oder $NH-(C_1-C_4$-Alkyl) substituiert sein kann,

$R^5$, $R^6$ unabhängig voneinander Wasserstoff oder ein $C_1-C_4$-Alkyl, und

$X^2$ ein Stickstoff, wenn $X^1$ und $X^3$ eine CH-Gruppe sind, oder

$X^3$ ein Stickstoff, wenn $X^1$ und $X^2$ eine CH-Gruppe sind,

bedeuten,
sowie deren Stereoisomere, Diastereomere, Enantiomere und Salze.

Die folgenden Verbindungen entsprechend der vorliegenden Erfindung sind ganz besonders bevorzugt:

1. 5-(4-tert-Butyl-phenylsulfonylamino)-3-phenyl-1 H-pyrrolo[3,2-b]pyridin-2-carbonsäure (tetrahydro-pyran-4-yl)-amid
2. 5-(4-tert-Butyl-phenylsulfonylamino)-3-phenyl-1 H-pyrrolo[3,2-b]pyridin-2-carbonsäure (2-morpholin-4-yl-ethyl)-amid
3. 5-(4-tert-Butyl-phenylsulfonylamino)-3-phenyl-1 H-pyrrolo[2,3-c]pyridin-2-carbonsäure (2-morpholin-4-yl-ethyl)-amid
4. 5-(4-tert-Butyl-phenylsulfonylamino)-3-phenyl-1 H-pyrrolo[2,3-b]pyridin-2-carbonsäure (2-morpholin-4-yl-ethyl)-amid
5. (±)-5-(4-tert-Butyl-phenylsulfonylamino)-3-phenyl-1H-pyrrolo[2,3-b]pyridin-2-carbonsäure (2-hydroxy-propyl)-amid
6. 5-(4-tert-Butyl-phenylsulfonylamino)-3-phenyl-1 H-pyrrolo[2,3-b]pyridin-2-carbonsäure (tetrahydro-pyran-4-yl)-amid
7. (±)-5-(4-tert-Butyl-phenylsulfonylamino)-3-phenyl-1 H-pyrrolo[2,3-b]pyridin-2-carbonsäure (2-hydroxy-1-methyl-ethyl)-amid
8. (1S,2R)-5-(4-tert-Butyl-phenylsulfonylamino)-3-phenyl-1H-pyrrolo[2,3-b]pyridin-2-carbonsäure (2-hydroxy-cyclopentyl)-amid
9. 3-Phenyl-5-(4-trifluormethyl-phenylsulfonylamino)-1 H-pyrrolo[2,3-b]pyridin-2-carbonsäure (2-morpholin-4-yl-ethyl)-amid
10. (1 S,2R)-5-(Biphenyl-4-sulfonylamino)-3-phenyl-1H-pyrrolo[2,3-b]pyridin-2-carbonsäure (2-hydroxy-cyclopentyl)-amid
11. 3-Phenyl-5-(4-trifluormethoxy-phenylsulfonylamino)-1 H-pyrrolo[2,3-b]pyridin-2-carbonsäure (2-morpholin-4-yl-ethyl)-amid
12. 5-(Biphenyl-4-sulfonylamino)-3-phenyl-1H-pyrrolo[2,3-b]pyridin-2-carbonsäure (2-morpholin-4-yl-ethyl)-amid
13. (1S,2R)-3-Phenyl-5-(4-trifluormethyl-phenylsulfonylamino)-1 H-pyrrolo[2,3-b]pyridin-2-carbonsäure (2-hydroxy-cyclopentyl)-amid
14. 5-(4-tert-Butyl-phenylsulfonylamino)-3-phenyl-1H-pyrrolo[2,3-b]pyridin-2-carbonsäure [2-(morpholin-4-sulfonyl)-ethyl]-amid
15. 5-(4-tert-Butyl-phenylsulfonylamino)-3-phenyl-1H-pyrrolo[2,3-b]pyridin-2-carbonsäure pyridin-4-ylamid
16. 5-(4-tert-Butyl-phenylsulfonylamino)-3-phenyl-1H-pyrrolo[2,3-b]pyridin-2-carbonsäure thiazol-2-ylamid
17. 5-(4-tert-Butyl-phenylsulfonylamino)-3-phenyl-1H-pyrrolo[2,3-b]pyridin-2-carbonsäure (pyridin-3-ylmethyl)-amid
18. (±)-5-(4-tert-Butyl-phenylsulfonylamino)-3-phenyl-1H-pyrrolo[2,3-b]pyridin-2-carbonsäure (tetrahydro-furan-2-ylmethyl)-amid

19. 5-(4-tert-Butyl-phenylsulfonylamino)-3-phenyl-1H-pyrrolo[2,3-b]pyridin-2-carbonsäure ethylamid

20. 5-(4-tert-Butyl-phenylsulfonylamino)-3-phenyl-1H-pyrrolo[2,3-b]pyridin-2-carbonsäure [2-(2-oxo-imidazolidin-1-yl)-ethyl]-amid

21. (1 S,2R)-3-Phenyl-5-(4-trifluormethoxy-phenylsulfonylamino)-1H-pyrrolo[2,3-b]pyridin-2-carbonsäure (2-hydroxy-cyclopentyl)-amid

22. 5-(4-tert-Butyl-phenylsulfonylamino)-3-phenyl-1H-pyrrolo[2,3-b]pyridin-2-carbonsäureamid

23. 5-(4-tert-Butyl-phenylsulfonylamino)-3-phenyl-1H-pyrrolo[2,3-b]pyridin-2-carbonsäure (2-sulfamoyl-ethyl)-amid

24. 5-(Biphenyl-4-sulfonylamino)-3-phenyl-1 H-pyrrolo[2,3-b]pyridine-2-carbonsäureamid

25. (1 S,2R)-3-Benzo[1,3]dioxol-5-yl-5-(4-trifluormethoxy-phenylsulfonylamino)-1 H-pyrrolo[2,3-b]pyridin-2-carbonsäure (2-hydroxy-cyclopentyl)-amid

26. (1 S,2R)-3-Thiophen-2-yl-5-(4-trifluormethoxy-phenylsulfonylamino)-1H-pyrrolo[2,3-b]pyridin-2-carbonsäure (2-hydroxy-cyclopentyl)-amid

27. (1 S,2R)-3-Thiophen-3-yl-5-(4-trifluormethoxy-phenylsulfonylamino)-1H-pyrrolo[2,3-b]pyridin-2-carbonsäure (2-hydroxy-cyclopentyl)-amid

28. (1 S,2R)-3-Furan-2-yl-5-(4-trifluormethoxy-phenylsulfonylamino)-1H-pyrrolo[2,3-b]pyridin-2-carbonsäure (2-hydroxy-cyclopentyl)-amid

29. (1 S,2R)-3-Furan-3-yl-5-(4-trifluormethoxy-phenylsulfonylamino)-1H-pyrrolo[2,3-b]pyridin-2-carbonsäure (2-hydroxy-cyclopentyl)-amid

30. (1 S,2R)-3-Benzo[1,3]dioxol-5-yl-5-(4-tert-butyl-phenylsulfonylamino)-1H-pyrrolo[2,3-b]pyridin-2-carbonsäure (2-hydroxy-cyclopentyl)-amid

31. (1S,2R)-3-Thiophen-2-yl-5-(4-tert-butyl-phenylsulfonylamino)-1H-pyrrolo[2,3-b]pyridin-2-carbonsäure (2-hydroxy-cyclopentyl)-amid

32. (1S,2R)-3-Thiophen-3-yl-5-(4-tert-butyl-phenylsulfonylamino)-1H-pyrrolo[2,3-b]pyridin-2-carbonsäure (2-hydroxy-cyclopentyl)-amid

33. (1 S,2R)-3-Furan-2-yl-5-(4-tert-butyl-phenylsulfonylamino)-1H-pyrrolo[2,3-b]pyridin-2-carbonsäure (2-hydroxy-cyclopentyl)-amid

34. (1S,2R)-3-Furan-3-yl-5-(4-tert-butyl-phenylsulfonylamino)-1H-pyrrolo[2,3-b]pyridin-2-carbonsäure (2-hydroxy-cyclopentyl)-amid

35. (±)-3-Thiophen-3-yl-5-(4-trifluormethoxy-phenylsulfonylamino)-1H-pyrrolo[2,3-b]pyridin-2- carbonsäure (tetrahydro-furan-2-ylmethyl)-amid

36. (±)-3-Thiophen-2-yl-5-(4-trifluormethoxy-phenylsulfonylamino)-1H-pyrrolo[2,3-b]pyridin-2- carbonsäure (tetrahydro-furan-2-ylmethyl)-amid

37. (±)-3-Thiophen-3-yl-5-(4-tert-butyl-phenylsulfonylamino)-1H-pyrrolo[2,3-b]pyridin-2- carbonsäure (tetrahydrofuran-2-ylmethyl)-amid

38. (±)-3-Thiophen-2-yl-5-(4-tert-butyl-phenylsulfonylamino)-1H-pyrrolo[2,3-b]pyridin-2- carbonsäure (tetrahydrofuran-2-ylmethyl)-amid

Außerdem betrifft die Erfindung ein Verfahren zur Herstellung der erfindungsgemäßen Verbindungen der allgemeinen Formel I, das dadurch gekennzeichnet ist, dass man eine Verbindung der Formel II,

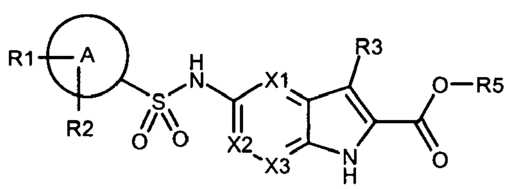

(II)

worin $R^1$, $R^2$, $R^3$, $X^1$, $X^2$ und $X^3$ die oben angegebenen Bedeutungen haben und $R^5$ ein Wasserstoff oder ein $C_1$-$C_6$-Akylrest sein kann, bevorzugt ist Wasserstoff, der Methyl- oder Ethyl-Rest, mit einem Amin der allgemeinen Formel III

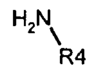

(III)

worin $R^4$ die oben angegebene Bedeutung aufweist, nach dem Fachmann bekannten Methoden umsetzt und/oder gegebenenfalls benötigte Schutzgruppen anschließend gespalten und/oder gegebenenfalls vorhandene Doppelbindungen hydriert werden.

Für den Fall, dass $R^5$ gleich Wasserstoff ist, kann die Umsetzung zunächst durch Aktivierung der Säurefunktion erfolgen, hierbei wird beispielsweise die Carbonsäure der allgemeinen Formel II zunächst in Gegenwart eines tertiären Amins, wie beispielsweise Triethylamin, mit Chlorameisensäure-isobutylester in das gemischte Anhydrid überführt. Die Umsetzung des gemischten Anhydrids mit dem Alkalisalz des entsprechenden Amins erfolgt in einem inerten Lösungsmittel oder Lösungsmittelgemisch, wie beispielsweise Tetrahydrofuran, Dimethoxyethan, Dimethylformamid, Hexamethylphosphor-säuretriamid, bei Temperaturen zwischen - 30 ˚ C und + 60 ˚ C, vorzugsweise bei 0 ˚ C bis 30 ˚ C.

Eine weitere Möglichkeit besteht darin, die Carbonsäure der allgemeinen Formel II durch Reagenzien, wie beispielsweise HOBt oder HATU, zu aktivieren. Die Umsetzung der Säure erfolgt z.B. mit HATU in einem inerten Lösungsmittel, wie beispielsweise DMF, in Gegenwart des entsprechenden Amins der allgemeinen Formel III und einem tertiären Amin, wie beispielsweise Ethyldiisopropylamin, bei Temperaturen zwischen - 50 und + 60 ˚ C, vorzugsweise bei 0 ˚ C bis 30 ˚C.

Für den Fall, dass $R^5$ gleich $C_1$-$C_6$-Alkyl ist, kann beispielsweise auch eine direkte Amidolyse des Esters mit dem entsprechenden Amin eventuell unter zu Hilfenahme von Aluminiumtrialkyl-Reagenzien, vorzugsweise Aluminiumtrimethyl, durchgeführt werden.

Synthese der 4- und der 6-Azaindole:

Für die Fälle, dass $X^1$ ein Stickstoff und $X^2$ und $X^3$ eine CH-Gruppe ist oder $X^2$ ein Stickstoff und $X^1$ und $X^3$ eine CH-Gruppe ist:

Die als Ausgangsmaterialien dienenden Verbindungen der allgemeinen Formel II können beispielsweise hergestellt werden, indem man in an sich bekannter Weise die Aminogruppe in den Aza-Indolestern der allgemeinen Formel IV,

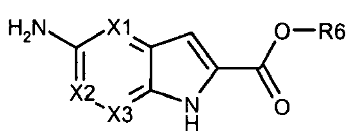

(IV)

deren Herstellung im experimentellen Teil beschrieben ist, und
worin $R^6$ ein $C_1$-$C_6$-Alkylrest, vorzugsweise ein Methyl- oder Ethylrest, ist, mit einem Sulfonsäure-Derivat der allgemeinen Formel V,

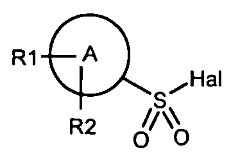

(V),

worin $R^1$, $R^2$ und A die oben angegebenen Bedeutungen aufweisen und Hal für ein Halogen, vorzugsweise Chlorid oder Bromid steht, in Gegenwart einer Base wie beispielsweise Pyridin, Diisopropylethylamin, Triethylamin oder Kaliumcarbonat zu den Verbindungen der allgemeinen Formel VI umsetzt.

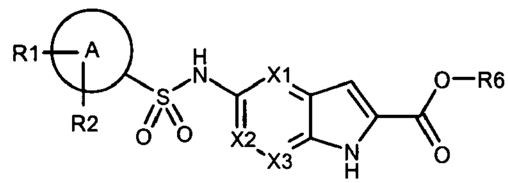

(VI).

Die Ester der allgemeinen Formel VI werden dann in der 3-Position beispielsweise mittels Iod, NBI, NBS oder auch $CuBr_2$ halogeniert, dann intermediär mit einer Stickstoff-Schutzgruppe versehen, beispielsweise mittels Rühren in $Boc_2O$ mit der Boc-Schutzgruppe, und anschließend in einer Pdkatalysierten Reaktion mit Boronsäurederivaten der allgemeinen Formel VII,

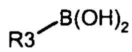

(VII),

worin $R^3$ die oben angegebene Bedeutung aufweist, und anschließende Abspaltung der Schutzgruppen, im Falle der Boc-Schutzgruppe durch Säuren wie z.B. Trifluoressigsäure und notfalls durch Verseifung und nachfolgende Veresterung mit $R^5$-OH in die Ester der allgemeinen Formel II,

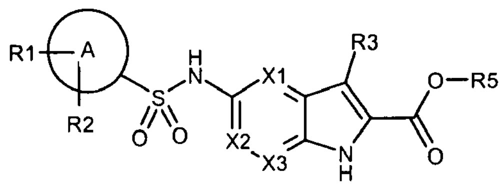

(II)

worin $R^1$, $R^2$, $R^3$, $R^5$ und A die oben angegebenen Bedeutungen aufweisen und $X^1$ ein Stickstoff und $X^2$ und $X^3$ eine CH-Gruppe oder $X^2$ ein Stickstoff und $X^1$ und $X^3$ eine CH-Gruppe ist, überführt.

Synthese der 7-Azaindole:

Für die Fälle, dass $X^3$ ein Stickstoff und $X^1$ und $X^2$ eine CH-Gruppe bedeuten: Die als Ausgangsmaterialien dienenden Verbindungen der allgemeinen Formel II mit der angegeben Bedeutung von $X^1$, $X^2$ und $X^3$ können beispielsweise hergestellt werden, indem man in an sich bekannter Weise das bekannte Säurechlorid VIII

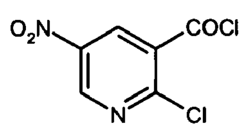

(VIII)

zunächst unter Friedel-Crafts-Bedingungen mit den Aryl- bzw. Heteroaryl-Derivaten der allgemeinen Formel IX,

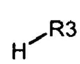

(IX),

worin $R^3$ die oben angegebene Bedeutung aufweist, in Gegenwart eines Katalysators, wie beispielsweise $AlCl_3$ oder

Ln(OTf)$_3$, zu den Verbindungen der allgemeinen Formel X umsetzt.

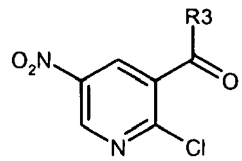

(X).

Eine alternative Möglichkeit, zu den Verbindungen der allgemeinen Formel X zu kommen, bestände auch darin, die Aryl- bzw. Heteroaryl-Derivate der allgemeinen Formel XI,

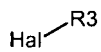

(XI),

worin $R^3$ die oben angegebene Bedeutung aufweist und Hal ein Halogenatom, vorzugsweise ein Iod, Brom oder Chlor ist, mit Magnesium in die entsprechende Grignard-Verbindung oder mit Lithium oder t-Butyl-Lithium in die entsprechende Organo-Lithium-Verbindung überführt und dann nach dem Fachmann bekannten Methoden mit dem Säurechlorid der allgemeinen Formel VIII, eventuell unter Zusatz von Cadmiundichlorid, in die Ketone der allgemeinen Formel X überführt.

Die Ketone der allgemeinen Formel X werden dann in der 2-Position, beispielsweise mittels nucleophiler Substitution, mit dem bekannten p-Methoxybenzyl-geschützten Glycin-ester XII umgesetzt,

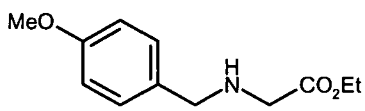

(XII),

und das so erhaltene Produkt mittels Essigsäureanhydrid in Pyridin oder auch durch Basen, wie bespielsweise LDA, $LiN(TMS)_2$, Kaliumcarbonat oder Diisopropylethylamin, in einem inerten Lösungsmittel bei Temperaturen zwischen 0 ° C und der Siedetemperatur des Lösungsmittels, wie beispielsweise Tetrahydrofuran, zu den Verbindungen der allgemeinen Formel XIII,

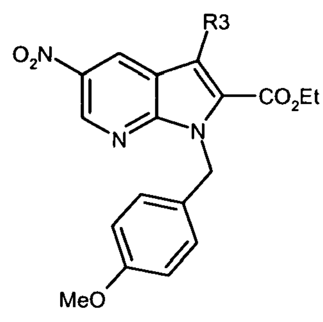

(XIII),

worin $R^3$ die oben angegebene Bedeutung aufweist, umgesetzt.

Die p-Methoxybenzyl-Schutzgruppe in den Derivaten XIII kann beispielsweise durch Trifluoressigsäure oder auch $AlCl_3$ in Anisol abgespalten werden. Die erhaltene Nitro-Verbindung wird dann durch Rühren in einer Wasserstoffatmosphäre gegebenenfalls unter Druck in Gegenwart eines Katalysators wie beispielsweise Palladium auf Kohle in die Amine der allgemeinen Formel XIV überführt. Für die Reinigung dieser Verbindungen kann es gegebenenfalls auch notwendig sein, die freien Basen in Salze, zum Beispiel in das Hydrochlorid durch Rühren mit HCl in Dioxan, zu

überführen,

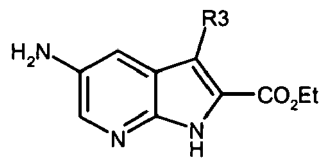

(XIV).

Die so erhaltenen Amine oder deren Salze werden dann durch Umsetzung mit einem Sulfonsäure-Derivat der allgemeinen Formel V,

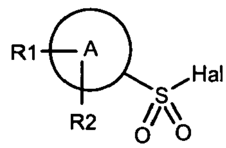

(V),

worin $R^1$, $R^2$ und A die oben angegebenen Bedeutungen aufweisen und Hal für ein Halogen, vorzugsweise Chlorid oder Bromid steht, in Gegenwart einer Base, wie beispielsweise Pyridin, Diisopropylethylamin, Triethylamin oder Kaliumcarbonat, und gegebenenfalls nach Verseifung des Ethylesters bzw. durch anschließende Veresterung mit $R^5$-OH nach den dem Fachmann bekannten Methoden in die Ester der allgemeinen Formel II,

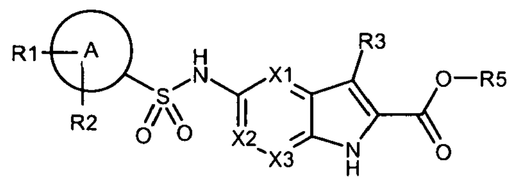

(II)

worin $R^1$, $R^2$, $R^3$, $R^5$ und A die oben angegebenen Bedeutungen aufweisen und $X^1$ und $X^2$ eine CH-Gruppe und $X^3$ ein Stickstoff ist, überführt.

Die erfindungsgemäßen Verbindungen inhibieren die lösliche Adenylatzyklase, worauf auch deren Wirkung zum Beispiel bei der männlichen Fertilitätskontrolle beruht.

Adenylatzyklasen sind die Effektormoleküle für einen der am meist genutzten Signaltransduktionswege, sie synthetisieren das second messenger Molekül zyklisches Adenosinmonophosphat (cAMP) aus Adenosintriphosphat (ATP) unter Abspaltung von Pyrophosphat (PP). cAMP vermittelt zahlreiche zelluläre Antworten für eine Vielzahl von Neurotransmittern und Hormonen.

Die lösliche, spermienspezifische Adenylatzyklase (sAC, humane mRNA-Sequenz (GenBank) NM_018417, humanes Gen ADCY X) ist eine von zehn beschriebenen Adenylatzyklasen im menschlichen Genom. sAC zeigt dabei einige spezifische Eigenschaften, die sie von den anderen Adenylatzyklasen unterscheidet. sAC wird, im Gegensatz zu allen anderen Adenylatzyklasen, durch die Konzentration an Bicarbonat im sie umgebenden Medium stimuliert und nicht durch G-Proteine. sAC besitzt keine Transmembranregionen in ihrer Aminosäuresequenz, sie ist nicht durch Forskolin inhibierbar, ist durch Mangan viel stärker stimulierbar als durch Magnesium, und zeigt nur geringe Sequenzhomologien zu den anderen Adenylatzyklasen (≤ 26 % Identität der katalytischen Domänen I und II der sAC mit anderen Adenylatzyklasen auf Aminosäureebene).

Spezifische, Mangan-abhängige Aktivität von sAC wurde zuerst von T. Braun et al. (1975, PNAS 73:1097ff) in Rattentestis und Spermien beschrieben. N. Okamura et al. (1985, J. Biol. Chem 260(17):9699ff) zeigten, dass es sich bei der Substanz, die die Aktivität der sAC in Eberseminalflüssigkeit stimuliert, um Bicarbonat handelt. Ebenfalls konnte gezeigt werden, dass nur in Rattentestis und Spermien, aber nicht in anderen Geweben, Bicarbonat-stimulierbare AC-Aktivität nachgewiesen werden kann. sAC wurde von der Gruppe Buck und Levin aus Rattentestis gereinigt und erstmalig sequenziert (J. Buck et al. 1999 PNAS 96:79ff, WO 01/85753). Die zu erwartenden Eigenschaften (z.B. Bikarbonat- und

Magnesiumstimulierbarkeit) wurden am rekombinant exprimierten Protein bestätigt (Y. Chen et al. 2000 Science 289: 625ff).

Daten zur Verteilung der sAC mRNA und zu Bicarbonat-stimulierbarer sAC Aktivität lassen auf eine Testis- und Spermienspezifische Expression des Enzyms schließen (ML Sinclair et al. 2000 Mol Reprod Develop 56:6ff; N Okamura et al. 1985 J. Biol. Chem 260(17):9699ff; J. Buck et al. 1999 PNAS 96:79ff). Im Hoden wird sAC mRNA dabei nur in späteren Stadien der sich zu Spermien entwickelnden Keimzellen exprimiert, nicht aber in somatischen Zellen (ML Sinclair *et al.* 2000 Mol Reprod Develop 56:6ff).

Zur Funktion der sAC in Spermien in Säugetieren gibt es eine Reihe pharmakologischer Untersuchungen. Spermien müssen, bevor sie die Zona Pellucida des Eies durchdringen können, um anschließend mit dem Oolemma des Eies zu verschmelzen, für diese Funktionalität vorbereitet sein. Dieser Prozess, die Spermienkapazitation, ist recht gut untersucht. Ein kapazitiertes Spermium zeichnet sich durch ein verändertes Bewegungsmuster aus und durch die Fähigkeit, durch einen geeigneten Stimulus den Prozess der akrosomalen Reaktion (einer Ausschüttung lytischer Enzyme, die vermutlich dem Durchdringen der Zona Pellucida durch das Spermium dienen) zu durchlaufen. Die Spermienkapazitation erfolgt *in vivo* und *in vitro* u.a. abhängig von einer erhöhten Bikarbonatkonzentration im Medium (PE Visconti & GS Kopf (1998) Biol Reprod 59:1ff; E de Lamirande et al. 1997 Mol Hum Reprod 3(3):175ff). Die Spermienkapazitation kann auch stimuliert werden durch die Zugabe geeigneter membrangängiger cAMP-Analoga, z.B. db-cAMP, und einem Inhibitor, der deren Abbau hemmt (z.B. IBMX). Die vermutete Abhängigkeit der Spermienfunktion von sAC wurde erst kürzlich durch ein genetisches Deletionsmodell, eine sog. Knock-out Maus, bestätigt (G Esposito et al. 2004 PNAS 101(9):2993ff). Männliche Mäuse, denen das Gen für sAC fehlt, zeigen eine normal verlaufende Spermatogenese, sind aber infertil. Die Spermien haben Bewegungsdefekte und sind nicht in der Lage, ein Ei zu befruchten. Die Tiere zeigten keine sonstigen Defekte oder abnorme Befunde, was gegen andere hypothetisierte Funktionen der sAC spricht (JH Zippin et al 2003 FASEB 17:82ff).

Die sAC hat eine einzigartige Sequenz und nur eine geringe Homologie zu anderen somatischen Adenylatzyklasen. Sie ist die einzige Adenylatzyklase im Säugetiersperma und die Aktivität ist für die Beweglichkeit der Spermien und die Kapazitation essentiell. Spezifische Inhibitoren der sAC stellen demnach eine wichtige Möglichkeit dar, die männliche Fertilität zu regulieren.

Die erfindungsgemäßen Verbindungen der allgemeinen Formel I sind hervorragende Inhibitoren der löslichen Adenylatzyklase.

Gegenstand der vorliegenden Erfindung sind Arzneimittel, die mindestens eine der Verbindungen gemäß Formel I enthalten.

Ebenfalls Gegenstand der vorliegenden Erfindung sind Arzneimittel, die die erfindungsgemäßen Verbindungen enthalten, mit geeigneten Formulierungs-und Trägerstoffen.

Zur Verwendung der erfindungsgemäßen Verbindungen als Arzneimittel werden diese in die Form eines pharmazeutischen Präparats gebracht, das neben dem Wirkstoff für die enterale oder parenterale Applikation geeignete pharmazeutische, organische oder anorganische inerte Trägermaterialien, wie zum Beispiel, Wasser, Gelatine, Gummi arabicum, Milchzucker, Stärke, Magnesiumstearat, Talk, pflanzliche Öle, Polyalkylenglykole usw. enthält. Die pharmazeutischen Präparate können in fester Form, zum Beispiel als Tabletten, Dragees, Suppositorien, Kapseln oder in flüssiger Form, zum Beispiel als Lösungen, Suspensionen oder Emulsionen vorliegen. Gegebenenfalls enthalten sie darüber hinaus Hilfsstoffe, wie Konservierungs-, Stabilisierungs-, Netzmittel oder Emulgatoren; Salze zur Veränderung des osmotischen Drucks oder Puffer. Diese pharmazeutischen Präparate sind ebenfalls Gegenstand der vorliegenden Erfindung.

Für die parenterale Anwendung sind insbesondere Injektionslösungen oder Suspensionen, insbesondere wässrige Lösungen der aktiven Verbindungen in polyhydroxyethoxyliertem Rizinusöl, geeignet.

Als Trägersysteme können auch grenzflächenaktive Hilfsstoffe wie Salze der Gallensäuren oder tierische oder pflanzliche Phospholipide, aber auch Mischungen davon sowie Liposomen oder deren Bestandteile verwendet werden.

Für die orale Anwendung sind insbesondere Tabletten, Dragees oder Kapseln mit Talkum und/oder Kohlenwasserstoffträger oder -binder, wie zum Beispiel Lactose, Mais- oder Kartoffelstärke, geeignet. Die Anwendung kann auch in flüssiger Form erfolgen, wie zum Beispiel als Saft, dem gegebenenfalls ein Süßstoff beigefügt ist.

Für die vaginale Applikation sind z. B. Suppositorien geeignet und üblich.

Die enteralen, parenteralen, vaginalen und oralen Applikationen sind ebenfalls Gegenstand der vorliegenden Erfindung.

Die Dosierung der Wirkstoffe kann je nach Verabfolgungsweg, Alter und Gewicht des Patienten, Art und Schwere der zu behandelnden Erkrankung und ähnlichen Faktoren variieren. Die tägliche Dosis beträgt 0,5 - 1000 mg, vorzugsweise 50 - 200 mg, wobei die Dosis als einmal zu verabreichende Einzeldosis oder unterteilt in 2 oder mehrere Tagesdosen gegeben werden kann.

Inhibitoren der löslichen Adenylatzyklase führen zu einer Erniedrigung des cAMP Signals. Der cAMP-Spiegel ist entscheidend für die Kontrolle der Prozesse, die bei der Zellproliferation, der Zelldifferenzierung und der Apoptose eine wichtige Rolle spielen. Erkrankungen, wie z.B. Krebs, bei denen die Erniedrigung des cAMP-Spiegels entscheidend

sind, können durch Inhibitoren der löslichen Adenylatzyklase moduliert werden. Diese Modulation kann prophylaktische und therapeutische Effekte haben für die Patienten, die an einer derartigen Erkrankung leiden. Im Moment werden Erkrankungen, die wie Krebs mit einer erhöhten Zellproliferation einhergehen, z.B. durch Strahlentherapie und Chemotherapie behandelt. Diese Verfahren sind unspezifisch und haben ein hohes Nebenwirkungspotential. Die Bereitstellung von neuen Substanzen, die direkt an bestimmten Zielorten eingreifen, sind deshalb vorteilhaft. Gegenstand der vorliegenden Erfindung sind Substanzen, die die cAMP-Produktion durch die Inhibition der löslichen Adenylatzyklase modulieren. So kann beispielsweise die anomale Zellproliferation erniedrigt oder gehemmt werden durch eine Regulation oder Inhibition der cAMP Produktion. Durch den Einsatz der erfindungsgemäßen Substanzen kann die lösliche Adenylatzyklase inhibiert werden, dies hat eine Erniedrigung der Zellproliferation zur Folge.
Gegenstand der vorliegenden Erfindung sind Substanzen, gemäß der allgemeinen Formel I, die die Aktivität der löslichen Adenylatzyklase reduzieren oder inhibieren.

Ebenfalls Gegenstand der vorliegenden Erfindung sind Arzneimittel, die mindestens eine Verbindung gemäß der allgemeinen Formel I enthalten, zur Behandlung von Erkrankungen, die dadurch gekennzeichnet sind, dass sie durch Störungen des Stoffwechsels des second messengers cAMP verursacht werden.

Der Einsatz der erfindungsgemäßen Substanzen führt zu einer Inhibition der löslichen Adenylatzyklase und so zu einer Erniedrigung der cAMP-Konzentration und daraus resultierend zu einer Reduktion oder Inhibition der Spermienkapazitation.

Gegenstand der vorliegenden Erfindung ist die Verwendung der erfindungsgemäßen Substanzen zur Erniedrigung und/oder zur Hemmung der männlichen Keimzell-Fertilität, vermittelt durch die Reduktion oder Inhibition der löslichen Adenylatzyklase Aktivität und dadurch resultierend der Spermienkapazitation.

Durch die Administration einer wirksamen Menge einer Substanz, die zur Inhibition der cAMP-Produktion führt, wird die Befruchtung des Ovums verhindert.
Ebenfalls Gegenstand der vorliegenden Erfindung ist die Verwendung der Verbindung der allgemeinen Formel I zur Herstellung eines empfängnisverhütenden Mittels.

Ebenfalls Gegenstand der vorliegenden Erfindung ist ein Suppositorium, dadurch gekennzeichnet, dass es mindestens eine der Verbindungen gemäß Formel I enthält und beispielsweise der weiblichen Kontrazeption dient.

Soweit die Herstellung der Ausgangsverbindungen nicht beschrieben wird, sind diese bekannt oder analog zu bekannten Verbindungen oder hier beschriebenen Verfahren herstellbar. Es ist ebenfalls möglich, alle hier beschriebenen Umsetzungen in Parallel-Reaktoren oder mittels kombinatorischer Arbeitstechniken durchzuführen.

Die Isomerengemische können nach üblichen Methoden wie beispielsweise Kristallisation, Chromatographie oder Salzbildung in die Enantiomeren bzw. E/Z-Isomeren aufgetrennt werden.

Die Herstellung der Salze erfolgt in üblicher Weise, indem man eine Lösung der Verbindung der Formel I mit der äquivalenten Menge oder einem Überschuss einer Base oder Säure, die gegebenenfalls in Lösung ist, versetzt und den Niederschlag abtrennt oder in üblicher Weise die Lösung aufarbeitet.

**Herstellung der erfindungsgemäßen Verbindungen**

Die nachfolgenden Beispiele erläutern die Herstellung der erfindungsgemäßen Verbindungen der allgemeinen Formel (I), ohne den Umfang der beanspruchten Verbindungen auf diese Beispiele zu beschränken.
Die erfindungsgemäßen Verbindungen der allgemeinen Formel (I) lassen sich wie nachstehend beschrieben herstellen.

**Beispiel 1**: 5-(4-tert-Butyl-phenylsulfonylamino)-3-phenyl-1 H-pyrrolo[3,2-b]pyridin-2-carbonsäure (tetrahydro-pyran-4-yl)-amid

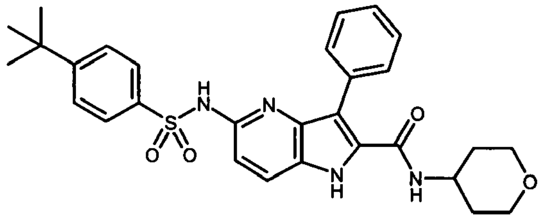

Zu einer Lösung von 50 mg der in Beispiel 1j) hergestellten Säure in 0.90 ml Dimethylformamid gibt man 46.1 mg N-[(Dimethylamino)-1*H*-1,2,3-triazolo[4,5-*b*]pyridin-1-ylmethylen]-*N*-Methylmethanaminiumhexafluorophosphat-N-oxid (HATU) und 11.3 mg 4-Aminotetrahydropyran. Bei 0 ˚ C werden dann 20.7 µl Ethyldiisopropylamin zugetropft und 20 Stunden bei Raumtemperatur gerührt. Anschließend gibt man 25 ml Wasser zu, rührt 30 Minuten und saugt über eine G4-Fritte ab. Der so erhaltene Rückstand wird durch Chromatographie an Kieselgel mit Hexan/0 - 90 % Ethylacetat gereinigt. Man erhält auf diese Weise 38 mg der Titelverbindung.
NMR (300 MHz, DMSO-d6): δ = 1.20 (9H), 1.27 (2H), 1.67 (2H), 3.33 (2H), 3.69 (2H), 3.91 (1H), 6.91 (1H), 7.26-7.58 (8H), 7.72 (1H), 7.75 (2H), 10.72 (1H), 11.82 (1 H).

Das Ausgangsmaterial für die obige Titelverbindung wird wie folgt hergestellt:

1a) 6-Methyl-5-nitro-pyridin-2-ylamin

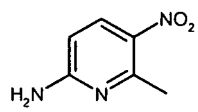

Zu einer Lösung von 211 g 6-Amino-2-picolin in 994 ml konzentrierter Schwefelsäure tropft man bei 0 ˚ C eine gekühlte Mischung aus 145 ml konzentrierter Salpetersäure und 145 ml konzentrierter Schwefelsäure. Die Reaktionsmischung wird eine Stunde bei 0 ˚ C und anschließend 16 Stunden bei 25 ˚ C gerührt. Anschließend wird auf 60 ˚ C für eine Stunde und abschließend auf 100 ˚ C für eine Stunde erhitzt. Die Reaktionsmischung wird dann vorsichtig auf Eis gegeben und mit einer konzentrierten NatriumhydroxidLösung auf einen pH-Wert von 5 bis 6 eingestellt. Der so erhaltene Feststoff wird abfiltriert und mit Ethanol gewaschen. Das so erhaltene Rohprodukt wird durch Säulenchromatographie an Kieselgel mit Chloroform/0 - 50 % Methanol vorgereinigt. Das Material wird dann in 100 ml 1 N Salzsäure teilweise gelöst, vom Niederschlag abfiltriert und die Lösung mit Natriumcarbonat versetzt. Den so erhaltenen Feststoff gewinnt man durch Filtration. Nach Trocknung erhält man 33 g der Titelverbindung als gelb-orange gefärbten Feststoff.
NMR (300 MHz, DMSO-d6): δ = 2.60 (3H), 6.37(1 H), 7.35 (2H), 8.09 (1 H).

1 b) 6-Methyl-5-nitro-2-(N,N-di-tert-butyloxycarbonylamino)pyridin

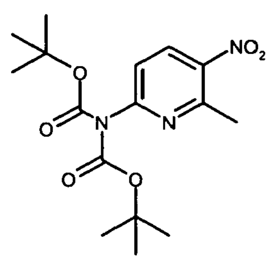

Zu einer Lösung von 24.3 g der vorstehend hergestellten Nitro-Verbindung und 0.35 g DMAP in 500 ml Methylenchlorid gibt man eine Lösung von 69 g $Boc_2O$ in 500 ml Methylenchlorid und rührt für 16 Stunden bei 25 ˚ C. Anschließend wäscht man die organische Phase dreimal mit je 700 ml Wasser und trocknet über Natriumsulfat. Nach Filtration erhält man ohne weitere Reinigung 49 g der Titelverbindung als gelb gefärbten Feststoff.
NMR (300 MHz, DMSO-d6): δ = 1.46 (18H), 2.69 (3H), 7.63 (1 H), 8.51 (1 H).

1c) 3-(6-tert-Butoxycarbonylamino-3-nitro-pyridin-2-yl)-2-oxo-propionsäure ethylester

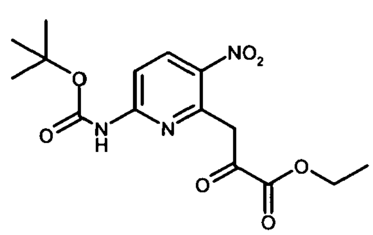

Zu einer Lösung von Natriumethylat in Ethanol (hergestellt durch Auflösen von 6.7 g Natrium in 490 ml Ethanol) gibt man 49 g der vorstehend hergestellten Picolin-Derivates. Nach 10 min. Rühren gibt man 42.6 g Oxalsäure diethylester in einer Portion hinzu und rührt die Mischung 5 Tage bei 25 ˚ C. Nach Zugabe von 1000 ml Ether wird der gebildete Niederschlag abfiltriert, anschließend in Wasser suspendiert und mit 1 N Salzsäure auf pH 4 angesäuert. Es wird abfiltriert, der Feststoff mit Wasser gewaschen und getrocknet. Man erhält auf diese Weise 18 g der Titelverbindung.
NMR (300 MHz, DMSO-d6): δ = 1.29 (3H), 1.51 (9H),. 4.28 (2H), 7.30 (1 H), 7.37 (1 H), 8.51 (1 H), 11.06 (1 H), 13.42 (1 H).

1d) 5-tert-Butoxycarbonylamino-1 H-pyrrolo[3,2-b]pyridin-2-carbonsäure ethylester

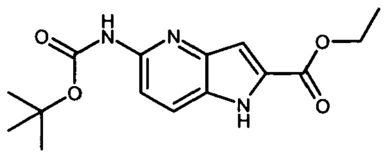

18 g des in Beispiel 1c) hergestellten Esters werden in 500 ml Ethanol gelöst und mit 3 g Palladium auf Kohle in einem Autoklaven bei einem Druck von 20 atm Wasserstoff und einer Temperatur von 50 ˚ C für 6 Stunden hydriert. Nach Filtration und Einengen im Vakuum wird das so erhaltene Rohprodukt durch Säulenchromatographie an Kieselgel mit Chloroform/0 - 10 % Essigester gereinigt. Man erhält auf diese Weise 4.2 g der Titelverbindung.
NMR (300 MHz, DMSO-d6): δ = 1.34 (3H), 1.46(9H), 4.35(2H), 6.99 (1H), 7.78(2H), 9.63(1 H), 12.03(1 H).

1e) 5-Amino-1H-pyrrolo[3,2-b]pyridin-2-carbonsäure ethylester

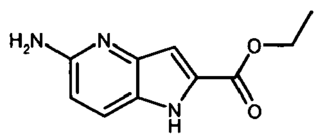

Zu 4.2 g der in Beispiel 1d) hergestellten Verbindung gibt man 30 ml einer 4 M Lösung von HCl in Dioxan und rührt 16 Stunden bei 25 ˚ C. Nach Filtration und Einengen im Vakuum wird der Rückstand in 30 ml Wasser gelöst und mit 50 ml Ether extrahiert. Dann wird die wässrige Phase mit gesättigter Natriumcarbonat-Lösung auf einen pHWert von 10 bis 11 eingestellt und mit einem Gemisch aus Chloroform und Methanol im Verhältnis 10 : 1 extrahiert. Die vereinigten organischen Phasen werden mit gesättigter Natriumchlorid-Lösung gewaschen, über Natriumsulfat getrocknet und nach Filtration im Vakuum eingeengt. Man erhält auf diese Weise 2.7 g der Titelverbindung.
NMR (300 MHz, DMSO-d6): δ = 1.31 (3H), 4.30 (2H), 5.68 (2H), 6.52(1 H), 6.73 (2H), 7.58 (1 H), 11.59 (1 H).

1f) 5-(4-tert-Butyl-phenylsulfonylamino)-1 H-pyrrolo[3,2-b]pyridin-2-carbonsäure ethylester

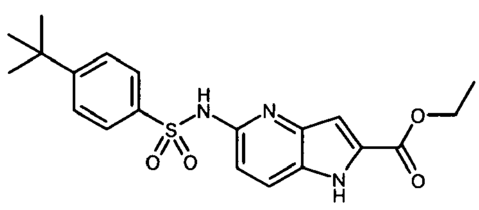

Zu einer Lösung von 2 g des in Beispiel 1f) hergestellten Amins in 97 ml Pyridin gibt man bei 25 ˚ C 2.27 g 4-tert.-Butyl-phenylsulfonylchlorid und rührt 20 Stunden bei dieser Temperatur. Unter Zusatz von Toluol wird das Lösungsmittel unter reduziertem Druck entfernt und der Rückstand durch Chromatographie an Kieselgel mit Hexan/ 0 - 80 % Ethylacetat gereinigt. Man erhält auf diese Weise 3.3 g der Titelverbindung.
NMR (300 MHz, DMSO-d6): δ = 1.21 (9H), 1.29 (3H), 4.29 (2H), 6.88 (1 H), 7.13 (1 H), 7.52 (2H), 7.76 (1 H), 7.80 (2H), 11.38 (1 H), 12.17 (1 H).

1g) 3-Brom-5-(4-tert-butyl-phenylsulfonylamino)-1 H-pyrrolo[3,2-b]pyridin-2-carbonsäure ethylester

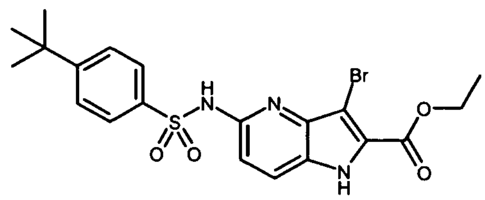

Zu einer Lösung von 500 mg des in Beispiel 1f) hergestellten Sulfonamids in 14 ml Tetrahydrofuran gibt man 222 mg N-Bromsuccinimid und rührt 60 Minuten bei 25 ˚ C. Man verdünnt mit 200 ml Ethylacetat, wäscht zweimal mit 20 ml halbkonzentrierter Natriumchlorid-Lösung und trocknet die organische Phase über Natriumsulfat. Nach Filtration und Einengen im Vakuum wird der so erhaltene Rückstand durch Chromatographie an Kieselgel mit Hexan/0 - 50 % Ethylacetat gereinigt. Man erhält auf diese Weise 585 mg der Titelverbindung.
NMR (300 MHz, DMSO-d6): δ = 1.22 (9H), 1.32 (3H), 4.33 (2H), 7.02 (1 H), 7.55 (2H), 7.76 (1 H), 8.02 (2H), 11.01 (1 H), 12.33 (1 H).

1 h) 1-tert-Butoxycarbonyl-3-Brom-5-[(tert-butoxycarbonyl)-(4-tert-butyl-phenylsulfonyl)-amino]-1 H-pyrrolo[3,2-b]pyridin-2-carbonsäure ethylester

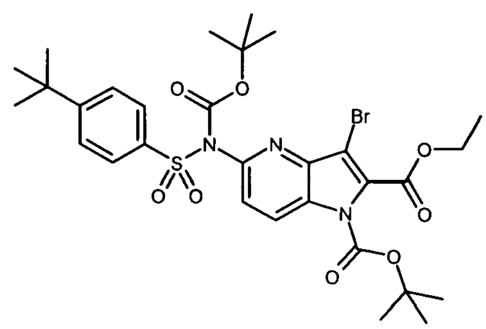

Zu einer Lösung von 638 mg des in Beispiel 1g) hergestellten Bromids in 6.6 ml Methylenchlorid gibt man 81 mg DMAP, 1.16 g Di-tert-butyl-dicarbonat und rührt 3 Tage bei 25 ˚ C. Nach Einengen im Vakuum wird der so erhaltene Rückstand durch Chromatographie an Kieselgel mit Hexan/0 - 50 % Ethylacetat gereinigt. Man erhält auf diese Weise 715 mg der Titelverbindung.
NMR (300 MHz, DMSO-d6): δ = 1.20 (9H), 1.31 (9H), 1.33 (3H), 1.58 (9H), 4.41 (2H), 7.69 (1 H), 7.72 (2H), 8.09 (2H), 8.47 (1 H).

1 i) 3-Phenyl-5-(4-tert-butyl-phenylsulfonylamino)-1 H-pyrrolo[3,2-b]pyridin-2-carbonsäure ethylester

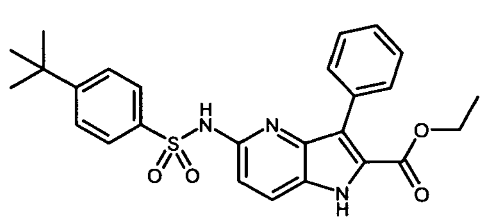

Zu einer Lösung von 350 mg des in Beispiel 1h) hergestellten geschützten Bromids in 11.2 ml Dioxan gibt man 627 mg Phenylboronsäure und 1.09 g Kaliumphosphat. Nach Zugabe von 75.3 mg 1,1'-Bis(diphenylphosphino)-ferrocendichlor palladium(II) wird die Reaktionsmischung 20 Stunden auf 90 ˚ C erhitzt. Nach Abkühlen verdünnt man mit 200 ml Ethylacetat und wäscht die organische Phase zweimal mit je 20 ml einer halbkonzentrierten Natriumchlorid-Lösung und trocknet über Natriumsulfat. Nach Filtration und Einengen im Vakuum wird der so erhaltene Rückstand durch Chromatographie an Kieselgel mit Hexan/0 - 50 % Ethylacetat gereinigt. Man erhält auf diese Weise 214 mg 1-tert-Butoxycarbonyl-3-phenyl-5-[(tert-butoxycarbonyl)-(4-tert-butyl-phenylsulfonyl)-amino]-1 H-pyrrolo[3,2-b]pyridin-2-carbonsäure ethylester. Hiervon werden 210 mg mit 5 ml Trifluoressigsäure versetzt und 20 Stunden bei 25 ˚ C gerührt. Nach Zusatz von Toluol wird im Vakuum eingeengt. Der so erhaltene Rückstand durch Chromatographie an Kieselgel mit Hexan/0 - 50 % Ethylacetat gereinigt. Man erhält auf diese Weise 135 mg der Titelverbindung.
NMR (300 MHz, DMSO-d6): δ = 1.14 (3H), 1.19 (9H), 4.18 (2H), 6.97 (1 H), 7.31-7.44 (7H), 7.72 (2H), 7.77 (1 H), 10.79 (1 H), 11.99 (1 H).

1j) 3-Phenyl-5-(4-tert-butyl-phenylsulfonylamino)-1 H-pyrrolo[3,2-b]pyridin-2-carbonsäure

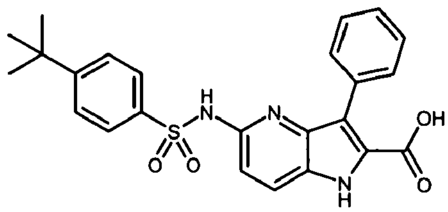

Zu einer Mischung von 130 mg des in Beispiel 1i) hergestellten Esters in 3.2 ml Ethanol und 2 ml Tetrahydrofuran gibt man 210 mg Natriumhydroxid, gelöst in 1.6 ml Wasser, und rührt 20 Stunden bei 25 ˚ C. Anschließend wird mit 100 ml Wasser verdünnt und mit 5 %iger wässriger Schwefelsäure angesäuert. Der ausgefallene Niederschlag wird abfiltriert und getrocknet. Man erhält auf diese Weise 122 mg der Titelverbindung, die ohne weitere Reinigung weiter umgesetzt wird.
NMR (300 MHz, DMSO-d6): δ = 1.19 (9H), 6.96 (1 H), 7.30-7.45 (7H), 7.71 (2H), 7.74 (1 H), 10.76 (1 H), 11.90 (1 H), 13.02 (1 H).

**Beispiel 2**: 5-(4-tert-Butyl-phenylsulfonylamino)-3-phenyl-1H-pyrrolo[3,2-b]pyridin-2-carbonsäure (2-morpholin-4-yl-ethyl)-amid

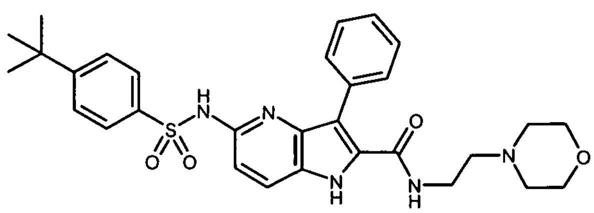

In Analogie zu Beispiel 1 erhält man aus 50 mg der Säure aus Beispiel 1j) und 14.5 mg 2-(Morpholin-4-yl)-ethylamin 30 mg der Titelverbindung.
NMR (300 MHz, DMSO-d6): δ = 1.25 (9H), 2.21-2.39 (6H), 3.30 (2H), 3.44 (4H), 6.96 (1 H), 7.21 (1 H), 7.39-7.52 (7H), 7.76 (3H), 10.76 (1 H), 11.86 (1 H).

**Beispiel 3**: 5-(4-tert-Butyl-phenylsulfonylamino)-3-phenyl-1 H-pyrrolo[2,3-c]pyridin-2-carbonsäure (2-morpholin-4-yl-ethyl)-amid

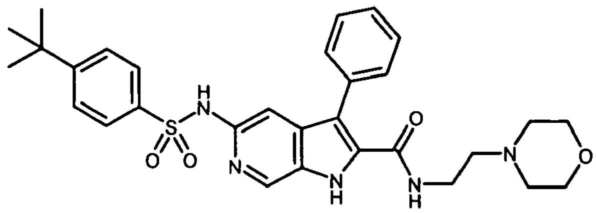

In Analogie zu Beispiel 1) erhält man aus 108 mg der in Beispiel 3i) hergestellten Säure und 31.4 mg 2-(Morpholin-4-yl)-ethylamin 54 mg der Titelverbindung.
NMR (300 MHz, DMSO-d6): δ = 1.21 (9H), 2.16-2.31 (6H), 3.27 (2H), 3.40 (4H), 7.19 (1 H), 7.34-7.41 (3H), 7.43 (1 H), 7.46-7.56 (4H), 7.67 (2H), 8.41 (1 H), 10.48 (1 H), 12.13 (1 H).

Das Ausgangsmaterial für die obige Titelverbindung wird wie folgt hergestellt:

3a) 4-Methyl-5-nitro-2-(N,N-di-tert-butyloxycarbonylamino)pyridin

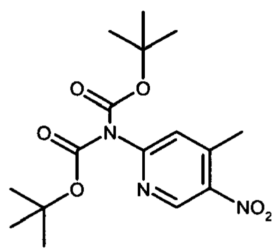

Zu einer Lösung von 1.0 g von 2-Amino-4-methyl-5-nitropyridin und 15 mg DMAP in 20 ml Methylenchlorid gibt man eine Lösung von 2.85 g $Boc_2O$ in 10 ml Methylenchlorid und rührt für 16 Stunden bei 25 ° C. Anschließend wäscht man die organische Phase dreimal mit je 50 ml Wasser und trocknet über Natriumsulfat. Nach Filtration erhält man ohne weitere Reinigung 2.2 g der Titelverbindung als dunkelgelb gefärbten Feststoff.
NMR (300 MHz, DMSO-d6): δ = 1.45 (18H), 2.62 (3H), 7.63 (1 H), 9.04 (1 H).

3b) 3-(6-tert-Butoxycarbonylamino-3-nitro-pyridin-4-yl)-2-oxo-propionsäure ethylester

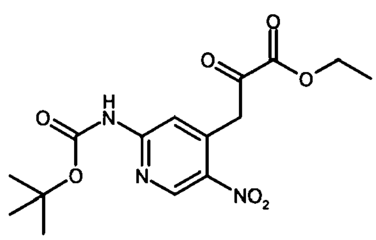

Zu einer Lösung von Natriumethylat in Ethanol (hergestellt durch Auflösen von 10.6 g Natrium in 770 ml Ethanol) gibt man 77 g des in Beispiel 3a) hergestellten Pyridin-Derivates. Nach 5 min. Rühren gibt man 67.3 g Oxalsäure diethylester in einer Portion hinzu und rührt die Mischung 7 Tage bei 25 ° C. Während dieser Zeit entsteht ein Niederschlag, der abfiltriert und mit 20 ml Ethanol gewaschen wird. Der Niederschlag wird in möglichst wenig Wasser suspendiert und der pH-Wert mittels einer 1 molaren Salzsäure auf 4 eingestellt. Nach wiederholter Filtration wird mit wenig Wasser und nach kurzem Trocknen mit Hexan gewaschen. Nach dem Trocknen an der Luft erhält man 53.0 g der Titelverbindung als gelb-orange gefärbten Feststoff.
NMR (300 MHz, DMSO-d6): δ = 1.24 (3H), 1.48 (9H), 4.93 (2H), 6.64 (1 H), 8.66 (1 H), 9.32 (1 H), 9.43 (1 H).

3c) 5-tert-Butoxycarbonylamino-1H-pyrrolo[2,3-c]pyridin-2-carbonsäure ethylester

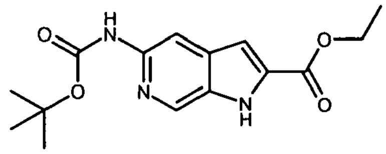

52 g des in Beispiel 2b) hergestellten Esters werden in 550 ml Ethanol gelöst und mit 6 g Palladium auf Kohle in einem Autoklaven bei einem Druck von 40 - 45 atm Wasserstoff und einer Temperatur von 50 ˚ C für 5 Stunden hydriert. Nach Filtration und Einengen im Vakuum erhält man 21.5 g der Titelverbindung, die ohne weitere Reinigung weiter umgesetzt wird.
NMR (300 MHz, DMSO-d6): δ = 1.15 (3H), 1.46 (9H), 4.35 (2H), 7.09 (1 H), 7.95 (1 H), 8.54 (1 H), 9.44 (1 H).

3d) 5-Amino-1H-pyrrolo[2,3-c]pyridin-2-carbonsäure ethylester

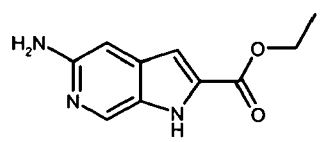

Zu 21 g der in Beispiel 3c) hergestellten Verbindung gibt man 40 ml einer 4 M Lösung von HCl in Dioxan und rührt 3 Stunden bei 25 ˚ C. Nach Filtration und Einengen im Vakuum wird der Rückstand in 100 ml Wasser gelöst. Die wässrige Phase wird mit gesättigter Natriumcarbonat-Lösung auf pH 10 bis 11 eingestellt und mit einem Gemisch aus Chloroform und t-Butanol im Verhältnis 8 : 1 extrahiert. Die vereinigten organischen Phasen werden mit gesättigter Natriumchlorid-Lösung gewaschen, über Natriumsulfat getrocknet und nach Filtration im Vakuum eingeengt. Man erhält auf diese Weise 7.5 g der Titelverbindung.
NMR (300 MHz, DMSO-d6): δ = 1.32 (3H), 4.32 (2H), 5.17 (1 H), 6.57 (1 H), 6.83 (1 H), 8.32 (1 H), 11.70 (1 H).

3e) 5-(4-tert-Butyl-phenylsulfonylamino)-1 H-pyrrolo[2,3-c]pyridin-2-carbonsäure ethylester

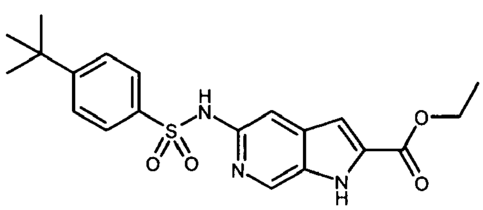

In Analogie zu Beispiel 1f) erhält man aus 1.93 g des in Beispiel 3d) hergestellten Amins 3.2 g der Titelverbindung.
NMR (300 MHz, DMSO-d6): δ = 1.20 (9H), 1.29 (3H), 4.32 (2H), 7.08 (1 H), 7.41 (1 H), 7.50 (2H), 7.72 (2H), 8.43 (1 H), 10.49 (1 H), 12.23 (1 H).

3f) 3-Brom-5-(4-tert-butyl-phenylsulfonylamino)-1 H-pyrrolo[2,3-c]pyridin-2-carbonsäure ethylester

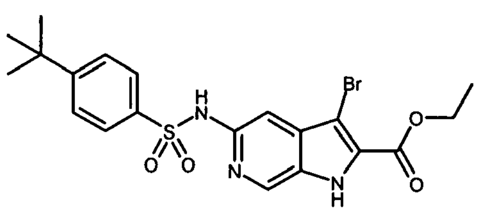

In Analogie zu Beispiel 1g) erhält man aus 3.2 g des in Beispiel 3e) hergestellten Esters 1.48 g der Titelverbindung.

NMR (300 MHz, 300 MHz, DMSO-d6): δ = 1.20 (9H), 1.32 (3H), 4.35 (2H), 7.22 (1 H), 7.52 (2H), 7.73 (2H), 8.47 (1 H), 10.70 (1 H), 12.60 (1 H).

3g) 1-tert-butoxycarbonyl-3-Brom-5-[(tert-butoxycarbonyl)-(4-tert-butyl-phenylsulfonyl)-amino]-1 H-pyrrolo[2,3-c] pyridin-2-carbonsäure ethylester

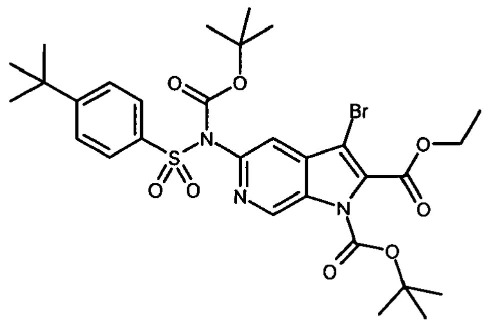

In Analogie zu Beispiel 1h) erhält man aus 1.48 g des in Beispiel 3f) hergestellten Bromids 1.68 g der Titelverbindung. NMR (300 MHz, DMSO-d6): δ = 1.17 (9H), 1.31 (9H), 1.33 (3H), 1.61 (9H), 4.41 (2H), 7.71 (2H), 7.77 (1 H), 8.03 (2H), 9.21 (1 H).

3h) 3-Phenyl-5-(4-tert-butyl-phenylsulfonylamino)-1 H-pyrrolo[2,3-c]pyridin-2-carbonsäure ethylester

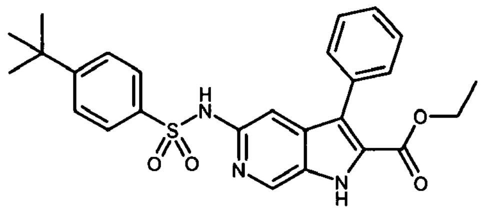

In Analogie zu Beispiel 1i) erhält man aus 300 mg des in Beispiel 3g) hergestellten Bromids 137 mg der Titelverbindung.
NMR (300 MHz, DMSO-d6): δ = 1.14 (3H), 1.21 (9H), 4.20 (2H), 7.18 (1 H), 7.34-7.41 (3H), 7.44 (2H), 7.52 (2H), 7.68 (2H), 8.50 (1 H), 10.51 (1 H), 11.29 (1H).

3i) 3-Phenyl-5-(4-tert-butyl-phenylsulfonylamino)-1H-pyrrolo[2,3-c]pyridin-2-carbonsäure

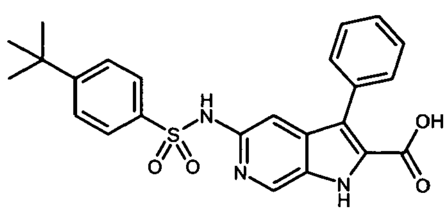

In Analogie zu Beispiel 1j) erhält man aus 120 mg des in Beispiel 3h) hergestellten Esters 112 mg der Titelverbindung. NMR (300 MHz, DMSO-d6): δ = 1.21 (9H), 7.16 (1 H), 7.32-7.47 (6H), 7.52 (2H), 7.66 (2H), 8.47 (1 H), 10.54 (1 H), 12.22 (1 H).

Beispiel 4 5-(4-tert-Butyl-phenylsulfonylamino)-3-phenyl-1 H-pyrrolo[2,3-b]pyridin-2-carbonsäure (2-morpholin-4-yl-ethyl)-amid

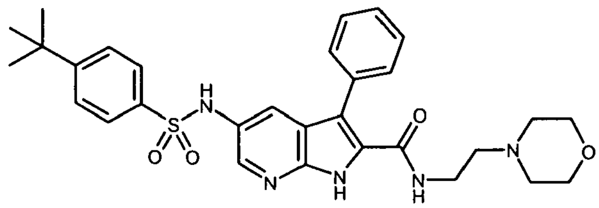

In Analogie zu Beispiel 1) erhält man aus 100 mg der in Beispiel 4h) hergestellten Säure und 29 mg 2-(Morpholin-4-yl)-ethylamin 63 mg der Titelverbindung.
NMR (300 MHz, DMSO-d6): δ = 1.26 (9H), 2.24-2.36 (6H), 3.30 (2H), 3.46 (4H), 7.30-7.53 (7H), 7.57 (4H), 8.08 (1 H), 10.01 (1 H), 12.32 (1 H).

Das Ausgangsmaterial für die obige Titelverbindung wird wie folgt hergestellt:

4a) 2-Hydroxy-5-nitro-nicotinsäure

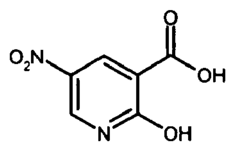

Eine Lösung von 210 g 2-Hydroxynicotinsäure in 1.5 l konz. Schwefelsäure wird auf - 5 ˚ C gekühlt und dann tropfenweise mit 100 ml konz. Salpetersäure versetzt. Anschließend lässt man auf 25 ˚ C erwärmen und rührt für 16 Stunden bei dieser Temperatur. Dann wird für 1 Stunde auf 50 ˚ C erhitzt und nach dem Abkühlen die Reaktionsmischung vorsichtig auf 3 kg Eis gegeben. Man filtriert den weißen Niederschlag ab, wäscht einmal mit Wasser und kristallisiert aus einem Wasser/Ethanol-Gemisch um. Man erhält auf diese Weise 190 g der Titelverbindung.
NMR (300 MHz, DMSO-d6): δ = 8.7 (1 H), 9.0 (1 H).

4b) (2-Chlor-5-nitro-pyridin-3-yl)-phenyl-keton

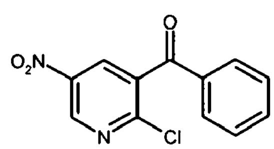

Zu 18.4 g der in Beispiel 4a) hergestellten Säure gibt man bei 25 ˚ C 120 ml Thionylchlorid gefolgt von 3 ml DMF und erhitzt anschließend für 3 Stunden am Rückfluss. Der Überschuss von Thionylchlorid wird im Vakuum entfernt und der zurückbleibende gelbe Feststoff in 100 ml Benzol gelöst. Zu dieser Lösung gibt man 40 g $AlCl_3$ bei 0 ˚ C und rührt dann für 16 Stunden bei 25 ˚ C. Die Reaktionsmischung wird dann vorsichtig in kalte (teils gefrorene) konz. Salzsäure gegossen, nach einer Stunde filtriert und die organische Phase abgetrennt. Die organische Phase wird mit Salzsäure gewaschen, über Natriumsulfat getrocknet und nach Filtration im Vakuum eingeengt. Der so erhaltene Rückstand durch Chromatographie an Kieselgel mit Hexan/10 % Ethylacetat gereinigt. Man erhält auf diese Weise 15 g der Titelverbindung.
NMR (300 MHz, DMSO-d6): δ = 7.60 (2H), 7.77 (1H), 7.87 (2H), 8.95 (1H), 9.4 (1 H).

4c) (3-Benzoyl-5-nitro-pyridin-2-yl)-(4-methoxy-benzyl)-amino]-essigsäure ethylester

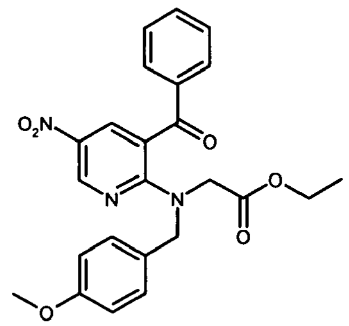

Zu einer Lösung von 18.4 g des in Beispiel 4b) hergestellten Ketons in 150 ml Acetonitril gibt man 23.1 g N-(4-Methoxy-benzyl)-glycin ethylester gefolgt von einer Lösung von 13 g Diisopropylethylamin in 100 ml Acetonitril. Nach 16 Stunden Rühren bei 25 ˚ C werden alle flüchtigen Bestandteile im Vakuum entfernt und der Rückstand in Ethylacetat gelöst. Diese Lösung wird dreimal mit je 100 ml Wasser gewaschen, über Natriumsulfat getrocknet und nach Filtration im Vakuum eingeengt. Nach Umkristallisation erhält man 32 g der Titelverbindung als weißen Feststoff.
NMR (300 MHz, DMSO-d6): δ = 1.1 (3H), 3.7 (3H), 4.0 (2H), 4.3 (2H), 4.7 (2H), 6.7 (2H), 7.0 (2H), 7.5 (2H), 7.7 (2H), 7.7 (1 H), 8.2 (1 H), 9.1 (1 H).

4d) 1-(4-Methoxy-benzyl)-5-nitro-3-phenyl-1 H-pyrrolo[2,3-b]pyridin-2-carbonsäure ethylester

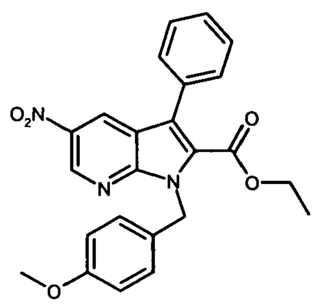

Zu einer Lösung von 32 g des in Beispiel 4c) hergestellten Esters in 50 ml Pyridin gibt man unter Argon 100 ml Essigsäureanhydrid zu und rührt anschließend für 16 Stunden bei 100 ˚ C. Dann werden alle flüchtigen Bestandteile im Vakuum entfernt und der so erhaltene Rückstand durch Chromatographie an Kieselgel mit Methylenchlorid gereinigt. Man erhält auf diese Weise 24.5 g der Titelverbindung.
NMR (300 MHz, DMSO-d6): δ = 1.0 (3H), 3.7 (3H), 4.2 (2H), 5.9 (2H), 6.9 (2H), 7.1 (2H), 7.5 (5H), 8.7 (1 H), 9.4 (1 H).

4e) 5-Nitro-3-phenyl-1 H-pyrrolo[2,3-b]pyridin-2-carbonsäure ethylester

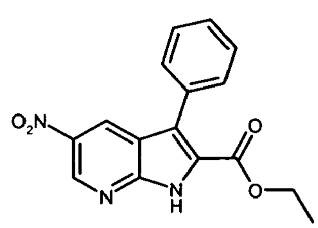

Zu einer Lösung von 27.2 g des in Beispiel 4d) hergestellten Indolderivates in 150 ml Trifluoressigsäure gibt man 2.5 ml Anisol und erhitzt diese Mischung 3 Tage am Rückfluss. Anschließend werden alle flüchtigen Bestandteile im Vakuum entfernt und der so erhaltene Rückstand durch Umkristallisation aus Ethylacetat gereinigt. Man erhält auf diese Weise 20 g der Titelverbindung.

NMR (300 MHz, DMSO-d6): δ = 1.2 (3H), 4.3 (2H), 7.5 (5H), 8.6 (1 H), 9.3 (1 H), 13.4 (1H).

4f) 5-Amino-3-phenyl-1 H-pyrrolo[2,3-b]pyridin-2-carbonsäure ethylester Hydrochlorid

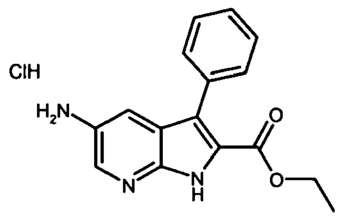

18 g des in Beispiel 4e) hergestellten Nitro-Indols werden in 500 ml Dioxan gelöst und mit 4 g Palladium auf Kohle in einem Autoklaven bei einem Druck von 50 psi Wasserstoff bei 25 ˚ C für 3 Tage hydriert. Nach Filtration und Einengen im Vakuum auf ca. 200 ml Volumen gibt man zu dieser Lösung 50 ml einer 7 M Lösung von HCl in Dioxan. Der gebildete Niederschlag wird abfiltriert, mit Dioxan gewaschen und getrocknet. Man erhält auf diese Weise 16 g der Titelverbindung als weißen Feststoff.
NMR (300 MHz, DMSO-d6): δ = 1.2 (3H), 4.2 (2H), 7.5 (5H), 8.0 (1 H), 8.5 (1 H), 10.4 (1 H), 12.9 (1 H).

4g) 5-(4-tert-Butyl-phenylsulfonylamino)-3-phenyl-1 H-pyrrolo[2,3-b]pyridin-2-carbonsäure ethylester

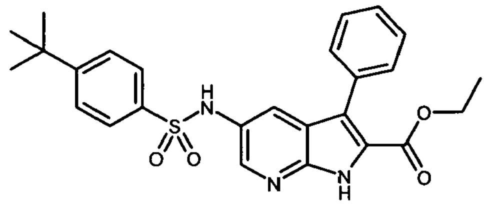

In Analogie zu Beispiel 1f) erhält man aus 2.0 g des in Beispiel 4f) hergestellten Amins 2.31 g der Titelverbindung.
NMR (300 MHz, DMSO-d6): δ = 1.16 (3H), 1.26 (9H), 4.21 (2H), 7.29-7.35 (2H), 7.38-7.48 (4H), 7.57 (4H), 8.18 (1 H), 10.07 (1 H), 12.60 (1 H).

4h) 3-Phenyl-5-(4-tert-butyl-phenylsulfonylamino)-1 H-pyrrolo[2,3-b]pyridin-2-carbonsäure

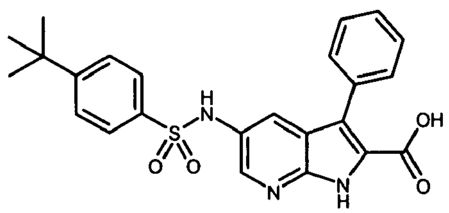

In Analogie zu Beispiel 1j) erhält man aus 2.3 g des in Beispiel 4g) hergestellten Esters 2.16 g der Titelverbindung.
NMR (300 MHz, DMSO-d6): δ = 1.21 (9H), 7.26-7.42 (6H), 7.52 (4H), 8.10 (1 H), 9.99 (1 H), 12.34 (1 H), 13.12 (1 H).

**Beispiel 5** 5-(4-tert-Butyl-phenylsulfonylamino)-3-phenyl-1H-pyrrolo[2,3-b]pyridin-2-carbonsäure (tetrahydro-pyran-4-yl)-amid

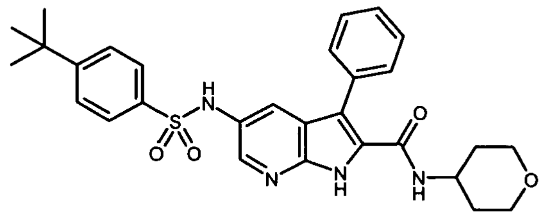

In Analogie zu Beispiel 1) erhält man aus 100 mg der in Beispiel 4h) hergestellten Säure und 23 mg 4-Aminotetrahydropyran 49 mg der Titelverbindung.
NMR (300 MHz, DMSO-d6): δ = 1.26 (9H), 1.35 (2H), 1.71 (2H), 3.35 (2H), 3.77 (2H), 3.93 (1 H), 7.28-7.52 (6H), 7.57 (4H), 7.86 (1 H), 8.07 (1 H), 10.02 (1 H), 12.32 (1 H).

**Beispiel 6** (±)-5-(4-tert-Butyl-phenylsulfonylamino)-3-phenyl-1H-pyrrolo[2,3-b]pyridin-2-carbonsäure (2-hydroxy-1-methyl-ethyl)-amid

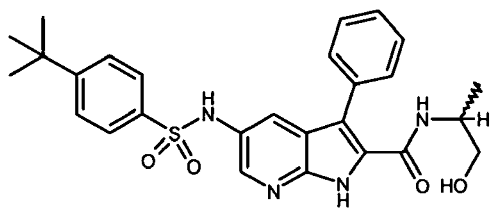

In Analogie zu Beispiel 1) erhält man aus 100 mg der in Beispiel 4h) hergestellten Säure und 17.7 µl (±)-2-Amino-propan-1-ol 75 mg der Titelverbindung.
NMR (300 MHz, DMSO-d6): δ = 0.97 (3H), 1.21 (9H), 3.18 (1 H), 3.30 (1 H), 3.89 (1H), 4.60 (1H), 7.23-7.45 (6H), 7.49-7.57 (6H), 8.03 (1H), 9.96 (1H), 12.23 (1 H).

**Beispiel 7** (±)-5-(4-tert-Butyl-phenylsulfonylamino)-3-phenyl-1H-pyrrolo[2,3-b]pyridin-2-carbonsäure (2-hydroxy-propyl)-amid

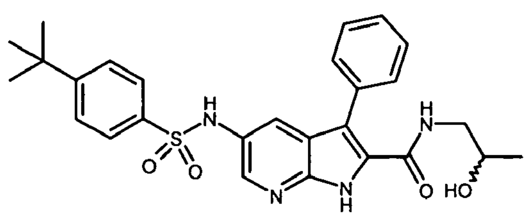

In Analogie zu Beispiel 1) erhält man aus 100 mg der in Beispiel 4h) hergestellten Säure und 17.6 µl (±)-2-Hydroxy-propylamin 88 mg der Titelverbindung.
NMR (300 MHz, DMSO-d6): δ = 0.93 (3H), 1.21 (9H), 3.18 (1 H), 3.08 (2H), 3.61 (1H), 4.60 (1H), 7.23-7.44 (6H), 7.52 (4H), 7.71 (1H), 8.04 (1H), 9.96 (1H), 12.23 (1 H).

**Beispiel 8** (1 S,2R)-5-(4-tert-Butyl-phenylsulfonylamino)-3-phenyl-1H-pyrrolo[2,3-b]pyridin-2-carbonsäure (2-hydroxy-cyclopentyl)-amid

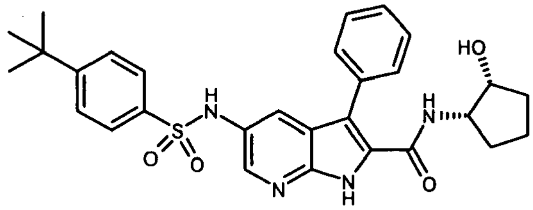

In Analogie zu Beispiel 1) erhält man aus 100 mg der in Beispiel 4h) hergestellten Säure und 30.6 mg (1S, 2R)-2-hydroxy-cyclopentylamin 88 mg der Titelverbindung.
NMR (300 MHz, DMSO-d6): δ = 1.21 (9H), 1.32-1.80 (6H), 3.87 (1 H), 3.95 (1 H), 4.52 (1H), 7.11 (1H), 7.24-7.44 (6H), 7.51 (4H), 8.05 (1H), 9.96 (1H), 12.29 (1 H).

Beispiel 9 5-(4-tert-Butyl-phenylsulfonylamino)-3-phenyl-1H-pyrrolo[2,3-b]pyridin-2-carbonsäure (pyridin-3-ylmethyl)-amid

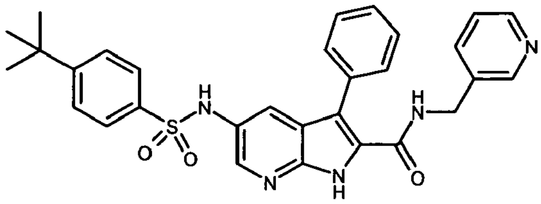

In Analogie zu Beispiel 1) erhält man aus 70 mg der in Beispiel 4h) hergestellten Säure und 15.9 µl 3-Pyridylmethylamin 58 mg der Titelverbindung.
NMR (300 MHz, DMSO-d6): δ = 1.25 (9H), 4.41 (2H), 7.24 (1 H), 7.26 (1 H), 7.29-7.43 (4H), 7.48 (1 H), 7.57 (4H), 7.65 (1 H), 8.08 (1 H), 8.46 (1 H), 8.50 (1 H), 8.59 (1 H), 10.03 (1 H), 12.37 (1 H).

**Beispiel 10** (±)-5-(4-tert-Butyl-phenylsulfonylamino)-3-phenyl-1H-pyrrolo[2,3-b]pyridin-2-carbonsäure (tetrahydrofuran-2-ylmethyl)-amid

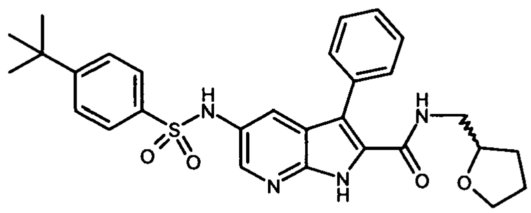

In Analogie zu Beispiel 1) erhält man aus 100 mg der in Beispiel 4h) hergestellten Säure und 22.5 mg (Tetrahydrofuran-2-yl)-methylamin 92 mg der Titelverbindung.
NMR (300 MHz, DMSO-d6): δ = 1.21 (9H), 1.40 (1 H), 1.64-1.84 (3H), 3.10-3.29 (2H), 3.52 (1 H), 3.61 (1 H), 3.79 (1 H), 7.23-7.44 (6H), 7.52 (4H), 7.70 (1 H), 8.03 (1 H), 8.96 (1 H), 12.25 (1 H).

**Beispiel 11** 5-(4-tert-Butyl-phenylsulfonylamino)-3-phenyl-1H-pyrrolo[2,3-b]pyridin-2-carbonsäure [2-(morpholine-4-sulfonyl)-ethyl]-amid

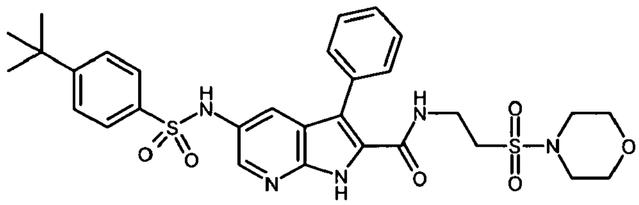

In Analogie zu Beispiel 1) erhält man aus 70 mg der in Beispiel 4h) hergestellten Säure und 35.9 mg 2-(Morpholin-4-sulfonyl)-ethylamin 58 mg der Titelverbindung.
NMR (300 MHz, DMSO-d6): δ = 1.26 (9H), 3.14 (4H), 3.21 (2H), 3.58 (2H), 3.64 (4H), 7.29-7.51 (6H), 7.57 (4H), 8.06 (1 H), 8.10 (1 H), 10.04 (1 H), 12.32 (1 H).

**Beispiel 12** 5-(4-tert-Butyl-phenylsulfonylamino)-3-phenyl-1 H-pyrrolo[2,3-b]pyridin-2-carbonsäure pyridin-4-ylamid

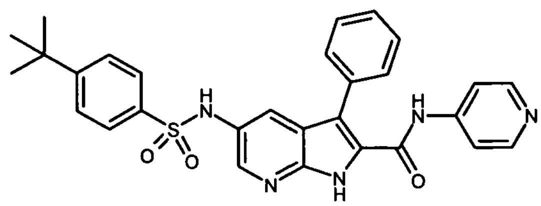

In Analogie zu Beispiel 1) erhält man aus 70 mg der in Beispiel 4h) hergestellten Säure und 14.7 mg 4-Amino-pyridin 25 mg der Titelverbindung.
NMR (300 MHz, DMSO-d6): δ = 1.26 (9H), 7.32-7.63 (13 H), 8.15 (1H), 8.46 (2H), 10.10 (1 H), 10.51 (1 H), 12.60 (1 H).

**Beispiel 13** 5-(4-tert-Butyl-phenylsulfonylamino)-3-phenyl-1H-pyrrolo[2,3-b]pyridin-2-carbonsäure [2-(2-oxo-imidazolidin-1-yl)-ethyl]-amid

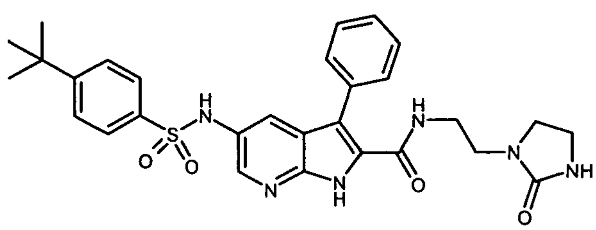

In Analogie zu Beispiel 1) erhält man aus 70 mg der in Beispiel 4h) hergestellten Säure und 40.2 mg 1-(2-Amino-ethyl)-imidazolidin-2-on 57 mg der Titelverbindung.
NMR (300 MHz, DMSO-d6): δ = 1.21 (9H), 3.12 (4H), 3.25 (4H), 6.30 (1 H), 7.22-7.45 (6 H), 7.52 (4H), 7.93 (1 H), 8.03 (1 H), 9.96 (1 H), 12.15 (1 H).

**Beispiel 14** 5-(4-tert-Butyl-phenylsulfonylamino)-3-phenyl-1 H-pyrrolo[2,3-b]pyridin-2-carbonsäure ethylamid

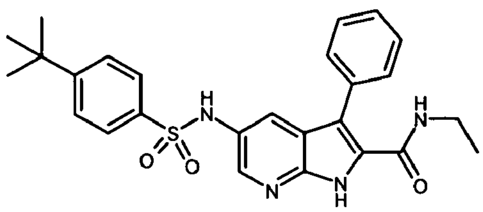

In Analogie zu Beispiel 1) erhält man aus 70 mg der in Beispiel 4h) hergestellten Säure und 80 µl Ethylamin 42 mg der Titelverbindung.
NMR (300 MHz, DMSO-d6): δ = 0.98 (3H), 1.21 (9H), 3.15 (2H), 7.22-7.46 (6 H), 7.52 (4H), 7.87 (1 H), 8.02 (1 H), 9.96 (1 H), 12.22 (1 H).

**Beispiel 15** 5-(4-tert-Butyl-phenylsulfonylamino)-3-phenyl-1H-pyrrolo[2,3-b]pyridin-2-carbonsäureamid

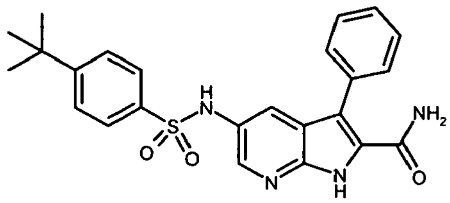

In Analogie zu Beispiel 1) erhält man aus 70 mg der in Beispiel 4h) hergestellten Säure und 75 µl 2M $NH_3$-Lösung in Methanol 40 mg der Titelverbindung.
NMR (300 MHz, DMSO-d6): δ = 1.26 (9H), 7.19 (2H), 7.28-7.49 (6 H), 7.56 (4H), 8.08 (1 H), 10.01 (1 H), 12.23 (1 H).

**Beispiel 16** 5-(4-tert-Butyl-phenylsulfonylamino)-3-phenyl-1 H-pyrrolo[2,3-b]pyridin-2-carbonsäure (2-sulfamoyl-ethyl)-amid

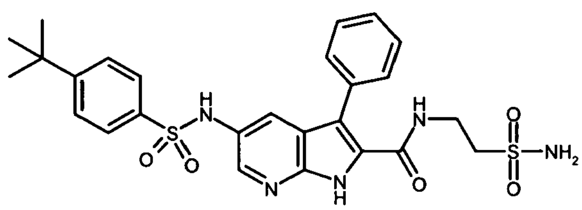

In Analogie zu Beispiel 1) erhält man aus 70 mg der in Beispiel 4h) hergestellten Säure und 19.3 mg 2-Amino-ethansulfonsäureamid 60 mg der Titelverbindung.
NMR (300 MHz, DMSO-d6): δ = 1.26 (9H), 3.14 (2H), 3.58 (2H), 6.94 (2H), 7.27-7.51 (6 H), 7.57 (4H), 8.09 (1 H), 8.13 (1 H), 10.03 (1 H), 12.29 (1 H).

**Beispiel 17** 5-(Biphenyl-4-sulfonylamino)-3-phenyl-1 H-pyrrolo[2,3-b]pyridin-2-carbonsäure (2-morpholin-4-yl-ethyl)-amid

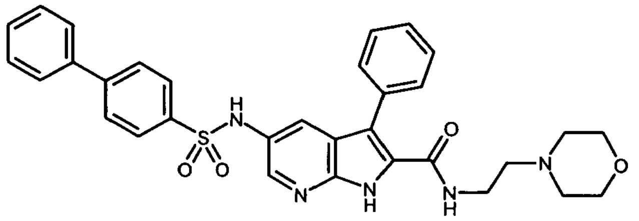

In Analogie zu Beispiel 1) erhält man aus 50 mg der in Beispiel 17b) hergestellten Säure und 13.9 mg 2-(Morpholin-4-yl)-ethylamin 15 mg der Titelverbindung.
NMR (300 MHz, DMSO-d6): δ = 2.27 (4H), 2.32 (2H), 3.30 (2H), 3.45 (4H), 7.27-7.39 (5H), 7.42 (1 H), 7.48 (2H), 7.52 (2H), 7.71 (2H), 7.76 (2H), 7.87 (2H), 8.10 (1 H), 10.09 (1 H), 12.33 (1 H).

Das Ausgangsmaterial für die obige Titelverbindung wird wie folgt hergestellt:

17a) 5-(Biphenyl-4-sulfonylamino)-3-phenyl-1H-pyrrolo[2,3-b]pyridin-2-carbonsäure ethylester

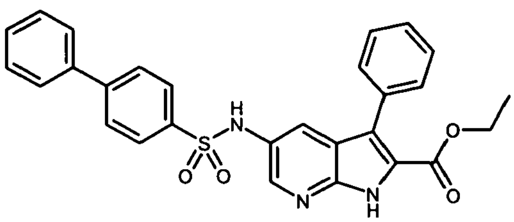

In Analogie zu Beispiel 1f) erhält man aus 200 mg des in Beispiel 4f) hergestellten Amins und 180 mg Biphenyl-4-sulfonsäurechlorid 210 mg der Titelverbindung.
NMR (300 MHz, DMSO-d6): δ = 1.10 (3H), 4.15 (2H), 7.22-7.32 (5H), 7.36 (1 H), 7.41 (1H), 7.47 (2H), 7.66 (2H), 7.69 (2H), 7.83 (2H), 8.16 (1H), 10.10 (1H), 12.58 (1 H).

17b) 5-(Biphenyl-4-sulfonylamino)-3-phenyl-1H-pyrrolo[2,3-b]pyridin-2-carbonsäure

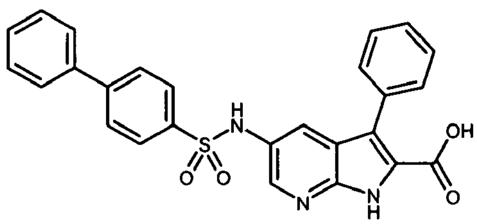

In Analogie zu Beispiel 1j) erhält man aus 196 mg des in Beispiel 17a) hergestellten Esters 184 mg der Titelverbindung.
NMR (300 MHz, DMSO-d6): δ = 7.27-7.36 (5H), 7.39 (1H), 7.41-7.56 (3H), 7.70 (2H), 7.73 (2H), 7.87 (2H), 8.18 (1 H), 10.10 (1 H), 12.45 (1 H), 13.08 (1 H).

**Beispiel 18** (1 S,2R)-5-(Biphenyl-4-sulfonylamino)-3-phenyl-1 H-pyrrolo[2,3-b]pyridin-2-carbonsäure (2-hydroxy-cyclopentyl)-amid

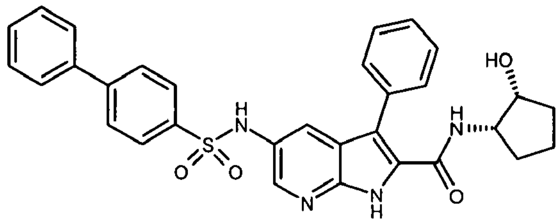

In Analogie zu Beispiel 1) erhält man aus 50 mg der in Beispiel 17b) hergestellten Säure und 14.7 mg (1S,2R)-(2-hydroxy-cyclopentyl)-amin 22 mg der Titelverbindung.
NMR (300 MHz, DMSO-d6): δ = 1.34-1.84 (6H), 3.91 (1 H), 3.99 (1 H), 4.56 (1 H), 7.16 (1 H), 7.25-7.38 (4H), 7.39 (1 H), 7.42-7.56 (4H), 7.71 (2H), 7.74 (2H), 7.87 (2H), 8.12 (1 H), 10.08 (1 H), 12.35 (1 H).

**Beispiel 19** 3-Phenyl-5-(4-trifluormethoxy-phenylsulfonylamino)-1H-pyrrolo[2,3-b]pyridin-2-carbonsäure (2-morpholin-4-yl-ethyl)-amid

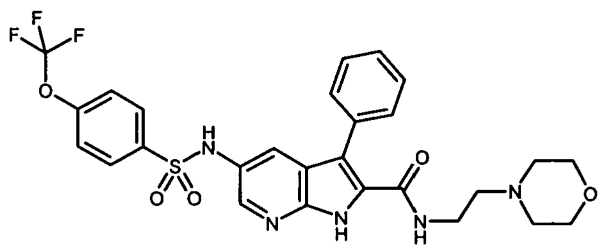

In Analogie zu Beispiel 1) erhält man aus 50 mg der in Beispiel 19b) hergestellten Säure und 13.6 mg 2-(Morpholin-4-yl)-ethylamin 20 mg der Titelverbindung.
NMR (300 MHz, DMSO-d6): δ = 2.28 (4H), 2.34 (2H), 3.31 (2H), 3.47 (4H), 7.30-7.61 (9H), 7.76 (2H), 8.05 (1 H), 10.18 (1 H), 12.37 (1 H).

Das Ausgangsmaterial für die obige Titelverbindung wird wie folgt hergestellt:

19a) 3-Phenyl-5-(4-trifluormethoxy-phenylsulfonylamino)-1H-pyrrolo[2,3-b]pyridin-2-carbonsäure ethylester

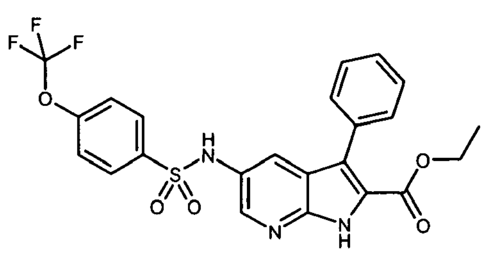

In Analogie zu Beispiel 1f) erhält man aus 200 mg des in Beispiel 4f) hergestellten Amins und 185 mg 4-Trifluormethoxyphenyl-sulfonsäurechlorid 232 mg der Titelverbindung.
NMR (300 MHz, DMSO-d6): δ = 1.11 (3H), 4.16 (2H), 7.29 (2H), 7.33-7.42 (4H), 7.52 (2H), 7.71 (2H), 8.11 (1 H), 10.18 (1 H), 12.60 (1 H).

19b) 3-Phenyl-5-(4-trifluormethoxy-phenylsulfonylamino)-1 H-pyrrolo[2,3-b]pyridin-2-carbonsäure

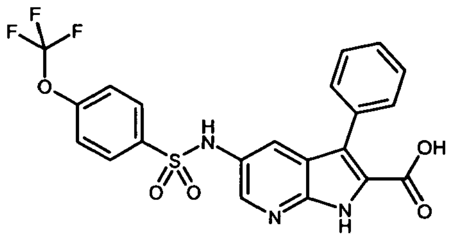

In Analogie zu Beispiel 1j) erhält man aus 216 mg des in Beispiel 19a) hergestellten Esters 202 mg der Titelverbindung.
NMR (300 MHz, DMSO-d6): δ = 7.28 (2H), 7.31-7.42 (4H), 7.52 (2H), 7.71 (2H), 8.08 (1 H), 10.15 (1 H), 12.44 (1 H), 13.09 (1 H).

**Beispiel 20** (1 S,2R)- 3-Phenyl-5-(4-trifluormethoxy-phenylsulfonylamino)-1 H-pyrrolo[2,3-b]pyridin-2-carbonsäure (2-hydroxy-cyclopentyl)-amid

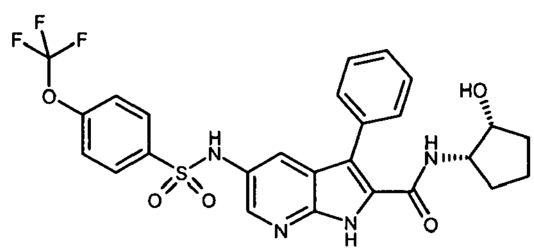

In Analogie zu Beispiel 1) erhält man aus 70 mg der in Beispiel 19b) hergestellten Säure und 20.2 mg (1S, 2R)-(2-hydroxy-cyclopentyl)-amin 15 mg der Titelverbindung.
NMR (300 MHz, DMSO-d6): δ = 1.35-1.85 (6H), 3.91 (1 H), 4.00 (1 H), 4.57 (1 H), 7.17 (1H), 7.25-7.48 (6H), 7.57 (2H), 7.75 (2H), 8.06 (1H), 10.16 (1H), 12.38 (1 H).

**Beispiel 21** 3-Phenyl-5-(4-trifluormethyl-phenylsulfonylamino)-1 H-pyrrolo[2,3-b]pyridin-2-carbonsäure (2-morpholin-4-yl-ethyl)-amid

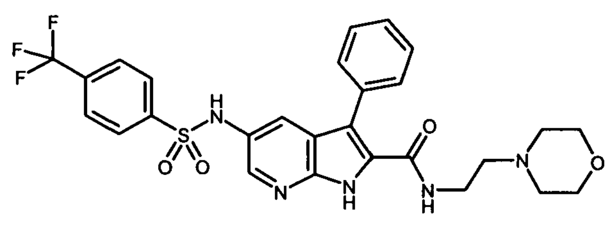

In Analogie zu Beispiel 1) erhält man aus 50 mg der in Beispiel 21b) hergestellten Säure und 14.1 mg 2-(Morpholin-4-yl)-ethylamin 22 mg der Titelverbindung.
NMR (300 MHz, DMSO-d6): δ = 2.24 (4H), 2.29 (2H), 3.25 (2H), 3.42 (4H), 7.27 (2H), 7.35 (2H), 7.40 (2H), 7.48 (1H), 7.80 (2H), 7.93 (2H), 8.00 (1H), 10.24 (1 H), 12.34 (1 H).

Das Ausgangsmaterial für die obige Titelverbindung wird wie folgt hergestellt:

21 a) 3-Phenyl-5-(4-trifluormethyl-phenylsulfonylamino)-1 H-pyrrolo[2,3-b]pyridin-2-carbonsäure ethylester

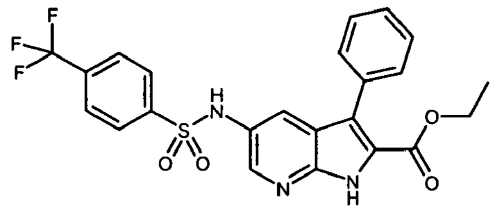

In Analogie zu Beispiel 1f) erhält man aus 200 mg des in Beispiel 4f) hergestellten Amins und 174 mg 4-Trifluormethylphenyl-sulfonsäurechlorid 234 mg der Titelverbindung.
NMR (300 MHz, DMSO-d6): $\delta$ = 1.11 (3H), 4.16(2H), 7.26 (2H), 7.31-7.42 (4H), 7.80 (2H), 7.92 (2H), 8.11 (1 H), 10.29 (1 H), 12.61 (1 H).

21 b) 3-Phenyl-5-(4-trifluormethyl-phenylsulfonylamino)-1 H-pyrrolo[2,3-b]pyridin-2-carbonsäure

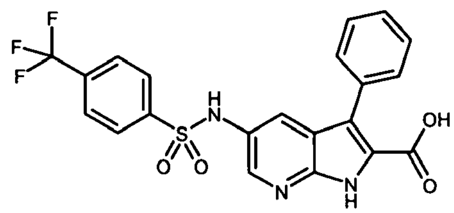

In Analogie zu Beispiel 1j) erhält man aus 222 mg des in Beispiel 21a) hergestellten Esters 205 mg der Titelverbindung.
NMR (300 MHz, DMSO-d6): $\delta$ = 7.26 (2H), 7.28-7.41 (4H), 7.79 (2H), 7.92 (2H), 8.08 (1 H), 10.26 (1 H), 12.45 (1 H), 13.09 (1 H).

**Beispiel 22** (1S,2R)-3-Phenyl-5-(4-trifluormethyl-phenylsulfonylamino)-1H-pyrrolo[2,3-b]pyridin-2-carbonsäure (2-hydroxy-cyclopentyl)-amid

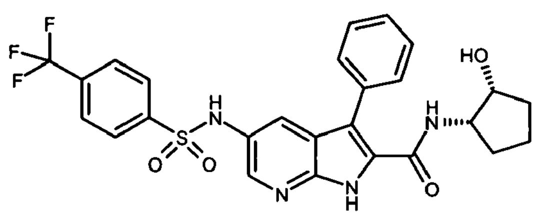

In Analogie zu Beispiel 1) erhält man aus 60 mg der in Beispiel 21b) hergestellten Säure und 19.9 mg (1S, 2R)-(2-hydroxy-cyclopentyl)-amin 24 mg der Titelverbindung.
NMR (300 MHz, DMSO-d6): $\delta$ = 1.32-1.83 (6H), 3.86 (1 H), 3.95 (1 H), 4.54 (1 H), 7.12 (1 H), 7.19-7.44 (6H), 7.79 (2H), 7.92 (2H), 8.02 (1 H), 10.23 (1 H), 12.36 (1 H).

**Beispiel 23** (1 S,2R)-5-(Biphenyl-4-sulfonylamino)-3-phenyl-1 H-pyrrolo[2,3-b]pyridin-2-carbonsäureamid

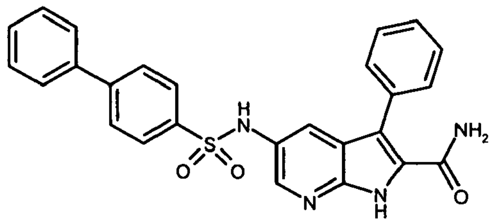

In Analogie zu Beispiel 15) erhält man aus 63 mg der in Beispiel 17b) hergestellten Säure 23 mg der Titelverbindung.
NMR (300 MHz, DMSO-d6): δ = 7.18 (1 H), 7.25-7.38 (5H), 7.39-7.55 (4H), 7.62 (1 H), 7.71 (2H), 7.73 (2H), 7.87 (2H), 8.11 (1 H), 10.08 (1 H), 12.24 (1 H).

**Biologische Beispiele:**

**Beispiel 1: sAC-Assay**

In einem geeigneten Puffersystem katalysiert die lösliche, spermienspezifische Adenylatzyklase die Umsetzung von Adenosintriphosphat (ATP) zu zyklischem Adenosinmonophosphat (cAMP) und Pyrophosphat. Auf diese Weise generiertes, freies cAMP wird anschließend in einem kompetitiven Nachweisverfahren eingesetzt, bei dem die Bindung eines mit Europiumkryptate (Eu[K]) markierten anti-cAMP Antikörpers (anti cAMP-Eu[K]-AK) an ein mit cAMP-Molekülen markiertes, modifiziertes Allophycocyanin-1 Molekül (cAMP-XL665) verhindert wird. In Abwesenheit von exogenem cAMP kommt es nach Anregung bei 335 nm zu einem Fluoreszenz Resonanz Energie Transfer (FRET) zwischen dem anti cAMP-Eu[K]-AK (FRET-Donor) und dem cAMP-XL665 Molekül (FRET-Akzeptor). Dieser Prozess wird zeitlich versetzt (timeresolved) anhand der Emission des FRET-Akzeptors XL665 (665nm und 620nm) quantifiziert. Ein Signal-Abfall (gemessen als Well-Ratio; Berechnungsformel: [(E665nm/E620nm) X 10000]) lässt sich auf das Vorhandensein von cAMP und somit auf die Aktivität der sAC zurückführen. Pro Vertiefung einer 384-Loch Testplatte (Polystyrol; 384, NV) werden zunächst 1,5 μl der Testsubstanz (in 30 % DMSO) vorgelegt, bei den Lösemittelkontrollen lediglich 30 % DMSO. Anschließend werden 10 μl einer verdünnten sAC Enzymlösung ausgebracht (Enzym-Stocklösung in 300 mM NaCl, 10 % Glycerin; pH 7,6; Enzym-Zwischen- und Endverdünnung a) 1:10 und b) 1:2000 jeweils in: 1.0 mM $MnCl_2$; 0.2 % BSA; 50 mM Tris pH 7,5 in $H_2O$). Die Enzymreaktion wird durch Zugabe von 5 μl der ATP-Substrat-Lösung (200 μM ATP in $H_2O$) gestartet und nach einer Inkubation (25 Min. bei Raumtemperatur) durch die Zugabe von 5 μl der Stop-Lösung (200 μM EDTA in PBS) beendet. Zum Schluss wird die ganze Reaktion durch die Zugabe von 70 μl PBS auf ein Gesamtvolumen von 91,5 μl eingestellt.
Anschließend werden 8 μl der Detektionslösung 1 in eine Vertiefung der 384-Loch Mess-Platte vorgelegt (Messplatte: Polystyrol; 384, SV - black; Detektionslösung 1: 50 μl cAMP-XL665; 950 μl Rekonsititutionspuffer; 2200 μl PBS; cAMP-XL665: Herstellung durch Zugabe von 5 ml $H_2O$ zum lyophyliserten Produkt gemäß Vorschrift Cis bio Kit: #62AMPPEC; Lagerung: aliquotiert bei - 80 ˚ C). Anschließend werden 3 μl aus den 91,5 μl der entsprechenden Vertiefung der Testplatte zugegeben. Zum Schluss erfolgt die Zugabe von 8 μl der Detektionslösung 2 (Detektionslösung 2 : 50 μl anti cAMP-Eu[K]-AK; 950 μl Rekonsititutionspuffer; 2200 μl PBS; anti cAMP-Eu[K]-AK: Herstellung gemäß Vorschrift Cis bio Kit: #62AMPPEC; Lagerung: aliquotiert bei - 80 ˚ C).
Nach einer weiteren Inkubation von 90 Min. bei Raumtemperatur wird das HTRF-Ergebnis entweder am Packard Discovery oder mit dem RubiStar HTRF-Messgerät gemessen (Delay: 50 μs; Integration time: 400 μs).

**Beispiel 2. Isolierung von humanen Spermien aus Ejakulaten und Kapazitation**

2.1. Isolierung der Spermien

Humane Spermien werden aus dem Ejakulat durch ein zweischichtiges Gradientensystem auf Basis von colloidalen Silica-Partikeln gereinigt (Handelsname: Percoll oder ISolate).
Pro Ejakulat werden in einem 15 ml Zentrifugenröhrchen (konisch, Kunststoff) je 2,5 ml vorgewärmte untere Schicht ("90 % ISolate lower layer", Fa. Irvine) vorgelegt und vorsichtig mit 2,5 ml vorgewärmter oberer Schicht ("50 % ISolate upper layer", Fa. Irvine) überschichtet und im Wasserbad bei 37 ˚ C für < 1 h vorgehalten. Der Gradient wird vorsichtig mit maximal 3 ml normalen (in Bezug auf Spermienanzahl, Motilität und Verflüssigung) Ejakulates überschichtet. Die Sedimentation der Spermien erfolgt bei 1000 x *g* für 25 Min bei Raumtemperatur. Mittels einer Glaskapillare werden beide Schichten bis kurz oberhalb der Spermienpellets abgesaugt. Zum Auswaschen des ISolate-Gradienten werden

die in je ca. 200 µl resuspendierten Spermienpellets in ein 15 ml Kunststoffröhrchen mit 12 ml mHTF Medium (4 mM $NaHCO_3$; 0,01 % BSA; 37 ° C) überführt und die Spermien werden bei 1000 x *g* für 20 Min sedimentiert. Das Medium wird bis kurz über dem Pellet abgesaugt und mit mHTF Medium Medium (4 mM $NaHCO_3$; 0,01 % BSA; 37 ° C) auf 1000 µl eingestellt. Die Anzahl der Spermien wird in einer Neubauer-Zählkammer bestimmt und für die folgende Kapazitation gegebenenfalls mit mHTF-Medium (4 mM $NaHCO_3$; 0,01 % BSA; 37 ° C) auf 4 x 106 Spermien/150 µl eingestellt.

2.2. Kapazitation

Falls der Einfluss von Testsubstanzen auf die akrosomale Reaktion getestet werden soll, müssen die Spermien mit den Testsubstanzen vorinkubiert werden. Diese Vorinkubation (15 min im Wärmeschrank bei 37 ° C) ist notwendig, um das Eindringen der Testsubstanzen in die Spermien vor Beginn der Kapazitation zu ermöglichen, d.h. eine Präsättigung der Bindungsstellen im Spermium zu erreichen, insbesondere bei Substanzen, die schlecht durch die Membran gehen. Sie ist außerdem notwendig, da die Erhöhung der BSA-Konzentration bei der Kapazitation durch die hohe Lipidbindung des BSA zur Abnahme der effektiven Testsubstanzkonzentration im Ansatz führen könnte.
Die Testsubstanzen werden in DMSO gelöst und mit mHTF-Medium (4 mM $NaHCO_3$; 0,01 % BSA; 37 ° C) verdünnt, so dass im finalen Kapazitationsansatz von 400 µl die DMSO-Konzentration 0.5 % beträgt. Je 150 µl der temperierten obigen Testsubstanzlösung werden zu jeweils 150 µl Spermiensuspension pipettiert und für 15 min bei 37 ° C vorinkubiert. Die Kapazitation der Spermien wird gestartet durch Zugabe von 100 µl mHTF-Medium (88 mM $NaHCO_3$; 4 % BSA; 37 ° C). In dem finalen 400 µl Kapazitationsansatz beträgt die Spermienkonzentration 10 x 106/ml, die Bicarbonatkonzentration 4 mM und die BSA-Konzentration 1 %. Die Kapazitation erfolgt bei 37 ° C für 3 Stunden im Wärmeschrank.
Zum Beenden der Kapazitation werden die Ansätze (je 400 µl) komplett in jeweils ein 15 ml Probenröhren mit 1,5 ml mHTF (4 mM $NaHCO_3$; 37 ° C) überführt, 5 min bei 1000 x *g* zentrifugiert und der Überstand abgenommen. Mit diesem Schritt werden sowohl die hohe Proteinmenge als auch die TestSubstanzen entfernt.

**Beispiel 3. Flow cytometrische Bestimmung der akrosomalen Reaktion**

3.1. Einleitung der akrosomalen Reaktion durch Ionophorbehandlung und gleichzeitige CD46-FITC-Färbung

Die akrosomale Reaktion (AR) des Spermiums wird durch die Bindung des Spermiums an die Zona pellucida (ZP) ausgelöst. Dabei werden aus dem Akrosom Enzyme freigesetzt, die es dem Spermium ermöglichen, durch die ZP bis zur Eizelle vorzudringen. Bei der AR kommt es beim Spermium zu einer teilweisen Verschmelzung der Plasmamembran mit der äußeren akrosomalen Membran (OAM). Der Spermienkopf wird am Ende nur noch durch die innere akrosomale Membran (IAM) begrenzt. Nur an der IAM ist das CD46-Antigen nachweisbar.
*In vitro* lässt sich mit einer geeigneten Konzentration des Calcium-Ionophors A23187 an kapazitierten, aber nicht an unkapazitierten bzw. an durch Testsubstanzen an der Kapazitation gehemmten Spermien die akrosomale Reaktion induzieren. Mit Hilfe des FITC markierten Anti-CD46 Antikörpers (Fa. Pharmingen) gegen die IAM können die Akrosomreagierten Spermien von den Akrosom-intakten Spermien, bei denen die IAM nicht exponiert ist, im Flow Cytometer unterschieden werden. Durch die gleichzeitige Färbung der Spermien mit dem DNA-Farbstoff Ethidium Homodimer (EhD), der nur die DNA membran-defekter, also toter Zellen färbt, können die toten von den lebenden Spermien unterschieden werden.
Weil die Ionophorverdünnungen zum Auslösen der AR sehr instabil zu sein scheinen und für die gleichzeitige Färbung mit der CD46-FITC Lösung gemischt werden müssen, können die Lösungen nicht vor Versuchsbeginn angesetzt werden, sondern müssen während der Aufarbeitung der Kapazitationsansätze hergestellt werden.
Die Spermienpellets werden im Restüberstand resuspendiert und im Wasserbad (37 ° C) mit 450 µl mHTF (4 mM $NaHCO_3$; 0,01 % BSA; 37 ° C) verdünnt. 100 µl Aliquots der Spermiensuspensionen werden in vorbereitete Proben-FACS-Flow-Röhrchen pipettiert (im Wasserbad). Zu den Spermien werden 150 µl einer Lösung mit Ionophor und FITC-markiertem Anti-CD46 Antikörper pipettiert. Die Endkonzentration beträgt 800 nM Ionophor und eine 1 : 125 Verdünnung des Anti-CD46-Antikörpers in mHTF (4 mM $NaHCO_3$; 0,01 % BSA; 37 ° C). Die Spermien werden darin für 30 min lichtgeschützt im Wasserbad bei 37 ° C inkubiert.
Die Inkubation wird durch Zugabe von 3,5 ml PBS [0,1 % BSA] / Ansatz gestoppt, gefolgt von einer Zentrifugation für 5 min bei 700 x *g* (Raumtemperatur) und anschließendem Absaugen der Überstände. Nach der Zentrifugation werden die Proben bis zur Messung auf der Wärmeplatte warmgehalten.

3.2. EhD-Färbung (zur Differenzierung der toten/lebenden akrosomal reagierten Spermien)

Die Spermienpellets werden nach dem Absaugen mit je 500 µl frisch angesetzter EhD-Lösung (150 nM EhD in PBS [w/o BSA]; 37 ° C) versetzt. Die Proben können anschließend am Flow Cytometer (BD Facs Calibur) vermessen werden. Die Messung erfolgt bei einer Anregungswellenlänge des Lasers von 488 nm, es werden 10000 Spermien pro

Messung erfasst. Akrosomreagierte Spermien werden über CD46-FITC im Filter FL-1 bei 530 nm gemessen. Tote Spermien werden mittels der EhD - DNA-Färbung im Filter FL-2 bei 634 nm gemessen. Die Messkanäle werden zuvor entsprechend gegeneinander kompensiert.

3.3 Auswertung

Die Spermien werden als sehr einheitliche Zellpopulation in einem FSC-H (forward scatter) gegen SSC-H (sideward scatter) Dotblot ausgewählt. Da eine Zweifarbenfluoreszenzfärbung genutzt wird, erfolgt die Auswertung mit Hilfe der Quadrantenanalyse in einem FL-1 (EhD, X-Achse) vs. FL-2 (FITC-CD46, Y-Achse) Dotblot mit der ausgewählten Spermienpopulation aus dem FSC vs SSC Dotblot:

| | Quadrant im FL-1 vs. FL-2 Dotblot | Färbung | Analyse |
|---|---|---|---|
| Q1 = UL | upper left | nur EhD | tote, nicht akrosom reagierte Spermien |
| Q2 = UR | upper right | EhD und FITC-CD46 | tote, akrosom reagierte Spermien |
| Q3 = LL | lower left | ungefärbt | lebende, nicht akrosom reagierte Spermien |
| Q4 = LR | lower right | nur FITC-CD46 | lebende, akrosom reagierte Spermien |

Zur Berechnung der % induziert akrosomal reagierter Spermien (= "IAR[%]") werden nur die lebenden Spermien aus Q3 und Q4 herangezogen und ihre Gesamtzahl gleich 100 % gesetzt. IAR berechnet sich dann wie folgt:

$$IAR[\%] = \frac{LR \times 100}{LL + LR}$$

Ein Teil der Spermien reagiert bereits ohne lonophorzugabe spontan akrosomal (= "SAR[%]"). Daher erfolgt immer auch eine Kontrollmessung gleichbehandelter Spermien ohne lonophorzugabe. Die SAR berechnet analog zur IAR. Die wirklich durch das lonophor ausgelöste akrosomale Reaktion ( = "ARIC[%]") berechnet sich als Differenz: ARIC = IAR - SAR.

Für die folgende Analyse des Einflusses unserer Inhibitoren auf die sAC vermittelte Kapazitation (gemessen als Fähigkeit des Spermiums zur lonophorinduzierten akrosomalen Reaktion) wird der Prozentsatz akrosomal reagierter Spermien in der positiven Kapazitationskontrolle (= Inkubation mit mHTF-Medium mit 25 mM NaHC03; 1 % BSA ohne Prüfsubstanzen) = 100% gesetzt. Die Fähigkeit der mit den Prüfsubstanzen versetzten Spermien zur akrosomalen Reaktion wird relativ zu dieser maximalen akrosomalen Reaktion angegeben.

Verwendete Materialien

mHTF = modif. Human tubular fluid (Fa. Irvine Scientific), Dulbeccos's Phosphate-Buffered-Saline (Fa. Gibco) (mit $Ca^{2+}$, $Mg^{2+}$, 1 g/L D-Glucose, 36 mg/L Na-Pyruvate, w/o Phenolrot, w/o $NaHCO_3$); Rinderserumalbumin, Fraktion V (Fa. Fluka); Dimethylsulfoxid (DMSO), wasserfrei (Fa. Merck);

Sodium Bicarbonate 7,5%ige Lsg.(893mM) (Fa. Irvine Scientific); Isolate-Gradient (Fa. Irvine Scientific); lonophor-A23187 free acid, (Fa. Calbiochem); Ethidium Homodimer (EhD) (Fa. Molecular Probe), Mouse Anti Human CD46:FITC (Fa. Pharmingen).

Literaturzitat:

J. W. Carver-Ward, Human Reproduction Vol. 11, No. 9, pp:1923 ff, 1996 High fertilization prediction by flow cytometric analysis of the CD46 antigen on the inner acrosomal membrane of spermatozoa

O. J. D'Cruz, G. G. Haas, Fertility and Sterility Vol. 65, No. 4, pp: 843 ff, 1996 Fluorescence-labeled fucolectins are superior markers for flow cytometric quantitation of the sperm acrosome reaction

E. Nieschlag, H.M. Behre, Andrologie, Springer Verlag 1996

Beispiele:

| Beispiel | Strukturformel | IC50 (nM) |
|---|---|---|
| 10 | 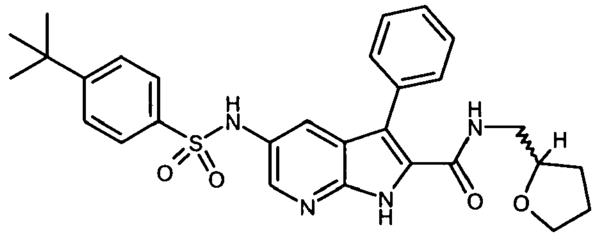 | 64 |
| 22 | 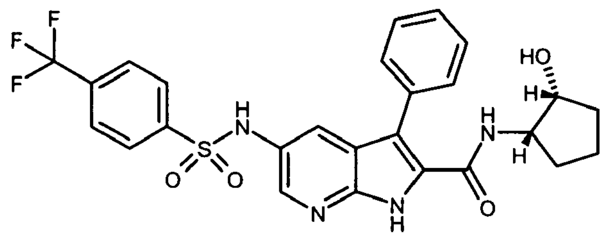 | 25 |
| 8 | 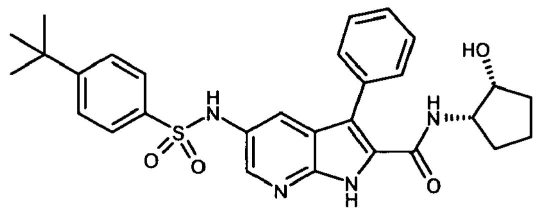 | 13 |
| 6 | 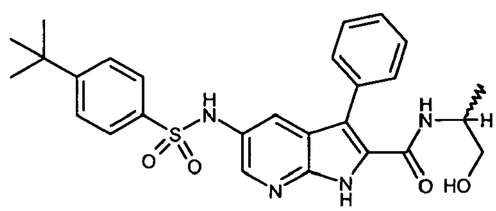 | 37 |
| Beispiel 27 aus WO 06/032541 | 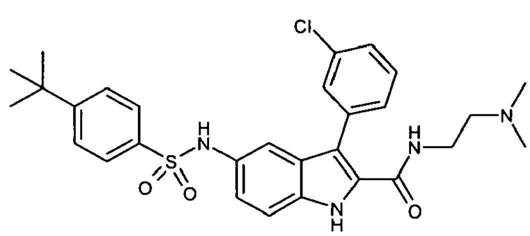 | 630 |
| Beispiel 43 aus WO 06/032541 | 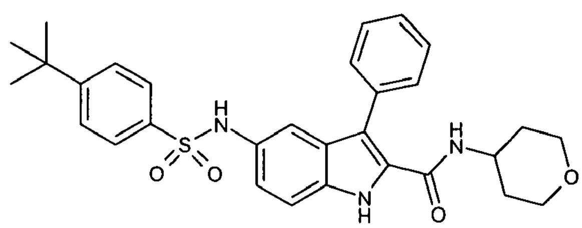 | 86 |

Aus der Tabelle ist zu erkennen, dass die erfindungsgemäßen Verbindungen in Bezug auf die Inhibition der löslichen Adenylatzyklase, ausgedrückt durch den $IC_{50}$-Wert, eine höhere Aktivität aufweisen als die bereits bekannten Catecholöstrogene (IC50 = 11 μM) und auch als die aus der Patentanmeldung WO 06/032541 bekannten Verbindungen. Die aus der Literatur bekannten Catecholöstrogene (OH-Östradiole) sind zudem toxisch, daher sind die erfindungsgemäßen Verbindungen den bekannten Verbindungen überlegen. Die erfindungsgemäßen Verbindungen sind auch potenter als die von Zippin vorgestellten Verbindungen.

## Patentansprüche

1. Verbindungen der allgemeinen Formel (I),

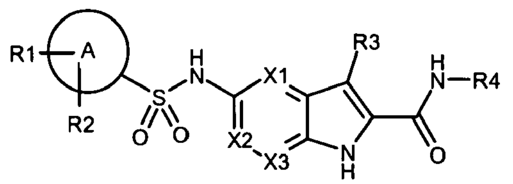

(I)

wobei

A ein $C_6$-$C_{12}$-Aryl- oder $C_5$-$C_{12}$-Heteroarylrest, der gegebenenfalls ein- oder mehrfach mit $R^1$ und/oder $R^2$ substituiert sein kann,
$R^1$ ein Wasserstoff, Halogen, Amino, eine -$S(O)_p$-$C_1$-$C_6$-Alkylgruppe, wobei p für 0 - 2 steht, ein $SO_2NH_2$, eine -$SO_2NH$-$C_1$-$C_6$-Alkylgruppe, eine -NH-$SO_2$-$C_1$- $C_6$-Alkylgruppe, ein $C_1$-$C_6$-Acyl-, NH-CO-$NH_2$, -O-CO-NH ($C_1$-$C_6$- Alkyl), -O-CO-N($C_1$-$C_6$-Alkyl)$_2$, NH-CO-$C_1$-$C_6$-Alkylrest, Hydroxy, Cyano, O-CO-($C_1$-$C_6$-Alkyl), $CO_2$-($C_1$-$C_6$-Alkyl), CO-NH-($C_1$-$C_6$- Alkyl), CO-N($C_1$-$C_6$-Alkyl)$_2$, CO-NH($C_5$-$C_{12}$-Heteroaryl), NH-($C_1$-$C_6$- Alkyl), N-($C_1$-$C_6$-Alkyl)$_2$, oder ein $C_1$-$C_6$-Alkyl, welches gegebenenfalls ein- oder mehrfach, gleich oder verschieden substituiert sein kann mit COOH, CO-$NH_2$, CO- NH-($C_1$-$C_6$-Alkyl), CO-N($C_1$-$C_6$-Alkyl)$_2$, $C_1$-$C_6$-Acyl, $C_1$-$C_6$-Alkoxy, Hydroxy, Cyano, $CO_2$-($C_1$-$C_6$-Alkyl), N-($C_1$-$C_6$- Alkyl)$_2$, $C_5$-$C_{12}$-Heteroaryl, welches gegebenenfalls mehrfach substituiert ist, $C_6$-$C_{12}$-Aryl, welches gegebenenfalls mehrfach substituiert ist, oder ein $C_1$-$C_6$-Alkoxy, welches gegebenenfalls ein- oder mehrfach, gleich oder verschieden substituiert sein kann mit Hydroxy, Cyano, $CO_2$-($C_1$-$C_6$-Alkyl), N-($C_1$-$C_6$-Alkyl)$_2$, NH-$C_3$-$C_6$-Cycloalkyl, COOH, CO-$NH_2$, CO-NH($C_1$-$C_6$-Alkyl) oder mit CO-N($C_1$-$C_6$- Alkyl)$_2$, ein O-$C_6$-$C_{12}$-Aryl, welches gegebenenfalls ein- oder mehrfach, gleich oder verschieden substituiert sein kann mit Halogen, Hydroxy, Cyano, COOH, CO-$NH_2$, $C_1$-$C_6$-Alkyl, $C_3$-$C_6$-Cycloalkyl, $C_1$-$C_6$-Acyl oder $C_1$-$C_6$-Alkoxy, oder ein O-$C_5$-$C_{12}$-Heteroaryl, welches gegebenenfalls ein- oder mehrfach, gleich oder verschieden mit Halogen, Hydroxy, Cyano, COOH, CO- $NH_2$, $C_1$-$C_6$-Alkyl, $C_3$-$C_6$-Cycloalkyl, $C_1$-$C_6$-Acyl oder $C_1$-$C_6$- Alkoxy substituiert sein kann, oder ein $C_6$-$C_{12}$-Aryl, welches gegebenenfalls ein- oder mehrfach, gleich oder verschieden substituiert sein kann mit Halogen, $C_1$-$C_6$-Alkyl, $C_3$-$C_6$-Cycloalkyl, $C_1$-$C_6$-Acyl, $C_1$-$C_6$-Alkoxy, $C_6$-$C_{12}$-Aryl, $C_5$-$C_{12}$-Heteroaryl, Hydroxy, $CH_2$-OH, Cyano, $CH_2$-CN, Amino, $CO_2$-($C_1$-$C_6$-Alkyl), N-($C_1$-$C_6$-Alkyl)$_2$, $NHSO_2CH_3$, $SO_2NH_2$, $SO_2NH$($C_1$-$C_6$-Alkyl), $SO2_N$($C_1$-$C_6$-Alkyl)$_2$, COOH, CO-$NH_2$, CO-NH-($C_1$-$C_6$-Alkyl), CO-N($C_1$-$C_6$-Alkyl)$_2$, CO-NH($C_5$-$C_{12}$- Heteroaryl), NH-CO($C_1$-$C_6$-Alkyl), $CH_2$-NH-CO($C_1$-$C_6$-Alkyl), NH-CO ($C_5$-$C_{12}$-Heteroaryl) oder ein -$S(O)_r$-$CH_3$, wobei r für 0 - 2 steht, oder zwei benachbarte Positionen durch -O-$CH_2$-O- oder -O-C($CH_3$)$_2$- O- substituiert sein können, oder ein $C_5$-$C_{16}$-Heteroaryl, welches gegebenenfalls ein- oder mehrfach, gleich oder verschieden substituiert sein kann mit Halogen, $C_1$-$C_6$-Alkyl, $C_3$-$C_6$-Cycloalkyl, $C_1$-$C_6$-Acyl, $C_1$-$C_6$-Alkoxy, $C_6$-$C_{12}$-Aryl, $C_5$- $C_{12}$-Heteroaryl, Hydroxy, $CH_2$-OH, Cyano, $CH_2$-CN, Amino, $CO_2$-($C_1$-$C_6$-Alkyl), N-($C_1$-$C_6$-Alkyl)$_2$, $NHSO_2CH_3$, $SO_2NH_2$, $SO_2NH$($C_1$-$C_6$-Alkyl), $SO_2N$($C_1$-$C_6$-Alkyl)$_2$, COOH, CO-$NH_2$, CO-NH-($C_1$-$C_6$-Alkyl), CO-N($C_1$-$C_6$-Alkyl)$_2$, CO-NH($C_5$-$C_{12}$- Heteroaryl), NH-CO($C_1$-$C_6$-Alkyl), $CH_2$-NH-CO($C_1$-$C_6$-Alkyl), NH-CO($C_5$-$C_{12}$-Heteroaryl) oder ein -$S(O)_r$-$CH_3$, wobei r für 0 - 2 steht, oder ein $C_3$-$C_6$-Cycloalkyl, welches gegebenenfalls ein- oder mehrfach, gleich oder verschieden mit Halogen, $C_1$-$C_6$-Alkyl, Hydroxy, Cyano, $CO_2$-($C_1$-$C_6$-Alkyl), $C_1$-$C_6$-Acyl, N-($C_1$-$C_6$-Alkyl)$_2$, COOH, CO- $NH_2$, CO-NH($C_1$-$C_6$-Alkyl), CO-N($C_1$-$C_6$-Alkyl)$_2$ oder $C_1$-$C_6$- Alkoxy substituiert sein kann,
$R^2$ ein Wasserstoff, Halogen, Amino, eine -$S(O)_p$-$C_1$-$C_6$-Alkylgruppe, wobei p für 0 - 2 steht, ein $SO_2NH_2$, -$SO_2NH$-$C_1$-$C_6$-Alkylgruppe, -NH-$SO_2$-$C_1$-$C_6$- Alkylgruppe, $C_1$-$C_6$-Acyl-, NH-CO-$NH_2$, -O-CO-NH($C_1$-$C_6$-Alkyl), -O- CO-N($C_1$-$C_6$-Alkyl)$_2$ oder NH-CO-$C_1$-$C_6$-Alkylrest, Hydroxy, Cyano, O-CO-($C_1$-$C_6$-Alkyl), $CO_2$-($C_1$-$C_6$-Alkyl), CO-NH-($C_1$-$C_6$-Alkyl), CO- N($C_1$-$C_6$-Alkyl)$_2$, CO-NH($C_5$-$C_{12}$-Heteroaryl), NH-($C_1$-$C_6$-Alkyl), N- ($C_1$-$C_6$-Al-

kyl)$_2$, oder ein $C_1$-$C_6$-Alkyl, welches gegebenenfalls ein- oder mehrfach, gleich oder verschieden substituiert sein kann mit COOH, CO-$NH_2$, CO- NH($C_1$-$C_6$-Alkyl) oder mit CO-N($C_1$-$C_6$-Alkyl)$_2$, $C_1$-$C_6$-Acyl, $C_1$-$C_6$-Alkoxy, Hydroxy, Cyano, $CO_2$-($C_1$-$C_6$-Alkyl), N-($C_1$-$C_6$- Alkyl)$_2$, $C_5$-$C_{12}$-Heteroaryl, welches gegebenenfalls mehrfach substituiert ist, $C_6$-$C_{12}$-Aryl, welches gegebenenfalls mehrfach substituiert ist, oder ein $C_1$-$C_6$-Alkoxy, welches gegebenenfalls ein- oder mehrfach, gleich oder verschieden substituiert sein kann mit Hydroxy, Cyano, $CO_2$-($C_1$-$C_6$-Alkyl), N-($C_1$-$C_6$-Alkyl)$_2$, NH-$C_3$-$C_6$-Cycloalkyl, COOH, CO-$NH_2$, CO-NH($C_1$-$C_6$-Alkyl) oder mit CO-N($C_1$-$C_6$- Alkyl)$_2$, ein O-$C_6$-$C_{12}$-Aryl, welches gegebenenfalls ein- oder mehrfach, gleich oder verschieden mit Halogen, Hydroxy, Cyano, COOH, CO- $NH_2$, $C_1$-$C_6$-Alkyl, $C_3$-$C_6$-Cycloalkyl, $C_1$-$C_6$-Acyl oder $C_1$-$C_6$- Alkoxy substituiert sein kann, oder ein O-$C_5$-$C_{12}$-Heteroaryl, welches gegebenenfalls ein- oder mehrfach, gleich oder verschieden mit Halogen, Hydroxy, Cyano, COOH, CO- $NH_2$, $C_1$-$C_6$-Alkyl, $C_3$-$C_6$-Cycloalkyl, $C_1$-$C_6$-Acyl oder $C_1$-$C_6$- Alkoxy substituiert sein kann, oder ein $C_6$-$C_{12}$-Aryl, welches gegebenenfalls ein- oder mehrfach, gleich oder verschieden substituiert sein kann mit Halogen, $C_1$-$C_6$-Alkyl, $C_3$-$C_6$-Cycloalkyl, $C_1$-$C_6$-Acyl, $C_1$-$C_6$-Alkoxy, $C_6$-$C_{12}$-Aryl, $C_5$- $C_{12}$-Heteroaryl, Hydroxy, $CH_2$-OH, Cyano, $CH_2$-CN, Amino, $CO_2$-($C_1$-$C_6$-Alkyl), N-($C_1$-$C_6$-Alkyl)$_2$, $NHSO_2CH_3$, $SO_2NH_2$, $SO_2NH$($C_1$-$C_6$-Alkyl), $SO_2N$($C_1$-$C_6$-Alkyl)$_2$, COOH, CO-$NH_2$, CO-NH($C_1$-$C_6$-Alkyl), CO-N($C_1$-$C_6$-Alkyl)$_2$, CO-NH($C_5$-$C_{12}$- Heteroaryl), NH-CO($C_1$-$C_6$-Alkyl), $CH_2$-NH-CO($C_1$-$C_6$-Alkyl), NH-CO ($C_5$-$C_{12}$-Heteroaryl) oder ein -S(O)$_r$-$CH_3$, wobei r für 0 - 2 steht, oder zwei benachbarte Positionen durch -O-$CH_2$-O- oder -O-C($CH_3$)$_2$- O- substituiert sein können, oder ein $C_5$-$C_{16}$-Heteroaryl, welches gegebenenfalls ein- oder mehrfach, gleich oder verschieden substituiert sein kann mit Halogen, $C_1$-$C_6$-Alkyl, $C_3$-$C_6$-Cycloalkyl, $C_1$-$C_6$-Acyl, $C_1$-$C_6$-Alkoxy, $C_6$-$C_{12}$-Aryl, $C_5$- $C_{12}$-Heteroaryl, Hydroxy, $CH_2$-OH, Cyano, $CH_2$-CN, Amino, $CO_2$-($C_1$-$C_6$-Alkyl), N-($C_1$-$C_6$-Alkyl)$_2$, $NHSO_2CH_3$, $SO_2NH_2$, $SO_2NH$($C_1$-$C_6$-Alkyl), $SO_2N$($C_1$-$C_6$-Alkyl)$_2$, COOH, CO-$NH_2$, CO-NH-($C_1$-$C_6$-Alkyl), CO-N($C_1$-$C_6$-Alkyl)$_2$, CO-NH($C_5$-$C_{12}$- Heteroaryl), NH-CO($C_1$-$C_6$-Alkyl), $CH_2$-NH-CO($C_1$-$C_6$-Alkyl), NH-CO($C_5$-$C_{12}$-Heteroaryl) oder ein -S(O)$_r$-$CH_3$, wobei r für 0 - 2 steht, oder ein $C_3$-$C_6$-Cycloalkyl, welches gegebenenfalls ein- oder mehrfach, gleich oder verschieden mit Halogen, $C_1$-$C_6$-Alkyl, Hydroxy, Cyano, $CO_2$- ($C_1$-$C_6$-Alkyl), $C_1$-$C_6$-Acyl, N-($C_1$-$C_6$-Alkyl)$_2$, COOH, CO-$NH_2$, CO-NH($C_1$-$C_6$-Alkyl), CO-N($C_1$-$C_6$-Alkyl)$_2$ oder $C_1$-$C_6$-Alkoxy substituiert sein kann,

$R^3$ ein $C_6$-$C_{12}$-Aryl, welches gegebenenfalls ein- oder mehrfach, gleich oder verschieden substituiert sein kann mit Halogen, $C_1$-$C_6$-Alkyl, $C_1$-$C_6$-Alkoxy, Hydroxy, $C_1$-$C_6$-Alkylen-OH, $C_1$-$C_6$-Alkylen- $NR^5R^6$, Cyano, $CO_2$-($C_1$-$C_6$-Alkyl), N-($C_1$-$C_6$-Alkyl)$_2$, CO-NH- ($C_1$-$C_6$-Alkyl), CO-NH-($C_1$-$C_6$-Alkylen-OH), CO-NH-($C_1$-$C_6$- Alkylen-$NR^5R^6$), CO-N($C_1$-$C_6$-Alkyl)$_2$ oder $C_1$-$C_6$-Acyl, welches gegebenenfalls ein- oder mehrfach substituiert ist, oder mit zwei benachbarten Positionen durch -O-$CH_2$-O-, -O- $CH_2$-$CH_2$-O-, oder -O-C($CH_3$)$_2$-O- substituiert sein kann, oder ein $C_5$-$C_{16}$-Heteroaryl, welches gegebenenfalls ein- oder mehrfach, gleich oder verschieden substituiert sein kann mit Halogen, $C_1$-$C_6$-Alkyl, $C_1$-$C_6$-Acyl, $C_1$-$C_6$-Alkoxy, Hydroxy, Cyano, $CO_2$-($C_1$-$C_6$- Alkyl), N-($C_1$-$C_6$-Alkyl)$_2$, CO-NH-($C_1$-$C_6$-Alkylen-OH), CO- NH-($C_1$-$C_6$-Alkylen-$NR^5R^6$), CO-NH($C_1$-$C_6$-Alkyl) oder CO-N($C_1$-$C_6$-Alkyl)$_2$ oder ein $C_3$-$C_6$-Cycloalkyl, welches gegebenenfalls ein- oder mehrfach, gleich oder verschieden mit Halogen, $CF_3$, Hydroxy, Cyano, $CO_2$-($C_1$-$C_6$-Alkyl), $C_1$-$C_6$-Alkyl, $C_1$-$C_6$-Acyl, N-($C_1$-$C_6$-Alkyl)$_2$, CO-NH-($C_1$-$C_6$-Alkylen-OH), CO-NH-($C_1$-$C_6$-Alkylen-$NR^5R^6$), CO-NH($C_1$-$C_6$-Alkyl) oder CO-N($C_1$-$C_6$-Alkyl)$_2$ oder $C_1$-$C_6$- Alkoxy substituiert sein kann,

$R^4$ ein Wasserstoff, ein $C_1$-$C_6$-Alkyl, welches gegebenenfalls ein- oder mehrfach, gleich oder verschieden substituiert sein kann mit einer -S(O)$_p$-$C_1$-$C_6$- Alkylgruppe oder -S(O)$_p$-$C_3$-$C_6$-Cycloalkylgruppe, wobei p für 0 - 2 steht, Hydroxy, Cyano, COOH, CO-$NH_2$, $SO_2NH_2$, $SO_2NH$-($C_1$-$C_6$-Alkyl), $C_3$-$C_6$-Cycloalkyl, $C_1$-$C_6$-Acyl, $C_1$-$C_6$- Alkoxy, $CO_2$-($C_1$-$C_6$-Alkyl), N-($C_1$-$C_6$-Alkyl)$_2$, NH-($C_1$-$C_6$- Alkyl), NH-($C_3$-$C_6$-Cycloalkyl), CO-NH ($C_1$-$C_6$-Alkyl), CO- N($C_1$-$C_6$-Alkyl)$_2$ oder $C_6$-$C_{12}$-Aryl, welches gegebenenfalls mehrfach substituiert ist, oder $C_5$-$C_{12}$-Heteroaryl, welches gegebenenfalls mehrfach substituiert ist, oder ein $C_3$-$C_6$-Cycloalkyl, welches gegebenenfalls ein- oder mehrfach, gleich oder verschieden substituiert sein kann mit Hydroxy, $CH_2$-OH, Cyano, COOH, CO-$NH_2$, $SO_2NH_2$, $C_1$-$C_6$-Alkyl, $C_1$-$C_6$-Acyl oder $C_1$-$C_6$-Alkoxy, oder ein $C_6$-$C_{12}$-Aryl, welches gegebenenfalls ein- oder mehrfach, gleich oder verschieden mit Halogen, Hydroxy, $C_1$-$C_6$-Alkyl, $C_1$-$C_6$-Acyl, $C_1$-$C_6$-Alkoxy, N-($C_1$-$C_6$-Alkyl)$_2$, NH-($C_1$-$C_6$-Alkyl) oder Cyano substituiert sein kann, oder ein $C_5$-$C_{12}$-Heteroaryl, welches gegebenenfalls ein- oder mehrfach, gleich oder verschieden mit Halogen, Amino, Hydroxy, Cyano, COOH, $C_1$-$C_6$-Alkyl, $C_1$-$C_6$-Acyl, $C_1$-$C_6$-Alkoxy, N-($C_1$-$C_6$-Alkyl)$_2$ oder NH-($C_1$-$C_6$-Alkyl) substituiert sein kann,

$R^5$, $R^6$ unabhängig voneinander ein Wasserstoff oder ein $C_1$-$C_6$-Alkyl, und

$X^1$ ein Stickstoff, wenn $X^2$ und $X^3$ eine CH-Gruppe sind, oder

$X^2$ ein Stickstoff, wenn $X^1$ und $X^3$ eine CH-Gruppe sind, oder

$X^3$ ein Stickstoff, wenn $X^1$ und $X^2$ eine CH-Gruppe sind,

**2.** Verbindungen gemäß Anspruch 1, wobei

A ein Phenyl-, Naphthyl- oder $C_5$-$C_{12}$-Heteroarylrest, der gegebenenfalls ein- oder mehrfach mit $R^1$ und/oder

$R^2$ substituiert sein kann,

$R^1$ ein Wasserstoff, Halogen, Amino, eine $-S(O)_p-C_1-C_6$-Alkylgruppe, wobei p für 0 - 2 steht, ein $SO_2NH_2$, $-SO_2NH-C_1-C_6$-Alkylgruppe, $-NH-SO_2-C_1-C_6$- Alkylgruppe, $C_1-C_6$-Acyl-, $NH-CO-NH_2$, $-O-CO-NH(C_1-C_6$-Alkyl), -O- CO-N($C_1-C_6$-Alkyl)$_2$, NH-CO-$C_1-C_6$-Alkylrest, Hydroxy, Cyano, O- CO-($C_1-C_6$-Alkyl), $CO_2$-($C_1-C_6$-Alkyl), CO-NH-($C_1-C_6$-Alkyl), CO- N($C_1-C_6$-Alkyl)$_2$, CO-NH($C_5-C_{12}$-Heteroaryl), NH-($C_1-C_6$-Alkyl), N- ($C_1-C_6$-Alkyl)$_2$, oder ein $C_1-C_6$-Alkyl, welches gegebenenfalls ein- oder mehrfach, gleich oder verschieden substituiert sein kann mit COOH, $CO-NH_2$, CO- NH-($C_1-C_6$-Alkyl), CO-N($C_1-C_6$-Alkyl)$_2$, $C_1-C_6$-Acyl, $C_1-C_6$- Alkoxy, Hydroxy, Cyano, $CO_2$-($C_1-C_6$-Alkyl), N-($C_1-C_6$- Alkyl)$_2$, $C_5-C_{12}$-Heteroaryl, welches gegebenenfalls mehrfach substituiert ist, $C_6-C_{12}$-Aryl, welches gegebenenfalls mehrfach substituiert ist, oder ein $C_1-C_6$-Alkoxy, welches gegebenenfalls ein- oder mehrfach, gleich oder verschieden substituiert sein kann mit Hydroxy, Cyano, $CO_2$-($C_1-C_6$-Alkyl), N-($C_1-C_6$-Alkyl)$_2$, NH-$C_3-C_6$-Cycloalkyl, COOH, $CO-NH_2$, CO-NH($C_1-C_6$-Alkyl) oder mit CO-N($C_1-C_6$- Alkyl)$_2$, ein O-$C_6-C_{12}$-Aryl, welches gegebenenfalls ein- oder mehrfach, gleich oder verschieden mit Halogen, Hydroxy, Cyano, COOH, CO- $NH_2$, $C_1-C_6$-Alkyl, $C_3-C_6$-Cycloalkyl, $C_1-C_6$-Acyl oder $C_1-C_6$- Alkoxy substituiert sein kann, oder ein O-$C_5-C_{12}$-Heteroaryl, welches gegebenenfalls ein- oder mehrfach, gleich oder verschieden mit Halogen, Hydroxy, Cyano, COOH, CO- $NH_2$, $C_1-C_6$-Alkyl, $C_3-C_6$-Cycloalkyl, $C_1-C_6$-Acyl oder $C_1-C_6$- Alkoxy substituiert sein kann, oder ein $C_6-C_{12}$-Aryl, welches gegebenenfalls ein- oder mehrfach, gleich oder verschieden substituiert sein kann mit Halogen, $C_1-C_6$-Alkyl, $C_3-C_6$-Cycloalkyl, $C_1-C_6$-Acyl, $C_1-C_6$-Alkoxy, $C_6-C_{12}$-Aryl, $C_5-C_{12}$-Heteroaryl, Hydroxy, $CH_2$-OH, Cyano, $CH_2$-CN, Amino, $CO_2$-($C_1-C_6$-Alkyl), N-($C_1-C_6$-Alkyl)$_2$, $NHSO_2CH_3$, $SO_2NH_2$, $SO_2NH$($C_1-C_6$-Alkyl), $SO_2N$($C_1-C_6$-Alkyl)$_2$, COOH, $CO-NH_2$, CO-NH-($C_1-C_6$-Alkyl), CO-N($C_1-C_6$-Alkyl)$_2$, CO-NH($C_5-C_{12}$- Heteroaryl), NH-CO($C_1-C_6$-Alkyl), $CH_2$-NH-CO($C_1-C_6$-Alkyl), NH-CO($C_5-C_{12}$-Heteroaryl) oder ein $-S(O)_r-CH_3$, wobei r für 0 - 2 steht, oder zwei benachbarte Positionen durch -O-$CH_2$-O- oder -O-C($CH_3$)$_2$- O- substituiert sein können, oder ein $C_5-C_{16}$-Heteroaryl, welches gegebenenfalls ein- oder mehrfach, gleich oder verschieden substituiert sein kann mit Halogen, $C_1-C_6$-Alkyl, $C_3-C_6$-Cycloalkyl, $C_1-C_6$-Acyl, $C_1-C_6$-Alkoxy, $C_6-C_{12}$-Aryl, $C_5-C_{12}$-Heteroaryl, Hydroxy, $CH_2$-OH, Cyano, $CH_2$-CN, Amino, $CO_2$-($C_1-C_6$-Alkyl), N-($C_1-C_6$-Alkyl)$_2$, $NHSO_2CH_3$, $SO_2NH_2$, $SO_2NH$($C_1-C_6$-Alkyl), $SO_2N$($C_1-C_6$-Alkyl)$_2$, COOH, $CO-NH_2$, CO-NH-($C_1-C_6$-Alkyl), CO-N($C_1-C_6$-Alkyl)$_2$, CO-NH($C_5-C_{12}$- Heteroaryl), NH-CO($C_1-C_6$-Alkyl), $CH_2$-NH-CO($C_1-C_6$-Alkyl), NH-CO ($C_5-C_{12}$-Heteroaryl) oder ein $-S(O)_r-CH_3$, wobei r für 0 - 2 steht, oder ein $C_3-C_6$-Cycloalkyl, welches gegebenenfalls ein- oder mehrfach, gleich oder verschieden mit Halogen, $C_1-C_6$-Alkyl, Hydroxy, Cyano, $CO_2$-($C_1-C_6$-Alkyl), $C_1-C_6$-Acyl, N-($C_1-C_6$-Alkyl)$_2$, COOH, CO- $NH_2$, CO-NH($C_1-C_6$-Alkyl), CO-N($C_1-C_6$-Alkyl)$_2$ oder $C_1-C_6$-Alkoxy substituiert sein kann,

$R^2$ ein Wasserstoff, Halogen, Amino, eine $-S(O)_p-C_1-C_6$-Alkylgruppe, wobei p für 0 - 2 steht, ein $SO_2NH_2$, eine $-SO_2NH-C_1-C_6$-Alkylgruppe, eine $-NH-SO_2-C_1-C_6$-Alkylgruppe, $C_1-C_6$-Acyl-, $NH-CO-NH_2$, -O-CO-NH($C_1-C_6$-Alkyl), -O-CO-N($C_1-C_6$-Alkyl)$_2$ oder ein NH-CO-$C_1-C_6$-Alkylrest, Hydroxy, Cyano, O-CO-($C_1-C_6$-Alkyl), $CO_2$-($C_1-C_6$-Alkyl), CO-NH-($C_1-C_6$- Alkyl), CO-N($C_1-C_6$-Alkyl)$_2$, CO-NH($C_5-C_{12}$-Heteroaryl), NH-($C_1-C_6$- Alkyl), N-($C_1-C_6$-Alkyl)$_2$, oder ein $C_1-C_6$-Alkyl, welches gegebenenfalls ein- oder mehrfach, gleich oder verschieden substituiert sein kann mit COOH, $CO-NH_2$, CO- NH($C_1-C_6$-Alkyl) oder mit CO-N($C_1-C_6$-Alkyl)$_2$, $C_1-C_6$-Acyl, $C_1-C_6$-Alkoxy, Hydroxy, Cyano, $CO_2$-($C_1-C_6$-Alkyl), N-($C_1-C_6$- Alkyl)$_2$, $C_5-C_{12}$-Heteroaryl, welches gegebenenfalls mehrfach substituiert ist, $C_6-C_{12}$-Aryl, welches gegebenenfalls mehrfach substituiert ist, oder ein $C_1-C_6$-Alkoxy, welches gegebenenfalls ein- oder mehrfach, gleich oder verschieden substituiert sein kann mit Hydroxy, Cyano, $CO_2$-($C_1-C_6$-Alkyl), N-($C_1-C_6$-Alkyl)$_2$, NH-$C_3-C_6$-Cycloalkyl, COOH, $CO-NH_2$, CO-NH($C_1-C_6$-Alkyl) oder mit CO-N($C_1-C_6$- Alkyl)$_2$, ein O-$C_6-C_{12}$-Aryl, welches gegebenenfalls ein- oder mehrfach, gleich oder verschieden mit Halogen, Hydroxy, Cyano, COOH, CO- $NH_2$, $C_1-C_6$-Alkyl, $C_3-C_6$-Cycloalkyl, $C_1-C_6$-Acyl oder $C_1-C_6$-Alkoxy substituiert sein kann, oder ein O-$C_5-C_{12}$-Heteroaryl, welches gegebenenfalls ein- oder mehrfach, gleich oder verschieden mit Halogen, Hydroxy, Cyano, COOH, CO- $NH_2$, $C_1-C_6$-Alkyl, $C_3-C_6$-Cycloalkyl, $C_1-C_6$-Acyl oder $C_1-C_6$- Alkoxy substituiert sein kann, oder ein $C_6-C_{12}$-Aryl, welches gegebenenfalls ein- oder mehrfach, gleich oder verschieden substituiert sein kann mit Halogen, $C_1-C_6$-Alkyl, $C_3-C_6$-Cycloalkyl, $C_1-C_6$-Acyl, $C_1-C_6$-Alkoxy, $C_6-C_{12}$-Aryl, $C_5-C_{12}$-Heteroaryl, Hydroxy, $CH_2$-OH, Cyano, $CH_2$-CN, Amino, $CO_2$-($C_1-C_6$-Alkyl), N-($C_1-C_6$-Alkyl)$_2$, $NHSO_2CH_3$, $SO_2NH_2$, $SO_2NH$($C_1-C_6$-Alkyl), $SO_2N$($C_1-C_6$-Alkyl)$_2$, COOH, $CO-NH_2$, CO-NH($C_1-C_6$-Alkyl), CO-N($C_1-C_6$-Alkyl)$_2$, CO-NH($C_5-C_{12}$- Heteroaryl), NH-CO($C_1-C_6$-Alkyl), $CH_2$-NH-CO($C_1-C_6$-Alkyl), NH-CO ($C_5-C_{12}$-Heteroaryl) oder ein $-S(O)_r-CH_3$, wobei r für 0 - 2 steht, oder zwei benachbarte Positionen durch -O-$CH_2$-O- oder -O-C($CH_3$)$_2$- O- substituiert sein können, oder ein $C_5-C_{16}$-Heteroaryl, welches gegebenenfalls ein- oder mehrfach, gleich oder verschieden substituiert sein kann mit Halogen, $C_1-C_6$-Alkyl, $C_3-C_6$-Cycloalkyl, $C_1-C_6$-Acyl, $C_1-C_6$-Alkoxy, $C_6-C_{12}$-Aryl, $C_5$- $C_{12}$-Heteroaryl, Hydroxy, $CH_2$-OH, Cyano, $CH_2$-CN, Amino, $CO_2$-($C_1-C_6$-Alkyl), N-($C_1-C_6$-Alkyl)$_2$, $NHSO_2CH_3$, $SO_2NH_2$, $SO_2NH$($C_1-C_6$-Alkyl), $SO_2N$($C_1-C_6$-Alkyl)$_2$, COOH, $CO-NH_2$, CO-NH-($C_1-C_6$-Alkyl), CO-N($C_1-C_6$-Alkyl)$_2$, CO-NH($C_5-C_{12}$- Heteroaryl), NH-CO($C_1-C_6$-Alkyl), $CH_2$-NH-CO($C_1-C_6$-Alkyl), NH-CO($C_5-C_{12}$-Heteroaryl) oder ein $-S(O)_r-CH_3$, wobei r für 0 - 2 steht, oder ein $C_3-C_6$-Cycloalkyl, welches gegebenenfalls ein- oder mehrfach, gleich oder verschieden mit Halogen, $C_1-C_6$-Alkyl, Hydroxy, Cyano, $CO_2$-($C_1-C_6$-Alkyl), $C_1-C_6$-Acyl, N-($C_1-C_6$-Alkyl)$_2$, COOH, CO- $NH_2$, CO-NH($C_1-C_6$-Alkyl),

$CO-N(C_1-C_6-Alkyl)_2$ oder $C_1-C_6$- Alkoxy substituiert sein kann,

$R^3$ ein $C_6-C_{12}$-Aryl, welches gegebenenfalls ein- oder mehrfach, gleich oder verschieden substituiert sein kann mit Halogen, $C_1-C_6$-Alkyl, $C_1-C_6$-Alkoxy, Hydroxy, $C_1-C_6$-Alkylen-OH, $C_1-C_6$-Alkylen- $NR^5R^6$, Cyano, $CO_2-(C_1-C_6-Alkyl)$, $N-(C_1-C_6-Alkyl)_2$, CO-NH- $(C_1-C_6-Alkyl)$, CO-NH-$(C_1-C_6$-Alkylen-OH), CO-NH-$(C_1-C_6$- Alkylen-$NR^5R^6$), $CO-N(C_1-C_6-Alkyl)_2$ oder $C_1-C_6$-Acyl, welches gegebenenfalls ein- oder mehrfach substituiert ist, oder mit zwei benachbarten Positionen durch $-O-CH_2-O-$, -O- $CH_2-CH_2-O-$, oder $-O-C(CH_3)_2-O-$ substituiert sein kann, oder ein $C_5-C_{16}$-Heteroaryl, welches gegebenenfalls ein- oder mehrfach, gleich oder verschieden substituiert sein kann mit Halogen, $C_1-C_6$-Alkyl, $C_1-C_6$-Acyl, $C_1-C_6$-Alkoxy, Hydroxy, Cyano, $CO_2-(C_1-C_6$- Alkyl), $N-(C_1-C_6-Alkyl)_2$, CO-NH-$(C_1-C_6$-Alkylen-OH), CO-NH- $(C_1-C_6$-Alkylen-$NR^5R^6$), CO-NH$(C_1-C_6$-Alkyl) oder CO-N$(C_1$- $C_6$-Alkyl$)_2$ oder ein $C_3-C_6$-Cycloalkyl, welches gegebenenfalls ein- oder mehrfach, gleich oder verschieden mit Halogen, $CF_3$, Hydroxy, Cyano, $CO_2-(C_1$- $C_6$-Alkyl), $C_1-C_6$-Alkyl, $C_1-C_6$-Acyl, $N-(C_1-C_6-Alkyl)_2$, CO-NH- $(C_1-C_6$-Alkylen-OH), CO-NH-$(C_1-C_6$-Alkylen-$NR^5R^6$), CO- NH$(C_1-C_6$-Alkyl) oder $CO-N(C_1-C_6-Alkyl)_2$ oder $C_1-C_6$-Alkoxy substituiert sein kann,

$R^4$ ein Wasserstoff, ein $C_1-C_6$-Alkyl, welches gegebenenfalls ein- oder mehrfach, gleich oder verschieden substituiert sein kann mit einer $-S(O)_p-C_1-C_6$- Alkylgruppe oder einer $-S(O)_p-C_3-C_6$-Cycloalkylgruppe, wobei p für 0 - 2 steht, Hydroxy, Cyano, COOH, $CO-NH_2$, $SO_2NH_2$, $SO_2NH-(C_1-C_6-Alkyl)$, $C_3-C_6$-Cycloalkyl, $C_1-C_6$-Acyl, $C_1-C_6$-Alkoxy, $CO_2-(C_1-C_6-Alkyl)$, $N-(C_1-C_6-Alkyl)_2$, NH- $(C_1-C_6$-Alkyl), NH-$(C_3-C_6$-Cycloalkyl), CO-NH $(C_1-C_6$-Alkyl) oder mit $CO-N(C_1-C_6-Alkyl)_2$, $C_6-C_{12}$-Aryl, welches gegebenenfalls mehrfach substituiert ist, $C_5-C_{12}$-Heteroaryl, welches gegebenenfalls mehrfach substituiert ist, oder ein $C_3-C_6$-Cycloalkyl, welches gegebenenfalls ein- oder mehrfach, gleich oder verschieden mit Hydroxy, $CH_2$-OH, Cyano, COOH, $CO-NH_2$, $SO_2NH_2$, $C_1-C_6$-Alkyl, $C_1-C_6$-Acyl oder $C_1-C_6$-Alkoxy, oder ein $C_6-C_{12}$-Aryl, welches gegebenenfalls ein- oder mehrfach, gleich oder verschieden mit Halogen, Hydroxy, $C_1-C_6$-Alkyl, $C_1-C_6$-Acyl, $C_1-C_6$- Alkoxy, $N-(C_1-C_6-Alkyl)_2$, NH-$(C_1-C_6$-Alkyl) oder Cyano substituiert sein kann, oder ein $C_5-C_{12}$-Heteroaryl, welches gegebenenfalls ein- oder mehrfach, gleich oder verschieden mit Halogen, Amino, Hydroxy, Cyano, COOH, $C_1-C_6$-Alkyl, $C_1-C_6$-Acyl, $C_1-C_6$-Alkoxy, $N-(C_1-C_6-Alkyl)_2$ oder NH-$(C_1-C_6$-Alkyl) substituiert sein kann,

$R^5$, $R^6$ unabhängig voneinander Wasserstoff oder ein $C_1-C_6$-Alkyl, und

$X^1$ ein Stickstoff, wenn $X^2$ und $X^3$ eine CH-Gruppe sind, oder

$X^2$ ein Stickstoff, wenn $X^1$ und $X^3$ eine CH-Gruppe sind, oder

$X^3$ ein Stickstoff, wenn $X^1$ und $X^2$ eine CH-Gruppe sind,

bedeuten,
sowie deren Stereoisomere, Diastereomere, Enantiomere und Salze.

**3.** Verbindungen gemäß den Ansprüchen 1 - 2, wobei

A ein Phenyl oder ein monocyclischer $C_5-C_7$-Heteroarylrest, der gegebenenfalls ein- oder mehrfach mit $R^1$ und/oder $R^2$ substituiert sein kann,

$R^1$ ein Wasserstoff, Halogen, Amino, eine $-S(O)_p-C_1-C_6$-Alkylgruppe, wobei p für 0 - 2 steht, ein $SO_2NH_2$, eine $-SO_2NH-C_1-C_6$-Alkylgruppe, eine $-NH-SO_2-C_1-C_6$-Alkylgruppe, $C_1-C_6$-Acyl-, $NH-CO-NH_2$, -O-CO-NH$(C_1-C_6$-Alkyl), $-O-CO-N(C_1-C_6-Alkyl)_2$, NH-CO-$C_1-C_6$-Alkylrest, Hydroxy, Cyano, O-CO-$(C_1-C_6$-Alkyl), $CO_2-(C_1-C_6-Alkyl)$, CO-NH-$(C_1-C_6$-Alkyl), CO- $N(C_1-C_6-Alkyl)_2$, CO-NH$(C_5-C_{12}$-Heteroaryl), NH-$(C_1-C_6$-Alkyl), N- $(C_1-C_6-Alkyl)_2$, oder ein $C_1-C_6$-Alkyl, welches gegebenenfalls ein- oder mehrfach, gleich oder verschieden substituiert sein kann mit COOH, $CO-NH_2$, CO- NH-$(C_1-C_6$-Alkyl), $CO-N(C_1-C_6-Alkyl)_2$, $C_1-C_6$-Acyl, $C_1-C_6$- Alkoxy, Hydroxy, Cyano, $CO_2-(C_1-C_6-Alkyl)$, $N-(C_1-C_6$- Alkyl$)_2$, $C_5-C_{12}$-Heteroaryl, welches gegebenenfalls mehrfach substituiert ist, $C_6-C_{12}$-Aryl, welches gegebenenfalls mehrfach substituiert ist, oder ein $C_1-C_6$-Alkoxy, welches gegebenenfalls ein- oder mehrfach, gleich oder verschieden substituiert sein kann mit Hydroxy, Cyano, $CO_2-(C_1-C_6-Alkyl)$, $N-(C_1-C_6-Alkyl)_2$, NH-$C_3-C_6$-Cycloalkyl, COOH, $CO-NH_2$, CO-NH$(C_1-C_6$-Alkyl) oder mit CO-N$(C_1-C_6$- Alkyl$)_2$, ein O-$C_6-C_{12}$-Aryl, welches gegebenenfalls ein- oder mehrfach, gleich oder verschieden mit Halogen, Hydroxy, Cyano, COOH, CO- $NH_2$, $C_1-C_6$-Alkyl, $C_3-C_6$-Cycloalkyl, $C_1-C_6$-Acyl oder $C_1-C_6$- Alkoxy substituiert sein kann, oder ein O-$C_5-C_{12}$-Heteroaryl, welches gegebenenfalls ein- oder mehrfach, gleich oder verschieden mit Halogen, Hydroxy, Cyano, COOH, CO- $NH_2$, $C_1-C_6$-Alkyl, $C_3-C_6$-Cycloalkyl, $C_1-C_6$-Acyl oder $C_1-C_6$- Alkoxy substituiert sein kann, oder ein $C_6-C_{12}$-Aryl, welches gegebenenfalls ein- oder mehrfach, gleich oder verschieden substituiert sein kann mit Halogen, $C_1-C_6$-Alkyl, $C_3-C_6$-Cycloalkyl, $C_1-C_6$-Acyl, $C_1-C_6$-Alkoxy, $C_6-C_{12}$-Aryl, $C_5-C_{12}$-Heteroaryl, Hydroxy, $CH_2$-OH, Cyano, $CH_2$-CN, Amino, $CO_2-(C_1-C_6-Alkyl)$, $N-(C_1-C_6-Alkyl)_2$, $NHSO_2CH_3$, $SO_2NH_2$, $SO_2NH(C_1-C_6-Alkyl)$, $SO_2N(C_1-C_6-Alkyl)_2$, COOH, $CO-NH_2$, CO-NH-$(C_1-C_6$-Alkyl), $CO-N(C_1-C_6-Alkyl)_2$, CO-NH$(C_5-C_{12}$- Heteroaryl), NH-CO$(C_1-C_6$-Alkyl), $CH_2$-NH-CO$(C_1-C_6$-Alkyl), NH-CO$(C_5-C_{12}$-Heteroaryl) oder ein $-S(O)_r-CH_3$, wobei r für 0 - 2 steht, oder zwei benachbarte Positionen durch $-O-CH_2-O-$ oder -O-C $(CH_3)_2$- O- substituiert sein können, oder ein $C_5-C_{16}$-Heteroaryl, welches gegebenenfalls ein- oder mehrfach,

gleich oder verschieden substituiert sein kann mit Halogen, $C_1$-$C_6$-Alkyl, $C_3$-$C_6$-Cycloalkyl, $C_1$-$C_6$-Acyl, $C_1$-$C_6$-Alkoxy, $C_6$-$C_{12}$-Aryl, $C_5$-$C_{12}$-Heteroaryl, Hydroxy, $CH_2$-OH, Cyano, $CH_2$-CN, Amino, $CO_2$-($C_1$-$C_6$-Alkyl), N-($C_1$-$C_6$-Alkyl)$_2$, $NHSO_2CH_3$, $SO_2NH_2$, $SO_2NH$($C_1$-$C_6$-Alkyl), $SO_2N$($C_1$-$C_6$-Alkyl)$_2$, COOH, CO-$NH_2$, CO-NH-($C_1$-$C_6$-Alkyl), CO-N($C_1$-$C_6$-Alkyl)$_2$, CO-NH($C_5$-$C_{12}$- Heteroaryl), NH-CO($C_1$-$C_6$-Alkyl), $CH_2$-NH-CO($C_1$-$C_6$-Alkyl), NH-CO ($C_5$-$C_{12}$-Heteroaryl) oder ein -S(O)$_r$-$CH_3$, wobei r für 0 - 2 steht, oder ein $C_3$-$C_6$-Cycloalkyl, welches gegebenenfalls ein- oder mehrfach, gleich oder verschieden mit Halogen, $C_1$-$C_6$-Alkyl, Hydroxy, Cyano, $CO_2$-($C_1$-$C_6$-Alkyl), $C_1$-$C_6$-Acyl, N-($C_1$-$C_6$-Alkyl)$_2$, COOH, CO- $NH_2$, CO-NH($C_1$-$C_6$-Alkyl), CO-N($C_1$-$C_6$-Alkyl)$_2$ oder $C_1$-$C_6$-Alkoxy substituiert sein kann,

$R^2$ ein Wasserstoff, Halogen, Amino, eine -S(O)$_p$-$C_1$-$C_6$-Alkylgruppe, wobei p für 0 - 2 steht, ein $SO_2NH_2$, eine -$SO_2NH$-$C_1$-$C_6$-Alkylgruppe, eine -NH-$SO_2$-$C_1$-$C_6$-Alkylgruppe, $C_1$-$C_6$-Acyl-, NH-CO-$NH_2$, -O-CO-NH($C_1$-$C_6$-Alkyl), -O-CO-N($C_1$-$C_6$-Alkyl)$_2$ oder NH-CO-$C_1$-$C_6$-Alkylrest, Hydroxy, Cyano, O-CO-($C_1$-$C_6$-Alkyl), $CO_2$-($C_1$-$C_6$-Alkyl), CO-NH-($C_1$-$C_6$- Alkyl), CO-N($C_1$-$C_6$-Alkyl)$_2$, CO-NH($C_5$-$C_{12}$-Heteroaryl), NH-($C_1$-$C_6$- Alkyl), N-($C_1$-$C_6$-Alkyl)$_2$, oder ein $C_1$-$C_6$-Alkyl, welches gegebenenfalls ein- oder mehrfach, gleich oder verschieden substituiert sein kann mit COOH, CO-$NH_2$, CO- NH($C_1$-$C_6$-Alkyl) oder mit CO-N($C_1$-$C_6$-Alkyl)$_2$, $C_1$-$C_6$-Acyl, $C_1$-$C_6$-Alkoxy, Hydroxy, Cyano, $CO_2$-($C_1$-$C_6$-Alkyl), N-($C_1$-$C_6$- Alkyl)$_2$, $C_5$-$C_{12}$-Heteroaryl, welches gegebenenfalls mehrfach substituiert ist, $C_6$-$C_{12}$-Aryl, welches gegebenenfalls mehrfach substituiert ist, oder ein $C_1$-$C_6$-Alkoxy, welches gegebenenfalls ein- oder mehrfach, gleich oder verschieden substituiert sein kann mit Hydroxy, Cyano, $CO_2$-($C_1$-$C_6$-Alkyl), N-($C_1$-$C_6$-Alkyl)$_2$, NH-$C_3$-$C_6$-Cycloalkyl, COOH, CO-$NH_2$, CO-NH($C_1$-$C_6$-Alkyl) oder mit CO-N($C_1$-$C_6$- Alkyl)$_2$, ein O-$C_6$-$C_{12}$-Aryl, welches gegebenenfalls ein- oder mehrfach, gleich oder verschieden mit Halogen, Hydroxy, Cyano, COOH, CO- $NH_2$, $C_1$-$C_6$-Alkyl, $C_3$-$C_6$-Cycloalkyl, $C_1$-$C_6$-Acyl oder $C_1$-$C_6$- Alkoxy substituiert sein kann, oder ein O-$C_5$-$C_{12}$-Heteroaryl, welches gegebenenfalls ein- oder mehrfach, gleich oder verschieden mit Halogen, Hydroxy, Cyano, COOH, CO- $NH_2$, $C_1$-$C_6$-Alkyl, $C_3$-$C_6$-Cycloalkyl, $C_1$-$C_6$-Acyl oder $C_1$-$C_6$- Alkoxy substituiert sein kann, oder ein $C_6$-$C_{12}$-Aryl, welches gegebenenfalls ein- oder mehrfach, gleich oder verschieden substituiert sein kann mit Halogen, $C_1$-$C_6$-Alkyl, $C_3$-$C_6$-Cycloalkyl, $C_1$-$C_6$-Acyl, $C_1$-$C_6$-Alkoxy, $C_6$-$C_{12}$-Aryl, $C_5$- $C_{12}$-Heteroaryl, Hydroxy, $CH_2$-OH, Cyano, $CH_2$-CN, Amino, $CO_2$-($C_1$-$C_6$-Alkyl), N-($C_1$-$C_6$-Alkyl)$_2$, $NHSO_2CH_3$, $SO_2NH_2$, $SO_2NH$($C_1$-$C_6$-Alkyl), $SO_2N$($C_1$-$C_6$-Alkyl)$_2$, COOH, CO-$NH_2$, CO-NH($C_1$-$C_6$-Alkyl), CO-N($C_1$-$C_6$-Alkyl)$_2$, CO-NH($C_5$-$C_{12}$- Heteroaryl), NH-CO($C_1$-$C_6$-Alkyl), $CH_2$-NH-CO($C_1$-$C_6$-Alkyl), NH-CO ($C_5$-$C_{12}$-Heteroaryl) oder ein -S(O)$_r$-$CH_3$, wobei r für 0 - 2 steht, oder zwei benachbarte Positionen durch -O-$CH_2$-O- oder -O-C($CH_3$)$_2$- O- substituiert sein können, oder ein $C_5$-$C_{16}$-Heteroaryl, welches gegebenenfalls ein- oder mehrfach, gleich oder verschieden substituiert sein kann mit Halogen, $C_1$-$C_6$-Alkyl, $C_3$-$C_6$-Cycloalkyl, $C_1$-$C_6$-Acyl, $C_1$-$C_6$-Alkoxy, $C_6$-$C_{12}$-Aryl, $C_5$- $C_{12}$-Heteroaryl, Hydroxy, $CH_2$-OH, Cyano, $CH_2$-CN, Amino, $CO_2$-($C_1$-$C_6$-Alkyl), N-($C_1$-$C_6$-Alkyl)$_2$, $NHSO_2CH_3$, $SO_2NH_2$, $SO_2NH$($C_1$-$C_6$-Alkyl), $SO_2N$($C_1$-$C_6$-Alkyl)$_2$, COOH, CO-$NH_2$, CO-NH-($C_1$-$C_6$-Alkyl), CO-N($C_1$-$C_6$-Alkyl)$_2$, CO-NH($C_5$-$C_{12}$- Heteroaryl), NH-CO($C_1$-$C_6$-Alkyl), $CH_2$-NH-CO($C_1$-$C_6$-Alkyl), NH-CO($C_5$-$C_{12}$-Heteroaryl) oder ein -S(O)$_r$-$CH_3$, wobei r für 0 - 2 steht, oder ein $C_3$-$C_6$-Cycloalkyl, welches gegebenenfalls ein- oder mehrfach, gleich oder verschieden mit Halogen, $C_1$-$C_6$-Alkyl, Hydroxy, Cyano, $CO_2$-($C_1$-$C_6$-Alkyl), $C_1$-$C_6$-Acyl, N-($C_1$-$C_6$-Alkyl)$_2$, COOH, CO- $NH_2$, CO-NH($C_1$-$C_6$-Alkyl), CO-N($C_1$-$C_6$-Alkyl)$_2$ oder $C_1$-$C_6$- Alkoxy substituiert sein kann,

$R^3$ ein $C_6$-$C_{12}$-Aryl, welches gegebenenfalls ein- oder mehrfach, gleich oder verschieden mit Halogen, $C_1$-$C_6$-Alkyl, $C_1$-$C_6$-Alkoxy, Hydroxy, $C_1$-$C_6$-Alkylen-OH, $C_1$-$C_6$-Alkylen-$NR^5R^6$, Cyano, $CO_2$-($C_1$-$C_6$-Alkyl), N-($C_1$-$C_6$-Alkyl)$_2$, CO-NH-($C_1$-$C_6$-Alkyl), CO-NH-($C_1$-$C_6$-Alkylen-OH), CO-NH-($C_1$-$C_6$-Alkylen-$NR^5R^6$), CO-N($C_2$-$C_6$-Alkyl)$_2$ oder $C_1$-$C_6$-Acyl, welches gegebenenfalls ein- oder mehrfach substituiert ist, oder mit zwei benachbarten Positionen durch -O-$CH_2$-O-, -O- $CH_2$-$CH_2$-O-, oder -O-C($CH_3$)$_2$-O- substituiert sein kann, oder ein $C_5$-$C_{16}$-Heteroaryl, welches gegebenenfalls ein- oder mehrfach, gleich oder verschieden substituiert sein kann mit Halogen, $C_1$-$C_6$-Alkyl, $C_1$-$C_6$-Acyl, $C_1$-$C_6$-Alkoxy, Hydroxy, Cyano, $CO_2$-($C_1$-$C_6$-Alkyl), N-($C_1$-$C_6$-Alkyl)$_2$, CO-NH-($C_1$-$C_6$-Alkylen-OH), CO- NH-($C_1$-$C_6$-Alkylen-$NR^5R^6$), CO-NH($C_1$-$C_6$-Alkyl) oder CO- N($C_1$-$C_6$-Alkyl)$_2$, oder ein $C_3$-$C_6$-Cycloalkyl, welches gegebenenfalls ein- oder mehrfach, gleich oder verschieden substituiert sein kann mit Halogen, $CF_3$, Hydroxy, Cyano, $CO_2$-($C_1$-$C_6$-Alkyl), $C_1$-$C_6$-Alkyl, $C_1$-$C_6$-Acyl, N-($C_1$-$C_6$-Alkyl)$_2$, CO-NH-($C_1$-$C_6$-Alkylen-OH), CO-NH-($C_1$- $C_6$-Alkylen-$NR^5R^6$), CO-NH($C_1$-$C_6$-Alkyl) oder CO-N($C_1$-$C_6$- Alkyl)$_2$ oder $C_1$-$C_6$-Alkoxy,

$R^4$ ein Wasserstoff, ein $C_1$-$C_6$-Alkyl, welches gegebenenfalls ein- oder mehrfach, gleich oder verschieden substituiert sein kann mit einer -S(O)$_p$-$C_1$-$C_6$- Alkylgruppe oder einer -S(O)$_p$-$C_3$-$C_6$-Cycloalkylgruppe, wobei p für 0-2 steht, Hydroxy, Cyano, COOH, CO-$NH_2$, $SO_2NH_2$, $SO_2NH$-($C_1$-$C_6$-Alkyl), $C_3$-$C_6$-Cycloalkyl, $C_1$-$C_6$- Acyl, $C_1$-$C_6$-Alkoxy, $CO_2$-($C_1$-$C_6$-Alkyl), N-($C_1$-$C_6$-Alkyl)$_2$, NH-($C_1$-$C_6$-Alkyl), NH-($C_3$-$C_6$-Cycloalkyl), CO-NH($C_1$-$C_6$-Alkyl) oder mit CO-N($C_1$-$C_6$-Alkyl)$_2$, $C_6$-$C_{12}$-Aryl, welches gegebenenfalls mehrfach substituiert ist, $C_5$-$C_{12}$-Heteroaryl, welches gegebenenfalls mehrfach substituiert ist, oder ein $C_3$-$C_6$-Cycloalkyl, welches gegebenenfalls ein- oder mehrfach, gleich oder verschieden mit Hydroxy, $CH_2$-OH, Cyano, COOH, CO-$NH_2$, $SO_2NH_2$, $C_1$-$C_6$-Alkyl, $C_1$-$C_6$-Acyl oder $C_1$-$C_6$-Alkoxy substituiert sein kann, oder ein $C_6$-$C_{12}$-Aryl, welches gegebenenfalls ein- oder mehrfach, gleich oder verschieden mit Halogen, Hydroxy, $C_1$-$C_6$-Alkyl, $C_1$-$C_6$-Acyl, $C_1$-$C_6$-Alkoxy, N-($C_1$-$C_6$-Alkyl)$_2$, NH-($C_1$-$C_6$-Alkyl) oder Cyano substituiert sein kann, oder ein $C_5$-$C_{12}$-Heteroaryl, welches

gegebenenfalls ein- oder mehrfach, gleich oder verschieden mit Halogen, Amino, Hydroxy, Cyano, COOH, $C_1$-$C_6$-Alkyl, $C_1$-$C_6$-Acyl, $C_1$-$C_6$-Alkoxy, N-($C_1$-$C_6$-Alkyl)$_2$ oder NH-($C_1$-$C_6$-Alkyl) substituiert sein kann,
$R^5$, $R^6$ unabhängig voneinander Wasserstoff oder ein $C_1$-$C_6$-Alkyl, und
$X^1$ ein Stickstoff, wenn $X^2$ und $X^3$ eine CH-Gruppe sind, oder
$X^2$ ein Stickstoff, wenn $X^1$ und $X^3$ eine CH-Gruppe sind, oder
$X^3$ ein Stickstoff, wenn $X^1$ und $X^2$ eine CH-Gruppe sind,

bedeuten,
sowie deren Stereoisomere, Diastereomere, Enantiomere und Salze.

**4.** Verbindungen gemäß den Ansprüchen 1 - 3, wobei

A ein Phenyl oder ein monocyclischer $C_5$-$C_7$-Heteroarylrest, der gegebenenfalls ein- oder mehrfach mit $R^1$ und/oder $R^2$ substituiert sein kann,
$R^1$ ein Wasserstoff, Halogen, Amino, eine -S(O)$_p$-$C_1$-$C_6$-Alkylgruppe, wobei p für 0 - 2 steht, ein $SO_2NH_2$, eine -$SO_2$NH-$C_1$-$C_6$-Alkylgruppe, eine -NH-$SO_2$-$C_1$-$C_6$-Alkylgruppe, $C_1$-$C_6$-Acyl-, NH-CO-$NH_2$, -O-CO-NH($C_1$-$C_6$-Alkyl), -O-CO-N($C_1$-$C_6$-Alkyl)$_2$, NH-CO-$C_1$-$C_6$-Alkylrest, Hydroxy, Cyano, O-CO-($C_1$-$C_6$-Alkyl), $CO_2$-($C_1$-$C_6$-Alkyl), CO-NH-($C_1$-$C_6$-Alkyl), CO- N($C_1$-$C_6$-Alkyl)$_2$, CO-NH($C_5$-$C_{12}$-Heteroaryl), NH-($C_1$-$C_6$-Alkyl), N- ($C_1$-$C_6$-Alkyl)$_2$, oder ein $C_1$-$C_6$-Alkyl, welches gegebenenfalls ein- oder mehrfach, gleich oder verschieden substituiert sein kann mit COOH, CO-$NH_2$, CO- NH-($C_1$-$C_6$-Alkyl), CO-N($C_1$-$C_6$-Alkyl)$_2$, $C_1$-$C_6$-Acyl, $C_1$-$C_6$- Alkoxy, Hydroxy, Cyano, $CO_2$-($C_1$-$C_6$-Alkyl), N-($C_1$-$C_6$- Alkyl)$_2$, $C_5$-$C_{12}$-Heteroaryl, welches gegebenenfalls mehrfach substituiert ist, $C_6$-$C_{12}$-Aryl, welches gegebenenfalls mehrfach substituiert ist, oder ein $C_1$-$C_6$-Alkoxy, welches gegebenenfalls ein- oder mehrfach, gleich oder verschieden substituiert sein kann mit Hydroxy, Cyano, $CO_2$-($C_1$-$C_6$-Alkyl), N-($C_1$-$C_6$-Alkyl)$_2$, NH-$C_3$-$C_6$-Cycloalkyl, COOH, CO-$NH_2$, CO-NH($C_1$-$C_6$-Alkyl) oder mit CO-N($C_1$-$C_6$- Alkyl)$_2$, oder ein O-$C_6$-$C_{12}$-Aryl, welches gegebenenfalls ein- oder mehrfach, gleich oder verschieden mit Halogen, Hydroxy, Cyano, COOH, CO- $NH_2$, $C_1$-$C_6$-Alkyl, $C_3$-$C_6$-Cycloalkyl, $C_1$-$C_6$-Acyl oder $C_1$-$C_6$- Alkoxy substituiert sein kann, oder ein O-$C_5$-$C_{12}$-Heteroaryl, welches gegebenenfalls ein- oder mehrfach, gleich oder verschieden mit Halogen, Hydroxy, Cyano, COOH, CO- $NH_2$, $C_1$-$C_6$-Alkyl, $C_3$-$C_6$-Cycloalkyl, $C_1$-$C_6$-Acyl oder $C_1$-$C_6$- Alkoxy substituiert sein kann, oder ein $C_6$-$C_{12}$-Aryl, welches gegebenenfalls ein- oder mehrfach, gleich oder verschieden substituiert sein kann mit Halogen, $C_1$-$C_6$-Alkyl, $C_3$-$C_6$-Cycloalkyl, $C_1$-$C_6$-Acyl, $C_1$-$C_6$-Alkoxy, $C_6$-$C_{12}$-Aryl, $C_5$- $C_{12}$-Heteroaryl, Hydroxy, $CH_2$-OH, Cyano, $CH_2$-CN, Amino, $CO_2$-($C_1$-$C_6$-Alkyl), N-($C_1$-$C_6$-Alkyl)$_2$, $NHSO_2CH_3$, $SO_2NH_2$, $SO_2$NH($C_1$-$C_6$-Alkyl), $SO_2$N($C_1$-$C_6$-Alkyl)$_2$, COOH, CO-$NH_2$, CO-NH-($C_1$-$C_6$-Alkyl), CO-N($C_1$-$C_6$-Alkyl)$_2$, CO-NH($C_5$-$C_{12}$- Heteroaryl), NH-CO($C_1$-$C_6$-Alkyl), $CH_2$-NH-CO($C_1$-$C_6$-Alkyl), NH-CO ($C_5$-$C_{12}$-Heteroaryl) oder ein -S(O)$_r$-$CH_3$, wobei r für 0 - 2 steht, oder zwei benachbarte Positionen durch -O-$CH_2$-O- oder -O-C($CH_3$)$_2$- O- substituiert sein können, oder ein $C_5$-$C_{16}$-Heteroaryl, welches gegebenenfalls ein- oder mehrfach, gleich oder verschieden substituiert sein kann mit Halogen, $C_1$-$C_6$-Alkyl, $C_3$-$C_6$-Cycloalkyl, $C_1$-$C_6$-Acyl, $C_1$-$C_6$-Alkoxy, $C_6$-$C_{12}$-Aryl, $C_5$- $C_{12}$-Heteroaryl, Hydroxy, $CH_2$-OH, Cyano, $CH_2$-CN, Amino, $CO_2$-($C_1$-$C_6$-Alkyl), N-($C_1$-$C_6$-Alkyl)$_2$, $NHSO_2CH_3$, $SO_2NH_2$, $SO_2$NH($C_1$-$C_6$-Alkyl), $SO_2$N($C_1$-$C_6$-Alkyl)$_2$, COOH, CO-$NH_2$, CO-NH-($C_1$-$C_6$-Alkyl), CO-N($C_1$-$C_6$-Alkyl)$_2$, CO-NH($C_5$-$C_{12}$- Heteroaryl), NH-CO($C_1$-$C_6$-Alkyl), $CH_2$-NH-CO($C_1$-$C_6$-Alkyl), NH-CO($C_5$-$C_{12}$-Heteroaryl) oder ein -S(O)$_r$-$CH_3$, wobei r für 0 - 2 steht, oder ein $C_3$-$C_6$-Cycloalkyl, welches gegebenenfalls ein- oder mehrfach, gleich oder verschieden mit Halogen, $C_1$-$C_6$-Alkyl, Hydroxy, Cyano, $CO_2$-($C_1$-$C_6$-Alkyl), $C_1$-$C_6$-Acyl, N-($C_1$-$C_6$-Alkyl)$_2$, COOH, CO- $NH_2$, CO-NH($C_1$-$C_6$-Alkyl), CO-N($C_1$-$C_6$-Alkyl)$_2$ oder $C_1$-$C_6$- Alkoxy substituiert sein kann,
$R^2$ ein Wasserstoff, Halogen, $C_1$-$C_6$-Alkyl, $C_1$-$C_6$-Alkoxy, $C_1$-$C_6$-Acyl-, $C_6$-$C_{12}$-Aryl, $C_5$-$C_{16}$-Heteroaryl, $C_3$-$C_6$-Cycloalkyl, NH-CO-$NH_2$, -O- CO-NH($C_1$-$C_6$-Alkyl), -O-CO-N($C_1$-$C_6$-Alkyl)$_2$, NH-CO-$C_1$-$C_6$- Alkylrest, Hydroxy, Cyano, O-CO-($C_1$-$C_6$-Alkyl), $CO_2$-($C_1$-$C_6$-Alkyl), CO-NH-($C_1$-$C_6$-Alkyl), CO-N($C_1$-$C_6$-Alkyl)$_2$, CO-NH($C_5$-$C_{12}$- Heteroaryl), NH-($C_1$-$C_6$-Alkyl), N-($C_1$-$C_6$-Alkyl)$_2$, O-$C_6$-$C_{12}$-Aryl, O- $C_5$-$C_{12}$-Heteroaryl, oder
$R^3$ ein $C_6$-$C_{12}$-Aryl, welches gegebenenfalls ein- oder mehrfach, gleich oder verschieden substituiert sein kann mit Halogen, $C_1$-$C_6$-Alkyl, $C_1$-$C_6$-Alkoxy, Hydroxy, $C_1$-$C_6$-Alkylen-OH, $C_1$-$C_6$-Alkylen- $NR^5R^6$, Cyano, $CO_2$-($C_1$-$C_6$-Alkyl), N-($C_1$-$C_6$-Alkyl)$_2$, CO-NH- ($C_1$-$C_6$-Alkyl), CO-NH-($C_1$-$C_6$-Alkylen-OH), CO-NH-($C_1$-$C_6$- Alkylen-$NR^5R^6$), CO-N($C_1$-$C_6$-Alkyl)$_2$ oder $C_1$-$C_6$-Acyl, welches gegebenenfalls ein- oder mehrfach substituiert ist, oder mit zwei benachbarten Positionen durch -O-$CH_2$-O-, -O- $CH_2$-$CH_2$-O-, oder -O-C($CH_3$)$_2$-O- substituiert sein kann, oder ein $C_5$-$C_{16}$-Heteroaryl, welches gegebenenfalls ein- oder mehrfach, gleich oder verschieden substituiert sein kann mit Halogen, $C_1$-$C_6$-Alkyl, $C_1$-$C_6$-Acyl, $C_1$-$C_6$-Alkoxy, Hydroxy, Cyano, $CO_2$-($C_1$-$C_6$- Alkyl), N-($C_1$-$C_6$-Alkyl)$_2$, CO-NH-($C_1$-$C_6$-Alkylen-OH), CO- NH-($C_1$-$C_6$-Alkylen-$NR^5R^6$), CO-NH($C_1$-$C_6$-Alkyl) oder CO-N($C_1$-$C_6$-Alkyl)$_2$ oder ein $C_3$-$C_6$-Cycloalkyl, welches gegebenenfalls ein- oder mehrfach, gleich oder verschieden mit Halogen, $CF_3$, Hydroxy, Cyano, $CO_2$-($C_1$- $C_6$-Alkyl), $C_1$-$C_6$-Alkyl, $C_1$-$C_6$-Acyl, N-($C_1$-$C_6$-Alkyl)$_2$, CO-NH-($C_1$-$C_6$-Alkylen-OH), CO-NH-($C_1$-$C_6$-Alkylen-$NR^5R^6$), CO- NH($C_1$-$C_6$-Alkyl) oder CO-N($C_1$-$C_6$-Alkyl)$_2$ oder

$C_1$-$C_6$-Alkoxy substituiert sein kann,
$R^4$ ein Wasserstoff, ein $C_1$-$C_6$-Alkyl, welches gegebenenfalls ein- oder mehrfach, gleich oder verschieden substituiert sein kann mit einer -$S(O)_p$-$C_1$-$C_6$- Alkylgruppe oder einer -$S(O)_p$-$C_3$-$C_6$-Cycloalkylgruppe, wobei p für 0 - 2 steht, Hydroxy, Cyano, COOH, CO-$NH_2$, $SO_2NH_2$, $SO_2$NH-($C_1$-$C_6$-Alkyl), $C_3$-$C_6$-Cycloalkyl, $C_1$-$C_6$-Acyl, $C_1$-$C_6$-Alkoxy, $CO_2$-($C_1$-$C_6$-Alkyl), N-($C_1$-$C_6$-Alkyl)$_2$, NH- ($C_1$-$C_6$-Alkyl), NH-($C_3$-$C_6$-Cycloalkyl), CO-NH ($C_1$-$C_6$-Alkyl) oder mit CO-N($C_1$-$C_6$-Alkyl)$_2$, $C_6$-$C_{12}$-Aryl, welches gegebenenfalls mehrfach substituiert ist, $C_5$-$C_{12}$-Heteroaryl, welches gegebenenfalls mehrfach substituiert ist, oder ein $C_3$-$C_6$-Cycloalkyl, welches gegebenenfalls ein- oder mehrfach, gleich oder verschieden mit Hydroxy, $CH_2$-OH, Cyano, COOH, CO-$NH_2$, $SO_2NH_2$, $C_1$-$C_6$-Alkyl, $C_1$-$C_6$-Acyl oder $C_1$-$C_6$-Alkoxy substituiert sein kann, oder ein $C_6$-$C_{12}$-Aryl, welches gegebenenfalls ein- oder mehrfach, gleich oder verschieden mit Halogen, Hydroxy, $C_1$-$C_6$-Alkyl, $C_1$-$C_6$-Acyl, $C_1$-$C_6$-Alkoxy, N-($C_1$-$C_6$-Alkyl)$_2$, NH-($C_1$-$C_6$-Alkyl) oder Cyano substituiert sein kann, oder ein $C_5$-$C_{12}$-Heteroaryl, welches gegebenenfalls ein- oder mehrfach, gleich oder verschieden mit Halogen, Amino, Hydroxy, Cyano, COOH, $C_1$-$C_6$-Alkyl, $C_1$-$C_6$-Acyl, $C_1$-$C_6$-Alkoxy, N-($C_1$-$C_6$-Alkyl)$_2$ oder NH-($C_1$-$C_6$-Alkyl) substituiert sein kann,
$R^5$, $R^6$ unabhängig voneinander Wasserstoff oder ein $C_1$-$C_6$-Alkyl, und
$X^1$ ein Stickstoff, wenn $X^2$ und $X^3$ eine CH-Gruppe sind, oder
$X^2$ ein Stickstoff, wenn $X^1$ und $X^3$ eine CH-Gruppe sind, oder
$X^3$ ein Stickstoff, wenn $X^1$ und $X^2$ eine CH-Gruppe sind,

bedeuten,
sowie deren Stereoisomere, Diastereomere, Enantiomere und Salze.

**5.** Verbindungen gemäß den Ansprüchen 1 - 4, wobei

A ein Phenyl oder ein monocyclischer $C_5$-$C_7$-Heteroarylrest, der gegebenenfalls ein- oder mehrfach mit $R^1$ und/ oder $R^2$ substituiert sein kann,
$R^1$ ein Wasserstoff, Halogen, Amino, eine -$S(O)_p$-$C_1$-$C_6$-Alkylgruppe, wobei p für 0 - 2 steht,
ein $SO_2NH_2$, eine -$SO_2$NH-$C_1$-$C_6$-Alkylgruppe, eine -NH-$SO_2$-$C_1$- $C_6$-Alkylgruppe, $C_1$-$C_6$-Acyl-, NH-CO-$NH_2$, -O-CO-NH($C_1$-$C_6$-Alkyl), -O-CO-N($C_1$-$C_6$-Alkyl)$_2$, NH-CO-$C_1$-$C_6$-Alkylrest, Hydroxy, Cyano, O-CO-($C_1$-$C_6$-Alkyl), $CO_2$-($C_1$-$C_6$-Alkyl), CO-NH-($C_1$-$C_6$-Alkyl), CO- N($C_1$-$C_6$-Alkyl)$_2$, CO-NH($C_5$-$C_{12}$-Heteroaryl), NH-($C_1$-$C_6$-Alkyl), N- ($C_1$-$C_6$-Alkyl)$_2$, oder
ein $C_1$-$C_6$-Alkyl,
welches gegebenenfalls ein- oder mehrfach, gleich oder verschieden substituiert sein kann mit COOH, CO-$NH_2$, CO- NH-($C_1$-$C_6$-Alkyl), CO-N($C_1$-$C_6$-Alkyl)$_2$, $C_1$-$C_6$-Acyl, $C_1$-$C_6$- Alkoxy, Hydroxy, Cyano, CO2-($C_1$-$C_6$-Alkyl), N-($C_1$-$C_6$- Alkyl)$_2$, $C_5$-$C_{12}$-Heteroaryl,
welches gegebenenfalls mehrfach substituiert ist, $C_6$-$C_{12}$-Aryl,
welches gegebenenfalls mehrfach substituiert ist,
oder
ein $C_1$-$C_6$-Alkoxy,
welches gegebenenfalls ein- oder mehrfach, gleich oder verschieden substituiert sein kann mit Hydroxy, Cyano, $CO_2$-($C_1$-$C_6$-Alkyl), N-($C_1$-$C_6$-Alkyl)$_2$, NH-$C_3$-$C_6$-Cycloalkyl, COOH, CO-$NH_2$, CO-NH($C_1$-$C_6$-Alkyl) oder mit CO-N($C_1$-$C_6$- Alkyl)$_2$, oder
ein O-$C_6$-$C_{12}$-Aryl,
welches gegebenenfalls ein- oder mehrfach, gleich oder verschieden mit Halogen, Hydroxy, Cyano, COOH, CO- $NH_2$, $C_1$-$C_6$-Alkyl, $C_3$-$C_6$-Cycloalkyl, $C_1$-$C_6$-Acyl oder $C_1$-$C_6$- Alkoxy substituiert sein kann, oder
ein O-$C_5$-$C_{12}$-Heteroaryl,
welches gegebenenfalls ein- oder mehrfach, gleich oder verschieden mit Halogen, Hydroxy, Cyano, COOH, CO- $NH_2$, $C_1$-$C_6$-Alkyl, $C_3$-$C_6$-Cycloalkyl, $C_1$-$C_6$-Acyl oder $C_1$-$C_6$- Alkoxy substituiert sein kann, oder
ein $C_6$-$C_{12}$-Aryl,
welches gegebenenfalls ein- oder mehrfach, gleich oder verschieden substituiert sein kann mit Halogen, $C_1$-$C_6$-Alkyl, $C_3$-$C_6$-Cycloalkyl, $C_1$-$C_6$-Acyl, $C_1$-$C_6$-Alkoxy, $C_6$-$C_{12}$-Aryl, $C_5$- $C_{12}$-Heteroaryl, Hydroxy, $CH_2$-OH, Cyano, $CH_2$-CN, Amino, $CO_2$-($C_1$-$C_6$-Alkyl), N-($C_1$-$C_6$-Alkyl)$_2$, $NHSO_2CH_3$, $SO_2NH_2$, $SO_2$NH($C_1$-$C_6$-Alkyl), $SO_2$N ($C_1$-$C_6$-Alkyl)$_2$, COOH, CO-$NH_2$, CO-NH-($C_1$-$C_6$-Alkyl), CO-N($C_1$-$C_6$-Alkyl)$_2$, CO-NH($C_5$-$C_{12}$- Heteroaryl), NH-CO($C_1$-$C_6$-Alkyl), $CH_2$-NH-CO($C_1$-$C_6$-Alkyl), NH-CO($C_5$-$C_{12}$-Heteroaryl) oder ein -$S(O)_r$-$CH_3$, wobei r für 0 - 2 steht, oder
zwei benachbarte Positionen durch -O-$CH_2$-O- oder -O-C($CH_3$)$_2$- O- substituiert sein können, oder
ein $C_5$-$C_{12}$-Heteroaryl,
welches gegebenenfalls ein- oder mehrfach, gleich oder verschieden substituiert sein kann mit Halogen, $C_1$-$C_6$-Al-

kyl, $C_3$-$C_6$-Cycloalkyl, $C_1$-$C_6$-Acyl, $C_1$-$C_6$-Alkoxy, $C_6$-$C_{12}$-Aryl, $C_5$- $C_{12}$-Heteroaryl, Hydroxy, $CH_2$-OH, Cyano, $CH_2$-CN, Amino, $CO_2$-($C_1$-$C_6$-Alkyl), N-($C_1$-$C_6$-Alkyl)$_2$, $NHSO_2CH_3$, $SO_2NH_2$, $SO_2NH$($C_1$-$C_6$-Alkyl), $SO_2N$($C_1$-$C_6$-Alkyl)$_2$, COOH, CO-$NH_2$, CO-NH-($C_1$-$C_6$-Alkyl), CO-N($C_1$-$C_6$-Alkyl)$_2$, CO-NH($C_5$-$C_{12}$- Heteroaryl), NH-CO($C_1$-$C_6$-Alkyl), $CH_2$-NH-CO($C_1$-$C_6$-Alkyl), NH-CO($C_5$-$C_{12}$-Heteroaryl) oder ein -S(O)$_r$-$CH_3$, wobei r für 0 - 2 steht, oder

ein $C_3$-$C_6$-Cycloalkyl,

welches gegebenenfalls ein- oder mehrfach, gleich oder verschieden mit Halogen, $C_1$-$C_6$-Alkyl, Hydroxy, Cyano, $CO_2$-($C_1$-$C_6$-Alkyl), $C_1$-$C_6$-Acyl, N-($C_1$-$C_6$-Alkyl)$_2$, COOH, CO- $NH_2$, CO-NH($C_1$-$C_6$-Alkyl), CO-N($C_1$-$C_6$-Alkyl)$_2$ oder $C_1$-$C_6$- Alkoxy substituiert sein kann,

$R^2$ ein Wasserstoff, Halogen, $C_1$-$C_6$-Acyl-, NH-CO-$NH_2$, -O-CO- NH($C_1$-$C_6$-Alkyl), -O-CO-N($C_1$-$C_6$-Alkyl)$_2$, ein NH-CO-$C_1$-$C_6$- Alkylrest, Hydroxy, Cyano, O-CO-($C_1$-$C_6$-Alkyl), $CO_2$-($C_1$-$C_6$-Alkyl), CO-NH-($C_1$-$C_6$-Alkyl), CO-N($C_1$-$C_6$-Alkyl)$_2$, CO-NH($C_5$-$C_{12}$- Heteroaryl), NH-($C_1$-$C_6$-Alkyl), N-($C_1$-$C_6$-Alkyl)$_2$, $C_1$-$C_6$-Alkyl, $C_1$-$C_6$- Alkoxy, O-$C_6$-$C_{12}$-Aryl, O-$C_5$-$C_{12}$-Heteroaryl, $C_6$-$C_{12}$-Aryl, $C_5$-$C_{16}$- Heteroaryl, $C_3$-$C_6$-Cycloalkyl,

$R^3$ ein $C_6$-$C_{12}$-Aryl,

welches gegebenenfalls ein- oder mehrfach, gleich oder verschieden substituiert sein kann mit Halogen, $C_1$-$C_6$-Alkyl, $C_1$-$C_6$-Alkoxy, Hydroxy, $C_1$-$C_6$-Alkylen-OH, $C_1$-$C_6$-Alkylen- $NR^5R^6$, Cyano, $CO_2$-($C_1$-$C_6$-Alkyl), N-($C_1$-$C_6$-Alkyl)$_2$, CO-NH- ($C_1$-$C_6$-Alkyl), CO-NH-($C_1$-$C_6$-Alkylen-OH), CO-NH-($C_1$-$C_6$- Alkylen-$NR^5R^6$), CO-N($C_1$-$C_6$-Alkyl)$_2$ oder $C_1$-$C_6$-Acyl,

welches gegebenenfalls ein- oder mehrfach substituiert ist,

oder

mit zwei benachbarten Positionen durch -O-$CH_2$-O-, -O- $CH_2$-$CH_2$-O-, oder -O-C($CH_3$)$_2$-O- substituiert sein kann, oder

ein $C_5$-$C_{12}$-Heteroaryl,

welches gegebenenfalls ein- oder mehrfach, gleich oder verschieden mit Halogen, $C_1$-$C_6$-Alkyl, $C_1$-$C_6$-Acyl, $C_1$-$C_6$- Alkoxy, Hydroxy, Cyano, $CO_2$-($C_1$-$C_6$-Alkyl), N-($C_1$-$C_6$- Alkyl)$_2$, CO-NH-($C_1$-$C_6$-Alkylen-OH), CO-NH-($C_1$-$C_6$-Alkylen- $NR^5R^6$), CO-NH($C_1$-$C_6$-Alkyl) oder CO-N($C_1$-$C_6$-Alkyl)$_2$ substituiert sein kann,

$R^4$ ein Wasserstoff,

ein $C_1$-$C_6$-Alkyl,

welches gegebenenfalls ein- oder mehrfach, gleich oder verschieden substituiert sein kann mit einer -S(O)$_p$-$C_1$-$C_6$- Alkylgruppe oder -S(O)$_p$-$C_3$-$C_6$-Cycloalkylgruppe, wobei p für 0 - 2 steht, Hydroxy, Cyano, COOH, CO-$NH_2$, $SO_2NH_2$, $SO_2NH$-($C_1$-$C_6$-Alkyl), $C_3$-$C_6$-Cycloalkyl, $C_1$-$C_6$-Acyl, $C_1$-$C_6$- Alkoxy, $CO_2$-($C_1$-$C_6$-Alkyl), N-($C_1$-$C_6$-Alkyl)$_2$, NH-($C_1$-$C_6$- Alkyl), NH-($C_3$-$C_6$-Cycloalkyl), CO-NH($C_1$-$C_6$-Alkyl) oder mit CO-N($C_1$-$C_6$-Alkyl)$_2$, oder $C_6$-$C_{12}$-Aryl,

welches gegebenenfalls mehrfach substituiert ist, $C_5$-$C_{12}$-Heteroaryl,

welches gegebenenfalls mehrfach substituiert ist, oder

ein $C_3$-$C_6$-Cycloalkyl,

welches gegebenenfalls ein- oder mehrfach, gleich oder verschieden mit Hydroxy, $CH_2$-OH, Cyano, COOH, CO-$NH_2$, $SO_2NH_2$, $C_1$-$C_6$-Alkyl, $C_1$-$C_6$-Acyl oder $C_1$-$C_6$-Alkoxy substituiert sein kann, oder

ein $C_6$-$C_{12}$-Aryl,

welches gegebenenfalls ein- oder mehrfach, gleich oder verschieden mit Halogen, Hydroxy, $C_1$-$C_6$-Alkyl, $C_1$-$C_6$-Acyl, $C_1$-$C_6$-Alkoxy, N-($C_1$-$C_6$-Alkyl)$_2$, NH-($C_1$-$C_6$-Alkyl) oder Cyano substituiert sein kann, oder

ein $C_5$-$C_{12}$-Heteroaryl,

welches gegebenenfalls ein- oder mehrfach, gleich oder verschieden mit Halogen, Amino, Hydroxy, Cyano, COOH, $C_1$-$C_6$-Alkyl, $C_1$-$C_6$-Acyl, $C_1$-$C_6$-Alkoxy, N-($C_1$-$C_6$-Alkyl)$_2$ oder NH-($C_1$-$C_6$-Alkyl) substituiert sein kann,

$R^5$, $R^6$ unabhängig voneinander Wasserstoff oder ein $C_1$-$C_6$-Alkyl, und

$X^1$ ein Stickstoff, wenn $X^2$ und $X^3$ eine CH-Gruppe sind, oder

$X^2$ ein Stickstoff, wenn $X^1$ und $X^3$ eine CH-Gruppe sind, oder

$X^3$ ein Stickstoff, wenn $X^1$ und $X^2$ eine CH-Gruppe sind,

bedeuten,
sowie deren Stereoisomere, Diastereomere, Enantiomere und Salze.

**6.** Verbindungen gemäß den Ansprüchen 1 - 5, wobei

A ein Phenyl oder ein monocyclischer $C_5$-$C_7$-Heteroarylrest, der gegebenenfalls ein- oder mehrfach mit $R^1$ und/oder $R^2$ substituiert sein kann,

$R^1$ ein Wasserstoff, Halogen, eine -S(O)$_p$-$C_1$-$C_6$-Alkylgruppe, wobei p für 0 - 2 steht,

ein $SO_2NH_2$, eine -$SO_2NH$-$C_1$-$C_6$-Alkylgruppe, eine -NH-$SO_2$-$C_1$- $C_6$-Alkylgruppe, oder

ein $C_1$-$C_6$-Acyl-, Hydroxy, Cyano, CO-NH-($C_1$-$C_6$-Alkyl), CO-N($C_1$- $C_6$-Alkyl)$_2$, CO-NH($C_5$-$C_{12}$-Heteroaryl), NH-($C_1$-$C_6$-Alkyl), N-($C_1$-$C_6$- Alkyl)$_2$, oder
ein $C_1$-$C_6$-Alkyl, welches gegebenenfalls ein- oder mehrfach, gleich oder verschieden substituiert sein kann mit $C_1$-$C_6$-Alkoxy, Hydroxy, Cyano, N-($C_1$-$C_6$-Alkyl)$_2$, $C_5$-$C_{12}$-Heteroaryl,
welches gegebenenfalls ein oder mehrfach, gleich oder verschieden mit Halogen oder $C_1$-$C_6$-Alkyl substituiert ist,
$C_6$-$C_{12}$-Aryl,
welches gegebenenfalls ein- oder mehrfach, gleich oder verschieden mit Halogen oder $C_1$-$C_6$-Alkyl substituiert ist, oder
ein $C_1$-$C_6$-Alkoxy, oder
ein O-$C_6$-$C_{12}$-Aryl,
welches gegebenenfalls ein- oder mehrfach, gleich oder verschieden substituiert sein kann mit Halogen oder $C_1$-$C_6$- Alkyl, $C_3$-$C_6$-Cycloalkyl oder $C_1$-$C_6$-Alkoxy substituiert sein kann, oder
ein O-$C_5$-$C_{12}$-Heteroaryl,
welches gegebenenfalls ein- oder mehrfach, gleich oder verschieden substituiert sein kann mit Halogen oder $C_1$-$C_6$- Alkyl, $C_3$-$C_6$-Cycloalkyl oder $C_1$-$C_6$-Alkoxy, oder
ein Phenyl,
welches gegebenenfalls ein- oder mehrfach, gleich oder verschieden substituiert sein kann mit Halogen, $C_1$-$C_6$-Alkyl, $C_3$-$C_6$-Cycloalkyl, $C_1$-$C_6$-Acyl, $C_1$-$C_6$-Alkoxy, $C_6$-$C_{12}$-Aryl, $C_5$- $C_{12}$-Heteroaryl, Hydroxy, $CH_2$-OH, Cyano, $CH_2$-CN, Amino, $CO_2$-($C_1$-$C_6$-Alkyl), N-($C_1$-$C_6$-Alkyl)$_2$, $NHSO_2CH_3$, $SO_2NH_2$, $SO_2$NH($C_1$-$C_6$-Alkyl), $SO_2$N($C_1$-$C_6$-Alkyl)$_2$, COOH, CO-$NH_2$, CO-NH-($C_1$-$C_6$-Alkyl), CO-N($C_1$-$C_6$-Alkyl)$_2$, CO-NH($C_5$-$C_{12}$- Heteroaryl), NH-CO($C_1$-$C_6$-Alkyl), $CH_2$-NH-CO($C_1$-$C_6$-Alkyl), NH-CO($C_5$-$C_{12}$-Heteroaryl) oder ein -S(O)$_r$-$CH_3$, wobei r für 0 - 2 steht, oder
zwei benachbarte Positionen durch -O-$CH_2$-O- oder -O-C($CH_3$)$_2$- O- substituiert sein können, oder
ein monocyclischer $C_5$-$C_7$-Heteroarylrest,
welcher gegebenenfalls ein- oder mehrfach, gleich oder verschieden substituiert sein kann mit Halogen, mit $C_1$-$C_6$- Alkyl, $C_3$-$C_6$-Cycloalkyl, $C_1$-$C_6$-Acyl, $C_1$-$C_6$-Alkoxy, $C_6$-$C_{12}$- Aryl, $C_5$-$C_{12}$-Heteroaryl, Hydroxy, $CH_2$-OH, Cyano, $CH_2$-CN, Amino, $CO_2$-($C_1$-$C_6$-Alkyl), N-($C_1$-$C_6$-Alkyl)$_2$, $NHSO_2CH_3$, $SO_2NH_2$, $SO_2$NH($C_1$-$C_6$-Alkyl), $SO_2$N($C_1$-$C_6$-Alkyl)$_2$, COOH, CO-$NH_2$, CO-NH($C_1$-$C_6$-Alkyl), CO-N($C_1$-$C_6$-Alkyl)$_2$, CO- NH($C_5$-$C_{12}$-Heteroaryl), NH-CO($C_1$-$C_6$-Alkyl), $CH_2$-NH- CO($C_1$-$C_6$-Alkyl), NH-CO($C_5$-$C_{12}$-Heteroaryl) oder ein -S(O)$_r$- $CH_3$, wobei r für 0 - 2 steht, oder
ein $C_3$-$C_6$-Cycloalkyl,
welches gegebenenfalls ein- oder mehrfach, gleich oder verschieden mit Halogen, mit $C_1$-$C_6$-Alkyl, Hydroxy, Cyano, $CO_2$-($C_1$-$C_6$-Alkyl), $C_1$-$C_6$-Acyl, N-($C_1$-$C_6$-Alkyl)$_2$, COOH, CO- $NH_2$, CO-NH($C_1$-$C_6$-Alkyl), CO-N($C_1$-$C_6$-Alkyl)$_2$ oder $C_1$-$C_6$- Alkoxy substituiert sein kann,
$R^2$ ein Wasserstoff,
$R^3$ ein $C_6$-$C_{12}$-Aryl,
welches gegebenenfalls ein- oder mehrfach, gleich oder verschieden substituiert sein kann mit Halogen, $C_1$-$C_6$-Alkyl, $C_1$-$C_6$-Alkoxy, Hydroxy, $C_1$-$C_6$-Alkylen-OH, $C_1$-$C_6$-Alkylen- $NR^5R^6$, Cyano, $CO_2$-($C_1$-$C_6$-Alkyl), N-($C_1$-$C_6$-Alkyl)$_2$, CO-NH- ($C_1$-$C_6$-Alkyl), CO-NH-($C_1$-$C_6$-Alkylen-OH), CO-NH-($C_1$-$C_6$- Alkylen-$NR^5R^6$), CO-N($C_1$-$C_6$-Alkyl)$_2$ oder $C_1$-$C_6$-Acyl,
welches gegebenenfalls ein- oder mehrfach substituiert ist,
oder
mit zwei benachbarten Positionen durch -O-$CH_2$-O-, -O- $CH_2$-$CH_2$-O-, oder -O-C($CH_3$)$_2$-O- substituiert sein kann,
oder
ein $C_5$-$C_{12}$-Heteroaryl,
welches gegebenenfalls ein- oder mehrfach, gleich oder verschieden mit Halogen, $C_1$-$C_6$-Alkyl, $C_1$-$C_6$-Acyl, $C_1$-$C_6$- Alkoxy, Hydroxy, Cyano, $CO_2$-($C_1$-$C_6$-Alkyl), N-($C_1$-$C_6$- Alkyl)$_2$, CO-NH-($C_1$-$C_6$-Alkylen-OH), CO-NH-($C_1$-$C_6$-Alkylen- $NR^5R^6$), CO-NH($C_1$-$C_6$-Alkyl) oder CO-N($C_1$-$C_6$-Alkyl)$_2$ substituiert sein kann,
$R^4$ ein Wasserstoff,
ein $C_1$-$C_6$-Alkyl,
welches gegebenenfalls ein- oder mehrfach, gleich oder verschieden substituiert sein kann mit einer -S(O)$_p$-$C_1$-$C_6$- Alkylgruppe oder -S(O)$_p$-$C_3$-$C_6$-Cycloalkylgruppe, wobei p für 0 - 2 steht, Hydroxy, Cyano, COOH, CO-$NH_2$, $SO_2NH_2$, $SO_2$NH-($C_1$-$C_6$-Alkyl), $C_3$-$C_6$-Cycloalkyl, $C_1$-$C_6$-Acyl, $C_1$-$C_6$- Alkoxy, $CO_2$-($C_1$-$C_6$-Alkyl), N-($C_1$-$C_6$-Alkyl)$_2$, NH-($C_1$-$C_6$- Alkyl), NH-($C_3$-$C_6$-Cycloalkyl), CO-NH($C_1$-$C_6$-Alkyl) oder mit CO-N($C_1$-$C_6$-Alkyl)$_2$, $C_6$-$C_{12}$-Aryl, welches gegebenenfalls mehrfach substituiert ist, $C_5$-$C_{12}$-Heteroaryl,
welches gegebenenfalls mehrfach substituiert ist,

oder
ein $C_3$-$C_6$-Cycloalkyl,
welches gegebenenfalls ein- oder mehrfach, gleich oder verschieden mit Hydroxy, $CH_2$-OH, Cyano, COOH, CO-$NH_2$, $SO_2NH_2$, $C_1$-$C_6$-Alkyl, $C_1$-$C_6$-Acyl oder $C_1$-$C_6$-Alkoxy substituiert sein kann, oder
ein $C_6$-$C_{12}$-Aryl,
welches gegebenenfalls ein- oder mehrfach, gleich oder verschieden mit Halogen, Hydroxy, $C_1$-$C_6$-Alkyl, $C_1$-$C_6$-Acyl, $C_1$-$C_6$-Alkoxy, N-($C_1$-$C_6$-Alkyl)$_2$, NH-($C_1$-$C_6$-Alkyl) oder Cyano substituiert sein kann, oder
ein $C_5$-$C_{12}$-Heteroaryl,
welches gegebenenfalls ein- oder mehrfach, gleich oder verschieden mit Halogen, Amino, Hydroxy, Cyano, COOH, $C_1$-$C_6$-Alkyl, $C_1$-$C_6$-Acyl, $C_1$-$C_6$-Alkoxy, N-($C_1$-$C_6$-Alkyl)$_2$ oder NH-($C_1$-$C_6$-Alkyl) substituiert sein kann,
$R^5$, $R^6$ unabhängig voneinander Wasserstoff oder ein $C_1$-$C_6$-Alkyl, und
$X^2$ ein Stickstoff, wenn $X^1$ und $X^3$ eine CH-Gruppe sind, oder
$X^3$ ein Stickstoff, wenn $X^1$ und $X^2$ eine CH-Gruppe sind,

bedeuten,
sowie deren Stereoisomere, Diastereomere, Enantiomere und Salze.

**7.** Verbindungen gemäß den Ansprüchen 1 - 6, wobei

A ein Phenyl-, Thienyl- oder Pyridinyl-Rest, der gegebenenfalls ein- oder zweifach mit $R^1$ und/oder $R^2$ substituiert sein kann,
$R^1$ ein Wasserstoff, Halogen, $C_1$-$C_6$-Alkoxy
ein $C_1$-$C_6$-Alkyl,
welches gegebenenfalls ein- oder mehrfach, gleich oder verschieden substituiert sein kann mit $C_1$-$C_6$-Alkoxy, Hydroxy, Cyano, N-($C_1$-$C_6$-Alkyl)$_2$, Phenyl, ein O-$C_6$-$C_{12}$-Aryl oder ein O-$C_5$-$C_{12}$-Heteroaryl, oder
ein Phenyl,
welches gegebenenfalls ein- oder zweifach, gleich oder verschieden substituiert sein kann mit Halogen, $C_1$-$C_6$-Alkyl, $C_3$-$C_6$-Cycloalkyl, $C_1$-$C_6$-Acyl, $C_1$-$C_6$-Alkoxy, $C_6$-$C_{12}$-Aryl, $C_5$- $C_{12}$-Heteroaryl, Hydroxy, $CH_2$-OH, Cyano, $CH_2$-CN, Amino, $CO_2$-($C_1$-$C_6$-Alkyl), N-($C_1$-$C_6$-Alkyl)$_2$, $NHSO_2CH_3$, $SO_2NH_2$, $SO_2NH$($C_1$-$C_6$-Alkyl), $SO_2N$($C_1$-$C_6$-Alkyl)$_2$, COOH, CO-$NH_2$, CO-NH-($C_1$-$C_6$-Alkyl), CO-N($C_1$-$C_6$-Alkyl)$_2$, CO-NH($C_5$-$C_{12}$- Heteroaryl), NH-CO($C_1$-$C_6$-Alkyl), $CH_2$-NH-CO($C_1$-$C_6$-Alkyl), NH-CO($C_5$-$C_{12}$-Heteroaryl) oder ein -S(O)$_r$-$CH_3$, wobei r für 0 - 2 steht, oder
zwei benachbarte Positionen durch -O-$CH_2$-O- oder -O-C($CH_3$)$_2$- O- substituiert sein können, oder
ein Thienyl- oder Pyridinyl Rest,
welcher gegebenenfalls ein- oder zweifach, gleich oder verschieden substituiert sein kann mit Halogen, mit $C_1$-$C_6$- Alkyl, $C_3$-$C_6$-Cycloalkyl, $C_1$-$C_6$-Acyl, $C_1$-$C_6$-Alkoxy, $C_6$-$C_{12}$- Aryl, $C_5$-$C_{12}$-Heteroaryl, Hydroxy, $CH_2$-OH, Cyano, $CH_2$-CN, Amino, $CO_2$-($C_1$-$C_6$-Alkyl), N-($C_1$-$C_6$-Alkyl)$_2$, $NHSO_2CH_3$, $SO_2NH_2$, $SO_2NH$($C_1$-$C_6$-Alkyl), $SO_2N$($C_1$-$C_6$-Alkyl)$_2$, COOH, CO-$NH_2$, CO-NH($C_1$-$C_6$-Alkyl), CO-N($C_1$-$C_6$-Alkyl)$_2$, CO- NH($C_5$-$C_{12}$-Heteroaryl), NH-CO($C_1$-$C_6$-Alkyl), $CH_2$-NH- CO($C_1$-$C_6$-Alkyl), NH-CO($C_5$-$C_{12}$-Heteroaryl) oder ein -S(O)$_r$- $CH_3$, wobei r für 0 - 2 steht, oder
ein $C_3$-$C_6$-Cycloalkyl,
welches gegebenenfalls ein- oder mehrfach, gleich oder verschieden mit Halogen, mit $C_1$-$C_6$-Alkyl, Hydroxy, Cyano, $CO_2$-($C_1$-$C_6$-Alkyl), $C_1$-$C_6$-Acyl, N-($C_1$-$C_6$-Alkyl)$_2$, COOH, CO- $NH_2$, CO-NH($C_1$-$C_6$-Alkyl), CO-N($C_1$-$C_6$-Alkyl)$_2$ oder $C_1$-$C_6$- Alkoxy substituiert sein kann,
$R^2$ ein Wasserstoff,
$R^3$ ein $C_6$-$C_{12}$-Aryl,
welches gegebenenfalls ein- oder zweifach, gleich oder verschieden substituiert sein kann mit Halogen, $C_1$-$C_6$-Alkyl, $C_1$-$C_6$-Alkoxy, Hydroxy, $C_1$-$C_6$-Alkylen-OH, $C_1$-$C_6$-Alkylen- $NR^5R^6$, Cyano, $CO_2$-($C_1$-$C_6$-Alkyl), N-($C_1$-$C_6$-Alkyl)$_2$, CO-NH- ($C_1$-$C_6$-Alkyl), CO-NH-($C_1$-$C_6$-Alkylen-OH), CO-NH-($C_1$-$C_6$- Alkylen-$NR^5R^6$), CO-N($C_1$-$C_6$-Alkyl)$_2$ oder $C_1$-$C_6$-Acyl, welches gegebenenfalls ein- oder mehrfach substituiert ist,
oder
mit zwei benachbarten Positionen durch -O-$CH_2$-O-, -O- $CH_2$-$CH_2$-O-, oder -O-C($CH_3$)$_2$-O- substituiert sein kann,
oder
ein $C_5$-$C_{12}$-Heteroaryl,
welches gegebenenfalls ein- oder mehrfach, gleich oder verschieden substituiert sein kann mit Halogen, $C_1$-$C_6$-Alkyl, $C_1$-$C_6$-Acyl, $C_1$-$C_6$-Alkoxy, Hydroxy, Cyano, $CO_2$-($C_1$-$C_6$- Alkyl), N-($C_1$-$C_6$-Alkyl)$_2$, CO-NH-($C_1$-$C_6$-Alkylen-OH), CO- NH-($C_1$-$C_6$-Alkylen-$NR^5R^6$), CO-NH($C_1$-$C_6$-Alkyl) oder CO- N($C_1$-$C_6$-Alkyl)$_2$,

$R^4$ ein Wasserstoff,
ein $C_1$-$C_6$-Alkyl,
welches gegebenenfalls ein- oder mehrfach, gleich oder verschieden substituiert sein kann mit einer -S(O)$_p$-$C_1$-$C_6$- Alkylgruppe oder -S(O)$_p$-$C_3$-$C_6$-Cycloalkylgruppe, wobei p für 0 - 2 steht, Hydroxy, Cyano, COOH, CO-$NH_2$, $SO_2NH_2$, $SO_2$NH-($C_1$-$C_6$-Alkyl), $C_3$-$C_6$-Cycloalkyl, $C_1$-$C_6$-Acyl, $C_1$-$C_6$- Alkoxy, $CO_2$-($C_1$-$C_6$-Alkyl), N-($C_1$-$C_6$-Alkyl)$_2$, NH-($C_1$-$C_6$- Alkyl), NH-($C_3$-$C_6$-Cycloalkyl), CO-NH($C_1$-$C_6$-Alkyl) oder mit CO-N($C_1$-$C_6$-Alkyl)$_2$,
$C_6$-$C_{12}$-Aryl,
welches gegebenenfalls mehrfach substituiert ist, $C_5$-$C_{12}$-Heteroaryl,
welches gegebenenfalls mehrfach substituiert ist,
oder
ein $C_3$-$C_6$-Cycloalkyl,
welches gegebenenfalls ein- oder mehrfach, gleich oder verschieden mit Hydroxy, $CH_2$-OH, Cyano, COOH, CO-$NH_2$, $SO_2NH_2$, $C_1$-$C_6$-Alkyl, $C_1$-$C_6$-Acyl oder $C_1$-$C_6$-Alkoxy substituiert sein kann, oder
ein $C_6$-$C_{12}$-Aryl,
welches gegebenenfalls ein- oder mehrfach, gleich oder verschieden mit Halogen, Hydroxy, $C_1$-$C_6$-Alkyl, $C_1$-$C_6$-Acyl, $C_1$-$C_6$-Alkoxy, N-($C_1$-$C_6$-Alkyl)$_2$, NH-($C_1$-$C_6$-Alkyl) oder Cyano substituiert sein kann, oder
ein $C_5$-$C_{12}$-Heteroaryl,
welches gegebenenfalls ein- oder mehrfach, gleich oder verschieden mit Halogen, Amino, Hydroxy, Cyano, COOH, $C_1$-$C_6$-Alkyl, $C_1$-$C_6$-Acyl, $C_1$-$C_6$-Alkoxy, N-($C_1$-$C_6$-Alkyl)$_2$ oder NH-($C_1$-$C_6$-Alkyl) substituiert sein kann,
$R^5$, $R^6$ unabhängig voneinander Wasserstoff oder ein $C_1$-$C_6$-Alkyl, und
$X^2$ ein Stickstoff, wenn $X^1$ und $X^3$ eine CH-Gruppe sind, oder
$X^3$ ein Stickstoff, wenn $X^1$ und $X^2$ eine CH-Gruppe sind,

bedeuten,
sowie deren Stereoisomere, Diastereomere, Enantiomere und Salze.

**8.** Verbindungen gemäß den Ansprüchen 1-7, wobei

A ein Phenyl-, Thienyl- oder Pyridinyl-Rest, der gegebenenfalls ein- oder zweifach mit $R^1$ und/oder $R^2$ substituiert sein kann,
$R^1$ ein Wasserstoff, Halogen, $C_1$-$C_6$-Alkoxy
ein $C_1$-$C_6$-Alkyl,
welches gegebenenfalls ein- oder mehrfach, gleich oder verschieden substituiert sein kann mit $C_1$-$C_6$-Alkoxy, Hydroxy, Cyano, N-($C_1$-$C_6$-Alkyl)$_2$, Phenyl,
ein O-$C_6$-$C_{12}$-Aryl oder ein O-$C_5$-$C_{12}$-Heteroaryl, oder ein Phenyl, welches gegebenenfalls ein- oder zweifach, gleich oder verschieden substituiert sein kann mit Halogen, $C_1$-$C_6$-Alkyl, $C_3$-$C_6$-Cycloalkyl, $C_1$-$C_6$-Acyl, $C_1$-$C_6$-Alkoxy, $C_6$-$C_{12}$-Aryl, $C_5$-$C_{12}$-Heteroaryl, Hydroxy, $CH_2$-OH, Cyano, $CH_2$-CN, Amino, $CO_2$-($C_1$-$C_6$-Alkyl), N-($C_1$-$C_6$-Alkyl)$_2$, $NHSO_2CH_3$, $SO_2NH_2$, $SO_2$NH($C_1$-$C_6$-Alkyl), $SO_2$N($C_1$-$C_6$-Alkyl)$_2$, COOH, CO-$NH_2$, CO-NH-($C_1$-$C_6$-Alkyl), CO-N($C_1$-$C_6$-Alkyl)$_2$, CO-NH($C_5$-$C_{12}$- Heteroaryl), NH-CO($C_1$-$C_6$-Alkyl), $CH_2$-NH-CO($C_1$-$C_6$-Alkyl), NH-CO($C_5$-$C_{12}$-Heteroaryl) oder ein -S(O)$_r$-$CH_3$, wobei r für 0 - 2 steht, oder
zwei benachbarte Positionen durch -O-$CH_2$-O- oder -O-C($CH_3$)$_2$- O- substituiert sein können, oder
ein Thienyl- oder Pyridinyl- Rest,
welcher gegebenenfalls ein- oder zweifach, gleich oder verschieden substituiert sein kann mit Halogen, mit $C_1$-$C_6$- Alkyl, $C_3$-$C_6$-Cycloalkyl, $C_1$-$C_6$-Acyl, $C_1$-$C_6$-Alkoxy, $C_6$-$C_{12}$- Aryl, $C_5$-$C_{12}$-Heteroaryl, Hydroxy, $CH_2$-OH, Cyano, $CH_2$-CN, Amino, $CO_2$-($C_1$-$C_6$-Alkyl), N-($C_1$-$C_6$-Alkyl)$_2$, $NHSO_2CH_3$, $SO_2NH_2$, $SO_2$NH($C_1$-$C_6$-Alkyl), $SO_2$N($C_1$-$C_6$-Alkyl)$_2$, COOH, CO-$NH_2$, CO-NH($C_1$-$C_6$-Alkyl), CO-N($C_1$-$C_6$-Alkyl)$_2$, CO- NH($C_5$-$C_{12}$-Heteroaryl), NH-CO($C_1$-$C_6$-Alkyl), $CH_2$-NH- CO($C_1$-$C_6$-Alkyl), NH-CO($C_5$-$C_{12}$-Heteroaryl) oder ein -S(O)$_r$- $CH_3$, wobei r für 0 - 2 steht, oder
ein $C_3$-$C_6$-Cycloalkyl,
welches gegebenenfalls ein- oder mehrfach, gleich oder verschieden mit Halogen, mit $C_1$-$C_6$-Alkyl, Hydroxy, Cyano, $CO_2$-($C_1$-$C_6$-Alkyl), $C_1$-$C_6$-Acyl, N-($C_1$-$C_6$-Alkyl)$_2$, COOH, CO- $NH_2$, CO-NH($C_1$-$C_6$-Alkyl), CO-N($C_1$-$C_6$-Alkyl)$_2$ oder $C_1$-$C_6$- Alkoxy substituiert sein kann,
$R^2$ ein Wasserstoff,
$R^3$ ein Phenyl, welches gegebenenfalls ein- oder zweifach, gleich oder verschieden substituiert sein kann mit Halogen, $C_1$-$C_6$-Alkyl, $C_1$-$C_6$-Alkoxy, Hydroxy, $C_1$-$C_6$-Alkylen-OH, $C_1$-$C_6$-Alkylen-$NR^5R^6$, Cyano, $CO_2$-($C_1$-$C_6$-Alkyl), N-($C_1$-$C_6$-Alkyl)$_2$, CO-NH- ($C_1$-$C_6$-Alkyl), CO-NH-($C_1$-$C_6$-Alkylen-OH), CO-NH-($C_1$-$C_6$- Alkylen-$NR^5R^6$), CO-N($C_1$-$C_6$-Alkyl)$_2$ oder $C_1$-$C_6$-Acyl, welches gegebenenfalls ein- oder mehrfach substituiert ist,
oder

mit zwei benachbarten Positionen durch $-O-CH_2-O-$, $-O-CH_2-CH_2-O-$, oder $-O-C(CH_3)_2-O-$ substituiert sein kann, oder

ein Thiophenyl-, Furanyl-, oder Pyridinyl-Rest,

welcher gegebenenfalls ein- oder mehrfach, gleich oder verschieden substituiert sein kann mit Halogen, $C_1$-$C_6$-Alkyl, $C_1$-$C_6$-Acyl, $C_1$-$C_6$-Alkoxy, Hydroxy, Cyano, $CO_2$-($C_1$-$C_6$- Alkyl), N-($C_1$-$C_6$-Alkyl)$_2$, CO-NH-($C_1$-$C_6$-Alkylen-OH), CO- NH-($C_1$-$C_6$-Alkylen-$NR^5R^6$), CO-NH($C_1$-$C_6$-Alkyl) oder CO- N($C_1$-$C_6$-Alkyl)$_2$,

$R^4$ ein Wasserstoff,

ein $C_1$-$C_6$-Alkyl,

welches gegebenenfalls ein- oder mehrfach, gleich oder verschieden substituiert sein kann mit einer -S(O)$_p$-$C_1$-$C_6$- Alkylgruppe oder -S(O)$_p$-$C_3$-$C_6$-Cycloalkylgruppe, wobei p für 0 - 2 steht, Hydroxy, Cyano, COOH, CO-$NH_2$, $SO_2NH_2$, $SO_2NH$-($C_1$-$C_6$-Alkyl), $C_3$-$C_6$-Cycloalkyl, $C_1$-$C_6$-Acyl, $C_1$-$C_6$- Alkoxy, $CO_2$-($C_1$-$C_6$-Alkyl), N-($C_1$-$C_6$-Alkyl)$_2$, NH-($C_1$-$C_6$- Alkyl), NH-($C_3$-$C_6$-Cycloalkyl), CO-NH($C_1$-$C_6$-Alkyl) oder mit CO-N($C_1$-$C_6$-Alkyl)$_2$,

$C_6$-$C_{12}$-Aryl,

welches gegebenenfalls mehrfach substituiert ist, $C_5$-$C_{12}$-Heteroaryl,

welches gegebenenfalls mehrfach substituiert ist, oder

ein $C_3$-$C_6$-Cycloalkyl,

welches gegebenenfalls ein- oder mehrfach, gleich oder verschieden mit Hydroxy, $CH_2$-OH, Cyano, COOH, CO-$NH_2$, $SO_2NH_2$, $C_1$-$C_6$-Alkyl, $C_1$-$C_6$-Acyl oder $C_1$-$C_6$-Alkoxy substituiert sein kann, oder

ein $C_6$-$C_{12}$-Aryl,

welches gegebenenfalls ein- oder mehrfach, gleich oder verschieden mit Halogen, Hydroxy, $C_1$-$C_6$-Alkyl, $C_1$-$C_6$-Acyl, $C_1$-$C_6$-Alkoxy, N-($C_1$-$C_6$-Alkyl)$_2$, NH-($C_1$-$C_6$-Alkyl) oder Cyano substituiert sein kann, oder

ein $C_5$-$C_{12}$-Heteroaryl,

welches gegebenenfalls ein- oder mehrfach, gleich oder verschieden mit Halogen, Amino, Hydroxy, Cyano, COOH, $C_1$-$C_6$-Alkyl, $C_1$-C6-Acyl, $C_1$-$C_6$-Alkoxy, N-($C_1$-$C_6$-Alkyl)$_2$ oder NH-($C_1$-$C_6$-Alkyl) substituiert sein kann,

$R^5$, $R^6$ unabhängig voneinander Wasserstoff oder ein $C_1$-$C_6$-Alkyl, und

$X^2$ ein Stickstoff, wenn $X^1$ und $X^3$ eine CH-Gruppe sind, oder

$X^3$ ein Stickstoff, wenn $X^1$ und $X^2$ eine CH-Gruppe sind,

bedeuten,
sowie deren Stereoisomere, Diastereomere, Enantiomere und Salze.

**9.** Verbindungen gemäß den Ansprüchen 1-8, wobei

A ein Phenyl-, Thienyl- oder Pyridinyl-Rest, der gegebenenfalls ein- oder zweifach mit $R^1$ und/oder $R^2$ substituiert sein kann,

$R^1$ ein Wasserstoff, Halogen, $C_1$-$C_4$-Alkoxy ein $C_1$-$C_4$-Alkyl, welches gegebenenfalls ein- oder mehrfach, gleich oder verschieden substituiert sein kann mit $C_1$-$C_4$-Alkoxy, Hydroxy, Cyano, N-($C_1$-$C_4$-Alkyl)$_2$, Phenyl, ein O-$C_6$-$C_{12}$-Aryl oder ein O-$C_5$-$C_{12}$-Heteroaryl, oder

ein Phenyl,

welches gegebenenfalls ein- oder zweifach, gleich oder verschieden substituiert sein kann mit Halogen, $C_1$-$C_4$-Alkyl, $C_3$-$C_6$-Cycloalkyl, $C_1$-$C_4$-Acyl, $C_1$-$C_4$-Alkoxy, $C_6$-$C_{12}$-Aryl, $C_5$- $C_{12}$-Heteroaryl, Hydroxy, $CH_2$-OH, Cyano, $CH_2$-CN, Amino, $CO_2$-($C_1$-$C_4$-Alkyl), N-($C_1$-$C_4$-Alkyl)$_2$, $NHSO_2CH_3$, $SO_2NH_2$, $SO_2NH$($C_1$-$C_4$-Alkyl), $SO_2N$($C_1$-$C_4$-Alkyl)$_2$, COOH, CO-$NH_2$, CO-NH-($C_1$-$C_4$-Alkyl), CO-N($C_1$-$C_4$-Alkyl)$_2$, CO-NH($C_5$-$C_{12}$- Heteroaryl), NH-CO($C_1$-$C_4$-Alkyl), $CH_2$-NH-CO($C_1$-$C_4$-Alkyl), NH-CO($C_5$-$C_{12}$-Heteroaryl) oder ein -S(O)$_r$-$CH_3$, wobei r für 0 - 2 steht, oder

zwei benachbarte Positionen durch $-O-CH_2-O-$ oder $-O-C(CH_3)_2-O-$ substituiert sein können, oder

ein Thienyl- oder Pyridinyl- Rest,

welcher gegebenenfalls ein- oder zweifach, gleich oder verschieden substituiert sein kann mit Halogen, mit $C_1$-$C_4$- Alkyl, $C_3$-$C_6$-Cycloalkyl, $C_1$-$C_4$-Acyl, $C_1$-$C_4$-Alkoxy, $C_6$-$C_{12}$- Aryl, $C_5$-$C_{12}$-Heteroaryl, Hydroxy, $CH_2$-OH, Cyano, $CH_2$-CN, Amino, $CO_2$-($C_1$-$C_4$-Alkyl), N-($C_1$-$C_4$-Alkyl)$_2$, $NHSO_2CH_3$, $SO_2NH_2$, $SO_2NH$($C_1$-$C_4$-Alkyl), $SO_2N$($C_1$-$C_4$-Alkyl)$_2$, COOH, CO-$NH_2$, CO-NH($C_1$-$C_4$-Alkyl), CO-N($C_1$-$C_4$-Alkyl)$_2$, CO- NH($C_5$-$C_{12}$-Heteroaryl), NH-CO($C_1$-$C_4$-Alkyl), $CH_2$-NH- CO($C_1$-$C_4$-Alkyl), NH-CO($C_5$-$C_{12}$-Heteroaryl) oder ein -S(O)$_r$- $CH_3$, wobei r für 0 - 2 steht, oder

ein $C_3$-$C_6$-Cycloalkyl,

welches gegebenenfalls ein- oder mehrfach, gleich oder verschieden mit Halogen, mit $C_1$-$C_4$-Alkyl, Hydroxy, Cyano, $CO_2$-($C_1$-$C_4$-Alkyl), $C_1$-$C_4$-Acyl, N-($C_1$-$C_4$-Alkyl)$_2$, COOH, CO- $NH_2$, CO-NH($C_1$-$C_4$-Alkyl), CO-N($C_1$-$C_4$-Alkyl)$_2$ oder $C_1$-$C_4$- Alkoxy substituiert sein kann,

$R^2$ ein Wasserstoff,

$R^3$ ein Phenyl,
welches gegebenenfalls ein- oder zweifach, gleich oder verschieden substituiert sein kann mit Halogen, $C_1$-$C_4$-Alkyl, $C_1$-$C_4$-Alkoxy, Hydroxy, $C_1$-$C_4$-Alkylen-OH, $C_1$-$C_4$-Alkylen- $NR^5R^6$, Cyano, $CO_2$-($C_1$-$C_4$-Alkyl), N-($C_1$-$C_4$-Alkyl)$_2$, CO-NH- ($C_1$-$C_4$-Alkyl), CO-NH-($C_1$-$C_4$-Alkylen-OH), CO-NH-($C_1$-$C_4$- Alkylen-$NR^5R^6$), CO-N($C_1$-$C_4$-Alkyl)$_2$ oder $C_1$-$C_4$-Acyl, welches gegebenenfalls ein- oder mehrfach substituiert ist,
oder
mit zwei benachbarten Positionen durch -O-$CH_2$-O-, -O- $CH_2$-$CH_2$-O-, oder -O-C($CH_3$)$_2$-O- substituiert sein kann, oder
ein Thiophenyl-, Furanyl-, oder Pyridinyl-Rest,
welcher gegebenenfalls ein- oder mehrfach, gleich oder verschieden substituiert sein kann mit Halogen, $C_1$-$C_4$-Alkyl, $C_1$-$C_4$-Acyl, $C_1$-$C_4$-Alkoxy, Hydroxy, Cyano, $CO_2$-($C_1$-$C_4$- Alkyl), N-($C_1$-$C_4$-Alkyl)$_2$, CO-NH-($C_1$-$C_4$-Alkylen-OH), CO- NH-($C_1$-$C_4$-Alkylen-$NR^5R^6$), CO-NH($C_1$-$C_4$-Alkyl) oder CO- N($C_1$-$C_4$-Alkyl)$_2$,
$R^4$ ein Wasserstoff, ein $C_1$-$C_4$-Alkyl, welches gegebenenfalls ein- oder mehrfach, gleich oder verschieden substituiert sein kann mit einer -S(O)$_p$-$C_1$-$C_4$- Alkylgruppe oder -S(O)$_p$-$C_3$-$C_6$-Cycloalkylgruppe, wobei p für 0 - 2 steht, Hydroxy, Cyano, COOH, CO-$NH_2$, $SO_2NH_2$, $SO_2$NH-($C_1$-$C_4$-Alkyl), $C_3$-$C_6$-Cycloalkyl, $C_1$-$C_4$-Acyl, $C_1$-$C_4$- Alkoxy, $CO_2$-($C_1$-$C_4$-Alkyl), N-($C_1$-$C_4$-Alkyl)$_2$, NH-($C_1$-$C_4$- Alkyl), NH-($C_3$-$C_6$-Cycloalkyl), CO-NH ($C_1$-$C_4$-Alkyl) oder mit CO-N($C_1$-$C_4$-Alkyl)$_2$, $C_6$-$C_{12}$-Aryl,
welches gegebenenfalls mehrfach substituiert ist, $C_5$-$C_{12}$-Heteroaryl,
welches gegebenenfalls mehrfach substituiert ist,
oder
ein $C_3$-$C_6$-Cycloalkyl,
welches gegebenenfalls ein- oder mehrfach, gleich oder verschieden mit Hydroxy, $CH_2$-OH, Cyano, COOH, CO-$NH_2$, $SO_2NH_2$, $C_1$-$C_4$-Alkyl, $C_1$-$C_4$-Acyl oder $C_1$-$C_4$-Alkoxy substituiert sein kann, oder
ein $C_6$-$C_{12}$-Aryl,
welches gegebenenfalls ein- oder mehrfach, gleich oder verschieden mit Halogen, Hydroxy, $C_1$-$C_4$-Alkyl, $C_1$-$C_4$-Acyl, $C_1$-$C_4$-Alkoxy, N-($C_1$-$C_4$-Alkyl)$_2$, NH-($C_1$-$C_4$-Alkyl) oder Cyano substituiert sein kann, oder
ein $C_5$-$C_{12}$-Heteroaryl,
welches gegebenenfalls ein- oder mehrfach, gleich oder verschieden mit Halogen, Amino, Hydroxy, Cyano, COOH, $C_1$-$C_4$-Alkyl, $C_1$-$C_4$-Acyl, $C_1$-$C_4$-Alkoxy, N-($C_1$-$C_4$-Alkyl)$_2$ oder NH-($C_1$-$C_4$-Alkyl) substituiert sein kann,
$R^5$, $R^6$ unabhängig voneinander Wasserstoff oder ein $C_1$-$C_4$-Alkyl, und
$X^2$ ein Stickstoff, wenn $X^1$ und $X^3$ eine CH-Gruppe sind, oder
$X^3$ ein Stickstoff, wenn $X^1$ und $X^2$ eine CH-Gruppe sind,

bedeuten,
sowie deren Stereoisomere, Diastereomere, Enantiomere und Salze.

**10.** Verbindungen gemäß den Ansprüchen 1-9, ausgewählt aus einer Gruppe, die die folgenden Verbindungen enthält:

1. 5-(4-tert-Butyl-phenylsulfonylamino)-3-phenyl-1 H-pyrrolo[3,2-b]pyridin-2-carbonsäure (tetrahydro-pyran-4-yl)-amid
2. 5-(4-tert-Butyl-phenylsulfonylamino)-3-phenyl-1 H-pyrrolo[3,2-b]pyridin-2-carbonsäure (2-morpholin-4-yl-ethyl)-amid
3. 5-(4-tert-Butyl-phenylsulfonylamino)-3-phenyl-1 H-pyrrolo[2,3-c]pyridin-2-carbonsäure (2-morpholin-4-yl-ethyl)-amid
4. 5-(4-tert-Butyl-phenylsulfonylamino)-3-phenyl-1 H-pyrrolo[2,3-b]pyridin-2-carbonsäure (2-morpholin-4-yl-ethyl)-amid
5. (±)-5-(4-tert-Butyl-phenylsulfonylamino)-3-phenyl-1H-pyrrolo[2,3-b]pyridin-2-carbonsäure (2-hydroxy-propyl)-amid
6. 5-(4-tert-Butyl-phenylsulfonylamino)-3-phenyl-1 H-pyrrolo[2,3-b]pyridin-2-carbonsäure (tetrahydro-pyran-4-yl)-amid
7. (±)-5-(4-tert-Butyl-phenylsulfonylamino)-3-phenyl-1H-pyrrolo[2,3-b]pyridin-2-carbonsäure (2-hydroxy-1-methyl-ethyl)-amid
8. (1 S,2R)-5-(4-tert-Butyl-phenylsulfonylamino)-3-phenyl-1H-pyrrolo[2,3-b]pyridin-2-carbonsäure (2-hydroxy-cyclopentyl)-amid
9. 3-Phenyl-5-(4-trifluormethyl-phenylsulfonylamino)-1 H-pyrrolo[2,3-b]pyridin-2-carbonsäure (2-morpholin-4-yl-ethyl)-amid
10. (1 S,2R)-5-(Biphenyl-4-sulfonylamino)-3-phenyl-1H-pyrrolo[2,3-b]pyridin-2-carbonsäure (2-hydroxy-cyclopentyl)-amid

11. 3-Phenyl-5-(4-trifluormethoxy-phenylsulfonylamino)-1 H-pyrrolo[2,3-b]pyridin-2-carbonsäure (2-morpholin-4-yl-ethyl)-amid
12. 5-(Biphenyl-4-sulfonylamino)-3-phenyl-1 H-pyrrolo[2,3-b]pyridin-2-carbonsäure (2-morpholin-4-yl-ethyl)-amid
13. (1 S,2R)-3-Phenyl-5-(4-trifluormethyl-phenylsulfonylamino)-1H-pyrrolo[2,3-b]pyridin-2-carbonsäure (2-hydroxy-cyclopentyl)-amid
14. 5-(4-tert-Butyl-phenylsulfonylamino)-3-phenyl-1 H-pyrrolo[2,3-b]pyridin-2-carbonsäure [2-(morpholin-4-sulfonyl)-ethyl]-amid
15. 5-(4-tert-Butyl-phenylsulfonylamino)-3-phenyl-1 H-pyrrolo[2,3-b]pyridin-2-carbonsäure pyridin-4-ylamid
16. 5-(4-tert-Butyl-phenylsulfonylamino)-3-phenyl-1 H-pyrrolo[2,3-b]pyridin-2-carbonsäure thiazol-2-ylamid
17. 5-(4-tert-Butyl-phenylsulfonylamino)-3-phenyl-1 H-pyrrolo[2,3-b]pyridin-2-carbonsäure (pyridin-3-ylmethyl)-amid
18. (±)-5-(4-tert-Butyl-phenylsulfonylamino)-3-phenyl-1H-pyrrolo[2,3-b]pyridin-2-carbonsäure (tetrahydrofuran-2-ylmethyl)-amid
19. 5-(4-tert-Butyl-phenylsulfonylamino)-3-phenyl-1 H-pyrrolo[2,3-b]pyridin-2-carbonsäure ethylamid
20. 5-(4-tert-Butyl-phenylsulfonylamino)-3-phenyl-1 H-pyrrolo[2,3-b]pyridin-2-carbonsäure [2-(2-oxo-imidazolidin-1-yl)-ethyl]-amid
21. (1S,2R)-3-Phenyl-5-(4-trifluormethoxy-phenylsulfonylamino)-1H-pyrrolo[2,3-b]pyridin-2-carbonsäure (2-hydroxy-cyclopentyl)-amid
22. 5-(4-tert-Butyl-phenylsulfonylamino)-3-phenyl-1H-pyrrolo[2,3-b]pyridin-2-carbonsäureamid
23. 5-(4-tert-Butyl-phenylsulfonylamino)-3-phenyl-1 H-pyrrolo[2,3-b]pyridin-2-carbonsäure (2-sulfamoyl-ethyl)-amid
24. 5-(Biphenyl-4-sulfonylamino)-3-phenyl-1 H-pyrrolo[2,3-b]pyridine-2-carbonsäureamid
25. (1 S,2R)-3-Benzo[1,3]dioxol-5-yl-5-(4-trifluormethoxy-phenylsulfonylamino)-1 H-pyrrolo[2,3-b]pyridin-2-carbonsäure (2-hydroxy-cyclopentyl)-amid
26. (1 S,2R)-3-Thiophen-2-yl-5-(4-trifluormethoxy-phenylsulfonylamino)-1H-pyrrolo[2,3-b]pyridin-2-carbonsäure (2-hydroxy-cyclopentyl)-amid
27. (1 S,2R)-3-Thiophen-3-yl-5-(4-trifluormethoxy-phenylsulfonylamino)-1H-pyrrolo[2,3-b]pyridin-2-carbonsäure (2-hydroxy-cyclopentyl)-amid
28. (1 S,2R)-3-Furan-2-yl-5-(4-trifluormethoxy-phenylsulfonylamino)-1H-pyrrolo[2,3-b]pyridin-2-carbonsäure (2-hydroxy-cyclopentyl)-amid
29. (1 S,2R)-3-Furan-3-yl-5-(4-trifluormethoxy-phenylsulfonylamino)-1H-pyrrolo[2,3-b]pyridin-2-carbonsäure (2-hydroxy-cyclopentyl)-amid
30. (1 S,2R)-3-Benzo[1,3]dioxol-5-yl-5-(4-tert-butyl-phenylsulfonylamino)-1H-pyrrolo[2,3-b]pyridin-2-carbonsäure (2-hydroxy-cyclopentyl)-amid
31. (1 S,2R)-3-Thiophen-2-yl-5-(4-tert-butyl-phenylsulfonylamino)-1H-pyrrolo[2,3-b]pyridin-2-carbonsäure (2-hydroxy-cyclopentyl)-amid
32. (1S,2R)-3-Thiophen-3-yl-5-(4-tert-butyl-phenylsulfonylamino)-1H-pyrrolo[2,3-b]pyridin-2-carbonsäure (2-hydroxy-cyclopentyl)-amid
33. (1 S,2R)-3-Furan-2-yl-5-(4-tert-butyl-phenylsulfonylamino)-1H-pyrrolo[2,3-b]pyridin-2-carbonsäure (2-hydroxy-cyclopentyl)-amid
34. (1 S,2R)-3-Furan-3-yl-5-(4-tert-butyl-phenylsulfonylamino)-1H-pyrrolo[2,3-b]pyridin-2-carbonsäure (2-hydroxy-cyclopentyl)-amid
35. (±)-3-Thiophen-3-yl-5-(4-trifluormethoxy-phenylsulfonylamino)-1H-pyrrolo[2,3-b]pyridin-2- carbonsäure (tetrahydro-furan-2-ylmethyl)-amid
36. (±)-3-Thiophen-2-yl-5-(4-trifluormethoxy-phenylsulfonylamino)-1H-pyrrolo[2,3-b]pyridin-2- carbonsäure (tetrahydro-furan-2-ylmethyl)-amid
37. (±)-3-Thiophen-3-yl-5-(4-tert-butyl-phenylsulfonylamino)-1H-pyrrolo[2,3-b]pyridin-2- carbonsäure (tetrahydro-furan-2-ylmethyl)-amid
38. (±)-3-Thiophen-2-yl-5-(4-tert-butyl-phenylsulfonylamino)-1H-pyrrolo[2,3-b]pyridin-2- carbonsäure (tetrahydro-furan-2-ylmethyl)-amid

**11.** Verbindungen der allgemeinen Formel 1 gemäß den Ansprüchen 1-10 als Arzneimittel.

**12.** Arzneimittel gemäß Anspruch 11, in denen die Verbindung der allgemeinen Formel 1 in einer wirksamen Dosis enthalten ist.

**13.** Arzneimittel gemäß Anspruch 11 und 12 zur Reduktion oder Inhibition der Aktivität der löslichen Adenylatzyklase.

**14.** Arzneimittel gemäß Anspruch 11 und 12 für die Behandlung von Erkrankungen.

**15.** Arzneimittel gemäß Anspruch 11 und 12, wobei die Erkrankungen verursacht werden durch Störungen im cAMP Stoffwechsel.

**16.** Verwendung der Verbindungen der allgemeinen Formel I in geeigneten Formulierungs- und Trägerstoffen für die Kontrazeption.

**17.** Arzneimittel gemäß den Ansprüchen 11 - 15 mit geeigneten Formulierungs-und Trägerstoffen.

**18.** Verwendung der Verbindungen der allgemeinen Formel 1 gemäß Anspruch 1 - 10 für die Herstellung eines pharmazeutischen Präparates für die enterale, parenterale, vaginale und orale Applikation.

**19.** Empfängnisverhütendes Suppositorium, enthaltend die Verbindungen gemäß den Ansprüchen 1 - 10.

**20.** Suppositorium gemäß Anspruch 19 für die weibliche Fertilitätskontrolle.

**21.** Verfahren zur Herstellung der Verbindungen der allgemeinen Formel (I), **dadurch gekennzeichnet, dass** eine Verbindung der Formel II,

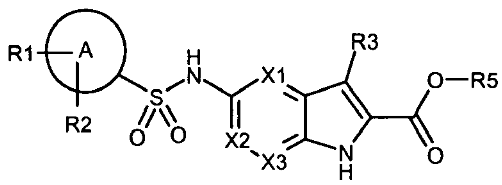

(II)

worin $R^1$, $R^2$, $R^3$, $X^1$, $X^2$ und $X^3$ die oben angegebenen Bedeutungen haben und $R^5$ ein Wasserstoff oder ein $C_1$-$C_6$-Akylrest sein kann, wobei ein Wasserstoff, ein Methyl- oder Ethyl-Rest bevorzugt ist, mit einem Amin der allgemeinen Formel III

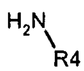

(III)

worin $R^4$ die oben angegebene Bedeutung aufweist, umsetzt und/oder gegebenenfalls benötigte Schutzgruppen anschließend gespalten und/oder gegebenenfalls vorhandene Doppelbindungen hydriert werden und zu den Verbindungen der allgemeinen Formel (I) umgesetzt werden.

**22.** Zwischenprodukte der allgemeinen Formel (II)

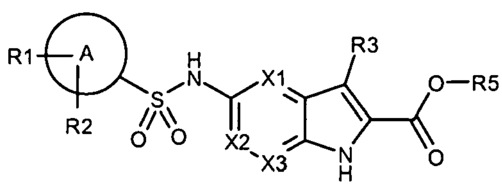

(II)

worin $R^1$, $R^2$, $R^3$, $X^1$, $X^2$ und $X^3$ die oben angegebenen Bedeutungen haben und $R^5$ ein Wasserstoff oder ein $C_1$-$C_6$-Akylrest sein kann, bevorzugt ist Wasserstoff, der Methyl- oder der Ethyl-Rest.

**23.** Verwendung der Zwischenverbindungen der allgemeinen Formel (II) zur Herstellung der Verbindungen der allgemeinen Formel (I).

Europäisches Patentamt

# EUROPÄISCHER RECHERCHENBERICHT

Nummer der Anmeldung
EP 07 07 5764

| EINSCHLÄGIGE DOKUMENTE | | | |
|---|---|---|---|
| Kategorie | Kennzeichnung des Dokuments mit Angabe, soweit erforderlich, der maßgeblichen Teile | Betrifft Anspruch | KLASSIFIKATION DER ANMELDUNG (IPC) |
| A,D | WO 2006/032541 A (SCHERING AG [DE]) 30. März 2006 (2006-03-30) Zusammenfassung; Ansprüche; Beispiele. ----- | 1-23 | INV. C07D471/04 A61K31/437 A61P15/16 |
| A | WO 2006/131398 A (SCHERING AG [DE]; BUCHMANN BERND [DE]; NGUYEN DUY [DE]; FRITSCH MARTIN) 14. Dezember 2006 (2006-12-14) Zusammenfassung; Ansprüche; Beispiele. ----- | 1-23 | |
| E | WO 2007/107384 A (BAYER SCHERING PHARMA AG [DE]; BUCHMANN BERND [DE]; KOSEMUND DIRK [DE]) 27. September 2007 (2007-09-27) Zusammenfassung; Ansprüche; Beispiele. ----- | 1-23 | |
| E | WO 2007/107385 A (BAYER SCHERING PHARMA AG [DE]; BUCHMANN BERND [DE]; KOSEMUND DIRK [DE]) 27. September 2007 (2007-09-27) Zusammenfassung; Ansprüche; Beispiele. ----- | 1-23 | |
| | | | RECHERCHIERTE SACHGEBIETE (IPC) |
| | | | C07D A61K A61P |

Der vorliegende Recherchenbericht wurde für alle Patentansprüche erstellt

| Recherchenort | Abschlußdatum der Recherche | Prüfer |
|---|---|---|
| München | 4. Februar 2008 | Weisbrod, Thomas |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument
.......................................................
& : Mitglied der gleichen Patentfamilie, übereinstimmendes Dokument

2

EPO FORM 1503 03.82 (P04C03)

**ANHANG ZUM EUROPÄISCHEN RECHERCHENBERICHT ÜBER DIE EUROPÄISCHE PATENTANMELDUNG NR.** EP 07 07 5764

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

04-02-2008

| Im Recherchenbericht angeführtes Patentdokument | Datum der Veröffentlichung | Mitglied(er) der Patentfamilie | Datum der Veröffentlichung |
|---|---|---|---|
| WO 2006032541 A | 30-03-2006 | DE 102004047272 A1 | 06-04-2006 |
| | | US 2006074084 A1 | 06-04-2006 |
| WO 2006131398 A | 14-12-2006 | AR 054378 A1 | 20-06-2007 |
| | | AU 2006256857 A1 | 14-12-2006 |
| | | DE 102005027274 A1 | 14-12-2006 |
| | | UY 29583 A1 | 31-01-2007 |
| WO 2007107384 A | 27-09-2007 | DE 102006014319 A1 | 27-09-2007 |
| | | US 2007259872 A1 | 08-11-2007 |
| | | UY 30237 A1 | 31-10-2007 |
| WO 2007107385 A | 27-09-2007 | DE 102006014320 A1 | 27-09-2007 |
| | | UY 30236 A1 | 31-10-2007 |

EPO FORM P0461

Für nähere Einzelheiten zu diesem Anhang : siehe Amtsblatt des Europäischen Patentamts, Nr.12/82

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- WO 0185753 A
- US 6544768 B
- WO 0121829 A, Conti
- WO 0220745 A
- WO 06032541 A


### In der Beschreibung aufgeführte Nicht-Patentliteratur


- **LEVIN ; BUCK.** *Proc. Natl. Acad. Sci. USA,* 1999, vol. 96 (1), 79-84
- **T. BRAUN.** *Proc Soc Exp Biol Med,* 1990, vol. 194 (1), 58ff
- **KL OLGIATI.** *Arch Biochem Biophys,* 1984, vol. 231 (2), 411 ff
- **MA BROWN ; ER CASILLAS.** *J Androl,* 1984, vol. 5, 361ff
- **JH ZIPPIN et al.** *J Cell Biol,* 2004, vol. 164 (4), 527ff
- **T. BRAUN et al.** *PNAS,* 1975, vol. 73, 1097ff
- **N. OKAMURA et al.** *J. Biol. Chem,* 1985, vol. 260 (17), 9699ff
- **J. BUCK et al.** *PNAS,* vol. 96, 79ff
- **Y. CHEN et al.** *Science,* 2000, vol. 289, 625ff
- **ML SINCLAIR et al.** *Mol Reprod Develop,* 2000, vol. 56, 6ff
- **N OKAMURA et al.** *J. Biol. Chem,* 1985, vol. 260 (17), 9699ff
- **J. BUCK et al.** *PNAS,* 1999, vol. 96, 79ff
- **PE VISCONTI ; GS KOPF.** *Biol Reprod,* 1998, vol. 59, 1ff
- **E DE LAMIRANDE et al.** *Mol Hum Reprod,* 1997, vol. 3 (3), 175ff
- **G ESPOSITO et al.** *PNAS,* 2004, vol. 101 (9), 2993ff
- **JH ZIPPIN et al.** *FASEB,* 2003, vol. 17, 82ff
- **J. W. CARVER-WARD.** *Human Reproduction,* 1996, vol. 11 (9), 1923 ff
- **O. J. D'CRUZ ; G. G. HAAS.** *Fertility and Sterility,* 1996, vol. 65 (4), 843 ff